# EUROPEAN PATENT APPLICATION

(11) **EP 1 878 804 A1**
(43) Date of publication of application: **16.01.2008**
(21) Application number: 06732047.3
(22) Date of filing: 18.04.2006
(51) Int. Cl.: C12Q 1/68, A61K 31/7088, A61K 48/00, A61P 25/16, C12N 15/09, G01N 33/15, G01N 33/50, G01N 33/53

(54) **PERKINSON DISEASE-RELATED GENE GRK5 AND USE THEREOF**

(30) Priority: 18.04.2005 JP 2005120225
(71) Applicant: Hubit Genomix, Inc., Tokyo 102-0092 (JP); Kato, Takeo, Kaminoyama-shi, Yamagata 990-9585 (JP)
(72) Inventor: ARAWAKA, Shigeki Yamagata University Fac. Medicine, Yamagata-shi, Yamagata 9909585 (JP); MATSUMINE, Hiroto, Chiyoda-ku, Tokyo 1020092 (JP); KATO, Takeo, c/o Yamagata University Faculty of Medicine, Yamagata, 9909585 (JP)
(74) Representative: Bosch, Matthias
(86) International application number: PCT/JP2006/308118
(87) International publication number: WO 2006/112456

(57) **Abstract**

Large-scale SNP analyses conducted on subjects in a Parkinson's disease patient group and a normal control group led to the successful identification of a gene (GRK5) associated with Parkinson's disease. In addition, it was newly discovered that phosphorylation of α-synuclein is promoted by enhanced expression of the GRK5 gene, and as a result, the formation of soluble α-synuclein oligomers is promoted, leading to Parkinson's disease. The present invention enables the assessment of Parkinson's disease as well as the screening of therapeutic agents for Parkinson's disease using as an index the expression of GRK5 gene.

## Description

### Technical Field

The present invention is directed to methods of testing for Parkinson's disease (PD) using the expression or polymorphic variation of the GRK5 gene as an index, and agents for treating Parkinson's disease or methods of screening for agents for treating Parkinson's disease using the GRK5 gene.

### Background Art

Parkinson's disease (PD) is a progressive neurodegenerative disease with a high prevalence (see Non-Patent Document 1). Patients with PD show symptoms such as bradykinesia, tremor, muscular rigidity, and/or postural reflex disturbance. PD occurs as a result of deficiency in cerebral/striatal dopamine due to selective degeneration/dropout of dopaminergic neurons in the mesencephalic nigra. The cerebral/striatal dopamine level is regulated by the balance between dopamine and acetylcholine. The mechanism of cell death of dopaminergic neurons remains undetermined.

Curable therapies that prevent the progression of cell death of dopaminergic neurons in PD have yet to be established, and currently there are only symptomatic therapies. For example, dopamine replacement therapies, which supplement deficient dopamine, are symptomatically effective for patients in early to intermediate stages of the disease. However, currently conductible treatments cannot block or reverse the progression of PD. As a result, PD patients generally become bedridden about 10 to 15 years after the onset of the disease. Accordingly, there is an urgent need for therapies that prevent cell death of dopaminergic neurons.

PD arises sporadically, or familially due to mutation of a single gene. The latter single-gene-caused PD is responsible for 5% to 10% of total PD cases, and causative genes identified so far include α-synuclein (αS) (see Non-Patent Documents 2 and 3), parkin (see Non-Patent Document 4), UCH-L1 (see Non-Patent Document 5), DJ-1 (see Non-Patent Document 6), and PINK1 (see Non-Patent Document 7). Sporadic PD (sPD) cases account for 90% to 95% of total PD patients, and sPD onset is considered to be complicatedly associated with multiple genetic factors and environmental factors.

The contribution of genetic factors to sPD is deduced from the familial aggregation of sPD and studies on twins using positron emission tomography (PET). The familial aggregation of sPD has been demonstrated by comparison with a control group in a case-control study (see Non-Patent Document 8). A study of the entire Iceland population has demonstrated that late-onset PD patients have strong consanguinity (the relative risk values for siblings, parent/offspring pairs, and cousins are 6.7, 3.2, and 2.7, respectively) (see Non-Patent Document 9). Studies on twins using PET have shown a concordance rate of 75% for the onset of late-onset PD in identical twins, which is higher than that (22%) in fraternal twins (see Non-Patent Documents 10 and 11). All of these findings indicate a genetic background to the pathogenic mechanism of sPD.

The importance of αS in the pathogenic mechanism of PD is apparent from the presence of familial PD caused by αS gene mutations (see Non-Patent Documents 12 and 3). In addition, αS accumulates in the Lewy bodies of sPD patients (see Non-Patent Documents 13 to 15). It has recently been revealed that αS phosphorylated at Ser-129 (p-αS) accumulates in Lewy bodies in sPD brains. Such p-αS molecules have the property of aggregating *in vitro* to form insoluble fibers (see Non-Patent Document 16), which suggests that the regulation of the phosphorylation of αS, particularly the phosphorylation of Ser-129, affects susceptibility to sPD (see Non-Patent Documents 16 and 17). It has been reported that αS is phosphorylated *in vitro* by protein kinases including casein kinase 1 (CK1), CK2, GRK2, and GRK5 (see Non-Patent Document 18). All of these protein kinases phosphorylate Ser-129 of αS (see Non-Patent Document 18). However, the pathophysiological association of these protein kinases with sPD is unknown.

It is also known that αS has structural characteristics similar to a fatty acid-binding protein family (see Non-Patent Document 19), and that the formation of the soluble oligomers of αS is promoted by fatty acids (see Non-Patent Document 20). The soluble oligomers of αS accumulate prior to the formation of insoluble oligomers. The soluble oligomers excessively accumulate in αS-transgenic mice and PD brains. Accordingly, the excessive accumulation of soluble oligomers is speculated to be a cause of nerve cell death in PD (see Non-Patent Documents 21 and 22).

However, the formation mechanism of soluble αS oligomers remains undetermined, and the factors involved in promoting the formation are also yet to be found.
[Non-Patent Document 1] Kimura H et al., "Female preponderance of Parkinson's disease in Japan", Neuroepidemiology, 2002, Vol.21, p.292-296
[Non-Patent Document 2] Polymeropoulos MH et al., "Mutation in the alpha-synuclein gene identified in families with Parkinson's disease", Science, 1997, Vol.276, p.2045-2047
[Non-Patent Document 3] Kruger R (u with umlaut) et al., "Ala30Pro mutation in the gene encoding alpha-synuclein in Parkinson's disease", Nat Genet, 1998, Vol. 18, p.106-108
[Non-Patent Document 4] Kitada T et al., "Mutations in parkin gene cause autosomal recessive juvenile parkinsonism", Nature, 1998, Vol. 392, p.605-608
[Non-Patent Document 5] Leroy E, Boyer R, Auburger G, Leube B, Ulm G, Mezey E, "The ubiquitin pathway in Parkinson's disease", Nature, 1998, Vol. 395, p.451-452
[Non-Patent Document 6] Bonifati V et al., "Mutations in the DJ-1 gene associated with autosomal recessive early-onset parkinsonism", Science, 2003, Vol. 299, p.256-259
[Non-Patent Document 7] Valente EM et al., "Hereditary early-onset Parkinson's disease caused by mutations in PINK1", Science, 2004, Vol. 304, p.1158-1160
[Non-Patent Document 8] Payami H et al., "Familial aggregation of Parkinson disease: a comparative study of early-onset and late-onset disease", Arch Neurol, 2002, Vol. 59, p.848-850
[Non-Patent Document 9] Sveinbjornsdottir S (the first o with umlaut) et al., "Familial aggregation of Parkinson's disease in Iceland", N Engl J Med, 2000, Vol. 343, p.1765-1770
[Non-Patent Document 10] Burn DJ et al., "Parkinson's disease in twins studied with 18F-dopa and positron emission tomography", Neurology, 1992, Vol. 42, p.1894-1900
[Non-Patent Document 11] Piccini P, et al., "The role of inheritance in sporadic Parkinson's disease: evidence from a longitudinal study of dopaminergic function in twins", Ann Neurol, 1999, Vol. 45, p.577-582
[Non-Patent Document 12] Polymeropoulos MH, et al., "Mutation in the alpha-synuclein gene identified in families with Parkinson's disease", Science, 1997, Vol. 276, p.2045-2047
[Non-Patent Document 13] Spillantini MG, et al., "α-Synuclein in Lewy bodies" Nature, 1997, Vol. 388, p.839-840
[Non-Patent Document 14] Spillantini MG, et al., "α-Synuclein in filame tous inclusions of Lewy bodies from Parkinson's disease and dementia with Lewy bodies", Proc Natl Acad Sci USA, 1998, Vol. 95, p.6469-6473
[Non-Patent Document 15] Baba M et al., "Aggregation of α-synuclein in Lewy bodies of sporadic Parkinson's disease and dementia with Lewy bodies", Am J Pathol, 1998, Vol. 152, p.879-884
[Non-Patent Document 16] Fujiwara H et al., "α-Synuclein is phosphorylated in synucleinopathy lesions", Nat Cell Biol, 2002, Vol. 4, p.160-164
[Non-Patent Document 17] Neumann M et al., "Misfolded proteinase K-resistant hyperphosphorylated alpha-synuclein in aged transgenic mice with locomotor deterioration and in human alpha-synucleinopathies", J. Clin. Invest., 2002, Vol. 110, p.1429-1439
[Non-Patent Document 18] Pronin AN et al., "Synucleins are a novel class of substrates for G protein-coupled receptor kinases", J Biol Chem, 2000, Vol. 275, p. 26515-26522
[Non-Patent Document 19] Sharon R et al., "α-Synuclein occurs in lipid-rich high molecular weight complexes, binds fatty acids, and shows homology to the fatty acid-binding proteins", Proc Natl Acad Sci USA, 2001, Vol. 98, p.9110-9115
[Non-Patent Document 20] Sharon R et al., "The formation of highly soluble oligomers of α-synuclein is regulated by fatty acids and enhanced in Parkinson's disease", Neuron, 2003, Vol. 37, p.583-595
[Non-Patent Document 21] Welch K, Yuan J., "Alpha-synuclein oligomerization: a role for lipids?", Trends Neurosci., 2003, Vol. 26, p.517-519
[Non-Patent Document 22] Selkoe DJ., "Cell biology of protein misfolding: The examples of Alzheimer's and Parkinson's diseases", Nature Cell Biology, 2004, No. 6, p.1054-1061

### Disclosure of the Invention

### [Problems to be Solved by the Invention]

An objective of the present invention is to identify genes associated with Parkinson's Disease (PD). Another objective of the present invention is to carry out genetic and biochemical analyses on the identified genes and, based on the resulting findings, provide methods of testing for PD, pharmaceutical agents for treating PD, and methods of screening for these agents, using the genes.

### [Means for Solving the Problems]

The present inventors conducted dedicated research to achieve the objectives. First, in order to identify genes associated with Parkinson's Disease (PD), they made an attempt to identify polymorphic variations or haplotypes that are significantly associated with PD. More specifically, the present inventors conducted a large-scale screening for genes or single nucleotide polymorphisms (SNPs) associated with PD in PD patients and healthy persons.

PD patients must be reliably selected to identify PD-associated genes in this screening. Namely, it is important to definitively diagnose whether each of a large number of patients to be screened actually suffers from PD.

Target patients were recruited and subjected to definitive diagnoses in principal medical institutions in Yamagata Prefecture, Japan. Diagnostic criteria for PD are as follows:
1) insidious onset;
2) apparent response to antiparkinson agents such as L-dopa;
3) exclusion of other diseases which show symptoms similar to those of PD (e.g., vascular parkinsonism, olivopontocerebellar atrophy, progressive supranuclear palsy, and corticobasal degeneration); and
4) no history of using drugs that cause Parkinsonian syndrome.

According to these criteria, PD patients can be distinguished from patients suffering from "Parkinsonian syndrome", who show clinical symptoms very similar to those of PD. Parkinsonian syndrome collectively refers to diseases that occur due to a certain defect in the nigrostriatal pathway. Parkinsonian syndrome includes diseases having pathogenic mechanisms different to those of PD, which is associated with the selective dropout of dopaminergic neurons. Accordingly, screening for only PD patients is very important in carrying out statistical genetic studies.

It was revealed that 1477 PD patients were receiving medical care in 208 hospitals and clinics out of the 782 medical institutions in Yamagata Prefecture. The prevalence of PD was about 118.7 per 100,000 individuals. This result substantially conforms to results of studies conducted in other regions in Japan.

Clinical data on 963 out of the 1477 patients were registered. The 963 PD patients were all subjected to detailed neurological inspections and cerebral MRI by neurologists and neurosurgeons to exclude other diseases more precisely. Definitive diagnoses of PD were made via inspections and examinations by specialists to minimize inclusion of Parkinsonian syndrome patients. Among the patients screened according to the above criteria, 317 patients gave their consent to provide blood samples for genetic epidemiological studies.

A control group of 496 persons was obtained as follows. All persons in certain parts of the Takahata Town and Sagae City in Yamagata Prefecture can receive free public medical examinations when they reach the ages of 60 and 70 years. Of the persons who had undergone medical examinations, persons showing no medical abnormality were selected as subjects. After obtaining written consent, MRI and neurological examinations were conducted, and the presence or absence of nerve diseases was checked by neurologists. According to the above method, a control group showing none of the symptoms of nerve diseases including PD was obtained through diagnoses by neurological specialists. Because elderly persons who underwent a medical examination were used as subjects, it was considered that persons with genetic factors susceptible to PD would have largely been excluded from the control group because persons with such genetic factors are expected to develop the disease before they become aged.

As described above, a disease group and a control group were successfully acquired based on accurate diagnoses. The inspections and MRI examinations conducted by specialists on both the control and disease groups ensure a marked increase in the accuracy of the genetic analyses. There has been no other study in the world in which such groups were obtained as analysis targets.

The disease group (patient group) and control group (normal group), selected as described above, were screened for 5632 SNPs on 520 genes.

As a result, two polymorphic variations (m22.1 and m24) that are significantly associated with PD were discovered. Both of the polymorphic variations were present in introns of the gene encoding G protein-coupled receptor kinase 5 (GRK5).

Furthermore, *in vitro* analyses showed that the transcription of the GRK5 gene is promoted by the two polymorphic variations found by the present inventors.

Specifically, it was strongly suggested that the enhancement of GRK5 gene expression is strongly associated with the onset of PD.

In addition, a low-frequency haplotype that increases the morbidity risk of PD was found in the GRK5 gene. Thus, the present inventors found, for the first time, that the GRK5 gene is associated with PD.

The present inventors also succeeded in finding polymorphisms (m22.1 and m24) in which a variation of a single nucleotide significantly alters the GRK5 gene expression. These polymorphisms exist in transcriptional regulatory regions of the YY1-recognition sequence and the CREB-recognition sequence, respectively. The polymorphic variations may alter the binding ability of the transcriptional regulatory regions with transcription factors, thereby increasing the transcriptional activity of GRK5. DNA comprising a transcriptional regulatory region having the above polymorphic variation can be used, for example, in screening for factors that suppress the GRK5 gene expression.

GRK5 is a member of the GRK family, which phosphorylates and thereby desensitizes an activated G protein-coupled receptor (GPCR) bound to an agonist, and is important for homeostasis of the GPCR signal transduction system (Inglese J, Freedman NJ, Koch WJ, Lefkowitz RJ. Structure and mechanism of the G protein-coupled receptor kinases. J Biol Chem 268, 23735-23738 (1993); Pitcher JA, Freedman NJ, Lefkowitz RJ. G protein-coupled receptor kinases. Annu Rev Biochem 67, 653-692 (1998)). In the brain, the GRK family, including GRK5, regulates the function of dopamine receptors belonging to the GPCR family (Tiberi M, Nash SR, Bertrand L, Lefkowitz RJ, Caron MG Differential regulation of dopamine D1A receptor responsiveness by various G protein-coupled receptor kinases. J Biol Chem 271, 3771-3778 (1996); Ito K, Haga T, Lameh J, Sadee W. Sequestration of dopamine D2 receptors depends on coexpression of G-protein-coupled receptor kinases 2 or 5. Eur J Biochem 260, 112-119 (1999)).

Sharon *et al.* proposed the hypothesis that nerve cells are damaged by the accumulation of soluble αS oligomers, and that the oligomers are then insolubilized (Sharon R, Bar-Joseph I, Frosch MP, Walsh DM, Hamilton JA, Selkoe DJ. The formation of highly soluble oligomers of α-synuclein is regulated by fatty acids and enhanced in Parkinson's disease. Neuron 37, 583-595 (2003)). sPD is considered to be a multifactorial disease triggered by a combination of some disease-susceptibility genes and unidentified environmental factors. The present invention verified that SNPs in introns of GRK5 increase the risk of sPD.

The present inventors performed further biochemical analyses of GRK5. As a result, disease susceptibility alleles of the SNPs found by the present inventors were found to enhance GRK5 expression, induce α-synuclein (αS) phosphorylation, and promote the formation of soluble αS oligomers having cytotoxicity.

Although it has been reported that some GRKs can phosphorylate αS as a substrate (Pronin AN et al., J. Biol. Chem. 275: 26515-26522 (2000)), the present inventors found, for the first time, that GRK5 phosphorylates αs on the cell membrane, that αS phosphorylated on the cell membrane migrates to the cytoplasm over time, and that the formation of soluble αS oligomers is thereby promoted.

It is possible to prevent or treat PD by inhibiting the expression or function of GRK5 based on the findings found by the present inventors. Specifically, PD can be tested using the enhanced expression or function of GRK5 as an index. Compounds that suppress the expression or function of GRK5 are expected to serve as agents for preventing or treating PD. In addition, screening of agents for preventing or treating PD can be conducted by using as an index the expression level or function of GRK5, or the enhanced formation of soluble αS oligomers.

The present invention also shows potential for novel neuroprotective treatments in which the progress of neurodegeneration, which underlies the dyskinesia of sPD, can be delayed by specifically inhibiting the binding between GRK5 and αS. In order to find a drug discovery target, it may be important to find a kinase acting on αS as a substrate and to study that kinase for the promotion of soluble oligomer formation. At any rate, the results found by the present inventors strongly suggest the potential of GRK5 as a fully novel target of dug discovery for sPD.

As described above, the present inventors identified GRK5 as a gene associated with the onset of PD, and also provided methods of testing for PD, agents for treating PD, and methods of screening for the agents, all based on the findings obtained by genetic and biochemical analyses of the gene, thereby completing the present invention.

In the present invention, accurate screening (definitive diagnosis) was conducted on both a control group (normal group) and a disease group through inspections and MRI examinations by specialists, as mentioned above. To obtain a high degree of reliability in polymorphic analyses, it is essential to precisely screen subjects in not only a patient group, but also a control group (normal group) to be analyzed. A symptom (manifestation) of PD may be found even in some apparently healthy persons by inspections and MRI examinations conducted by specialists. If these subjects are subjected to polymorphic analyses as a control group (normal group), it is difficult to obtain reliable results. In the present invention, the reliability of test samples from both the control group (normal group) and the disease group was increased by accurately screening subjects to be subjected to polymorphic analyses. This approach can be said to have been a major factor in the resulting success in actually identifying a gene associated with PD.

The present invention specifically provides:
[1] a method of testing whether or not a subject is susceptible to Parkinson's disease, which uses as an index the expression of G-protein-coupled receptor kinase-5 (GRK5) gene in the subject;
[2] a method of testing whether or not a subject is susceptible to Parkinson's disease, which comprises the step of detecting a mutation in a GRK5 gene of the subject;
[3] the method of [2], wherein the mutation is a polymorphic mutation;
[4] a method of testing whether or not a subject is susceptible to Parkinson's disease, which comprises the step of determining a nucleotide at a polymorphic site in a GRK5 gene of the subject;
[5] the method of [4], wherein the polymorphic site is (1) and/or (2):
   (1) a polymorphic site on the GRK5 gene located at position 34799 of the nucleotide sequence of SEQ ID NO: 1; and
   (2) a polymorphic site on the GRK5 gene located at position 60001 of the nucleotide sequence of SEQ ID NO: 1;
[6] the method of [5], wherein the subject is deemed to be susceptible to Parkinson's disease when nucleotides in the polymorphic sites (1) and (2) set forth in [5] are (1b) and (2b), respectively:
   (1b) the nucleotide at a site on the GRK5 gene located at position 34799 of the nucleotide sequence of SEQ ID NO: 1 is G; and
   (2b) the nucleotide at a site on the GRK5 gene located at position 60001 of the nucleotide sequence of SEQ ID NO: 1 is C;
[7] a method of testing whether or not a subject is susceptible to Parkinson's disease, wherein the subject is deemed to be susceptible to Parkinson's disease when a DNA block showing the haplotype of (1') is detected:
   (1') a haplotype in which the nucleotides at polymorphic sites on a GRK5 gene located at positions 27377, 54748, and 60001 of the nucleotide sequence of SEQ ID NO: 1 are G, A, and C, respectively;
[8] a method of testing whether or not a subject is susceptible to Parkinson's disease, comprising the steps of:
   (a) determining a nucleotide at a polymorphic site on a GRK5 gene of the subject; and
   (b) determining the subject as susceptible to Parkinson's disease when the nucleotide determined in step (a) is the same as a nucleotide type at said polymorphic site on a GRK5 gene that shows the haplotype of (1'):
      (1') a haplotype in which nucleotides at polymorphic sites on a GRK5 gene located
      at positions 27377, 54748, and 60001 of the nucleotide sequence of SEQ ID NO: 1 are G, A, and C, respectively;
[9] the method of [8], wherein the polymorphic site in step (a) is a polymorphic site set forth in (1):
   (1) any polymorphic site on the GRK5 gene located at position 27377, 27849, 27876, 28813, 29000, 29855, 30366, 31037, 31706, 31964, 32542, 32723, 32988, 33064, 33618, 33889, 33911, 33994, 34799, 35173, 35188, 35189, 35190, 35240, 35284, 35402, 35415, 35494, 35522, 36405, 36433, 36449, 36546, 36817, 36868, 36882, 37004, 37148, 37295, 37413, 37722, 38393, 39182, 39193, 39301, 39316, 39456, 39487, 39550, 39638, 40097, 40134, 40193, 41149, 41157, 41428, 41498, 41618, 41667, 41843, 42364, 42418, 43017, 43037, 43190, 44600, 44607, 45522, 45998, 48284, 49971, 50122, 50873, 51463, 52944, 53223, 53397, 53703, 53763, 54267, 54748, 54782, 54794, 55139, 55457, 55627, 56254, 56593, 57338, 57606, 57648, 57688, 57843, 57978, 58067, 58456, 58616, 59263, 59908, or 60001 of the nucleotide sequence of SEQ ID NO: 1;
[10] the method of [8], wherein the polymorphic site in step (a) is the polymorphic site set forth in (1) or (2):
   (1) a polymorphic site on the GRK5 gene located at position 34799 of the nucleotide sequence of SEQ ID NO: 1; and
   (2) a polymorphic site on the GRK5 gene located at position 60001 of the nucleotide sequence of SEQ ID NO: 1;
[11] a method of testing a subject for the presence or absence of a susceptibility for Parkinson's disease, comprising the step of determining the subject to be susceptible to Parkinson's disease when a measured expression level of a GRK5 gene in the subject is elevated as compared to a control;
[12] the method of any one of [1] to [11], wherein a biological sample derived from the subject is utilized as a test sample;
[13] the method of any one of [1] to [12], wherein the Parkinson's disease is sporadic Parkinson's disease;
[14] a reagent for testing whether or not a subject is susceptible to Parkinson's disease, comprising an oligonucleotide that hybridizes with a DNA comprising the polymorphic site of (1) or (2) of [5] and comprises at least 15 nucleotides in length;
[15] a reagent for testing whether or not a subject is susceptible to Parkinson's disease, comprising a solid phase to which a nucleotide probe is immobilized, wherein the nucleotide probe hybridizes with a DNA comprising the polymorphic site of (1) or (2) of [5];
[16] a reagent for testing whether or not a subject is susceptible to Parkinson's disease, comprising a primer oligonucleotide for amplifying a DNA comprising the polymorphic site of (1) or (2) of [5];
[17] a reagent for testing whether or not a subject is susceptible to Parkinson's disease, comprising as an active ingredient (a) or (b):
   (a) an oligonucleotide that hybridizes with a transcript of a GRK5 gene; and
   (b) an antibody that recognizes a GRK5 protein;
[18] a polynucleotide of (a) or (b):
   (a) a polynucleotide which comprises the nucleotide sequence of SEQ ID NO: 5 or a nucleotide sequence in which one or more nucleotides are added, deleted, or substituted in the nucleotide sequence of SEQ ID NO: 5, wherein the polynucleotide has a reduced ability to bind with a Yin Yang-1 (YY1) transcription factor; and
   (b) a polynucleotide which comprises the nucleotide sequence of SEQ ID NO: 3 or a nucleotide sequence in which one or more nucleotides are added, deleted, or substituted in the nucleotide sequence of SEQ ID NO: 3, wherein the polynucleotide has an increased ability to bind with a CRE-binding protein-1 (CREB-1) transcription factor;
[19] a reagent for screening for an agent for treating or preventing Parkinson's disease, comprising as an active ingredient any one of (a) to (d):
   (a) an oligonucleotide that hybridizes with a transcript of a GRK5 gene;
   (b) an antibody that recognizes a GRK5 protein;
   (c) an antibody that recognizes an α-synuclein protein in which a serine residue at position 129 is phosphorylated; and
   (d) the polynucleotide of [18];
[20] the reagent of any one of [14] to [17], and [19], wherein the Parkinson's disease is sporadic Parkinson's disease;
[21] an agent for treating or preventing Parkinson's disease, comprising as an active ingredient a substance that suppresses the expression of a GRK5 gene or the function of a protein encoded by the gene;
[22] the agent of [21], wherein the substance that suppresses the expression of the GRK5 gene is one selected from the group consisting of:
   (a) an antisense nucleic acid against a transcript of the GRK5 gene or a portion thereof;
   (b) a nucleic acid having a ribozyme activity of specifically cleaving a transcript of the GRK5 gene; and
   (c) a nucleic acid having an activity of inhibiting the expression of the GRK5 gene through an RNA interference effect;
[23] the agent of [21], wherein the substance that suppresses the function of the GRK5 protein is:
   (a) an antibody that binds with a GRK5 protein; or
   (b) a low-molecular-weight compound that binds with a GRK5 protein;
[24] an agent for treating or preventing Parkinson's disease, comprising as an active ingredient a substance that inhibits the formation of a soluble α-synuclein oligomer;
[25] a method of screening for an agent for treating or preventing Parkinson's disease, comprising the step of selecting a compound that reduces the expression level of a GRK5 gene or the activity of a protein encoded by the gene;
[26] a method of screening for an agent for treating or preventing Parkinson's disease, comprising the steps of:
   (a) contacting a test compound with a cell that expresses a GRK5 gene;
   (b) measuring the expression level of the GRK5 gene; and
   (c) selecting the compound that reduces the expression level as compared with that measured in the absence of the test compound;
[27] a method of screening for an agent for treating or preventing Parkinson's disease, comprising the steps of:
   (a) contacting a test compound with a cell or cell extract that comprises a DNA having a structure in which a transcriptional regulatory region of a GRK5 gene and a reporter gene are operably linked with each other;
   (b) measuring the expression level of the reporter gene; and
   (c) selecting the compound that reduces the expression level as compared with that measured in the absence of the test compound;
[28] the method of any one of [25] to [27], wherein the GRK5 gene is:
   (a) a mutant GRK5 gene in which a nucleotide on the GRK5 gene located at position 34799 of the nucleotide sequence of SEQ ID NO: 1 is G; or
   (b) a mutant GRK5 gene in which a nucleotide on the GRK5 gene located at position 60001 of the nucleotide sequence of SEQ ID NO: 1 is C,
   wherein the expression of the gene is elevated;
[29] a method of screening for an agent for treating or preventing Parkinson's disease, comprising the steps of:
   (a) contacting a test compound with a GRK5 protein and α-synuclein;
   (b) measuring the interaction activity between the GRK5 protein and α-synuclein; and
   (c) selecting the compound that reduces the interaction activity as compared with that measured in the absence of the test compound;
[30] a method of screening for an agent for treating or preventing Parkinson's disease, comprising the steps of:
   (a) contacting a test compound with a GRK5 protein and α-synuclein;
   (b) measuring the activity of the GRK5 protein to phosphorylate α-synuclein as a substrate; and
   (c) selecting the compound that reduces the phosphorylating activity as compared with that measured in the absence of the test compound;
[31] a method of screening for an agent for treating or preventing Parkinson's disease, comprising the steps of:
   (a) contacting a test compound with a GRK5 protein and an insoluble α-synuclein oligomer;
   (b) measuring the amount of a soluble α-synuclein oligomer; and
   (c) selecting the compound that reduces the amount of the soluble α-synuclein oligomer as compared with that measured in the absence of the test compound;
[32] a method of screening for an agent for treating or preventing Parkinson's disease, comprising the steps of:
   (a) contacting a test compound with a YY1 transcription factor, and a polynucleotide comprising a YY1-recognition sequence or the polynucleotide of [18](a);
   (b) measuring a binding activity between the polynucleotide and the transcription factor; and
   (c) selecting the compound that elevates the binding activity as compared with that measured in the absence of the test compound; and
[33] a method of screening for an agent for treating or preventing Parkinson's disease, comprising the steps of:
   (a) contacting a test compound with a CREB-1 transcription factor, and a polynucleotide comprising a CREB-recognition sequence or the polynucleotide of [18](b);
   (b) measuring a binding ability between the polynucleotide and the transcription factor; and
   (c) selecting the compound that reduces the binding activity as compared with that measured in the absence of the test compound.

   In preferred embodiments, the present invention provides:
[34] a transgenic non-human animal, which expresses an exogenous GRK5 protein and α-synuclein;
[35] a method of screening for an agent for treating or preventing Parkinson's disease, comprising the steps of:
   (a) administering a test compound to the transgenic non-human animal of [34];
   (b) monitoring a behavior in the animal caused by neurotoxicity; and
   (c) selecting the compound that suppresses occurrence of the behavior;
[36] a method of preventing and/or treating Parkinson's disease, including the step of administering to an individual (e.g., a patient) a substance that suppresses the expression of a GRK5 gene or the function of a GRK5 protein; and
[37] use of a substance that suppresses the expression of a GRK5 gene or the function of a GRK5 protein in production of an agent for treating Parkinson's disease.

### Brief Description of the Drawings

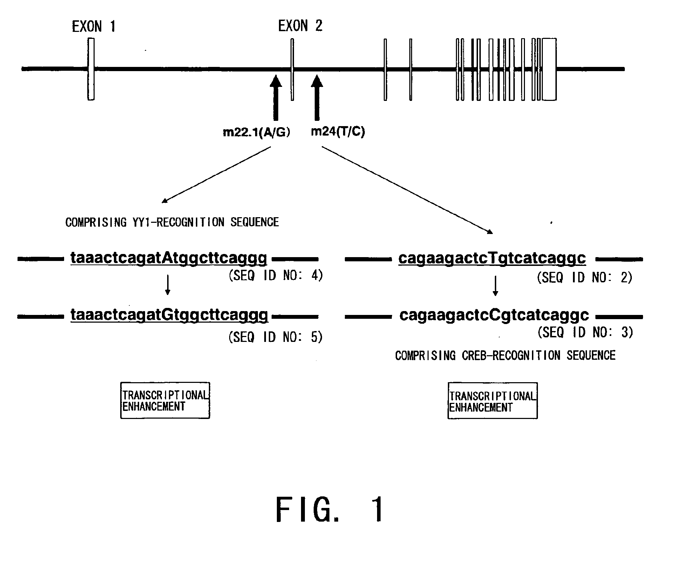

Fig. 1 is a schematic diagram depicting positions and sequences of m22.1 and m24 polymorphic mutations on the GRK5 gene.

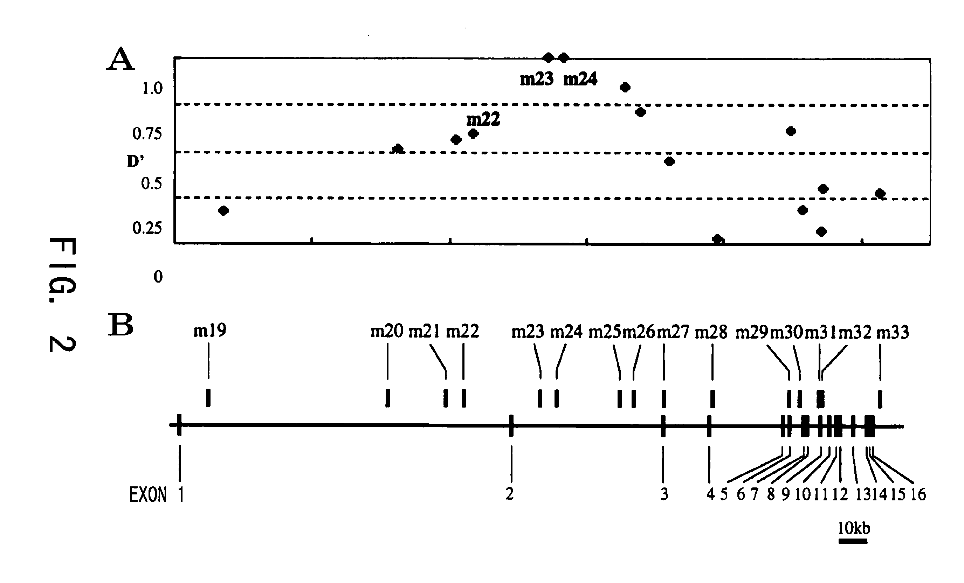

Fig. 2A is a graph demonstrating the distribution of D' for polymorphic mutations m19 to m33 and the D' scores between SNP m24 and other SNPs. Fig. 2B is a schematic diagram depicting the positions of SNPs m19 to m33 and exons 1 to 16 on the GRK5 gene.

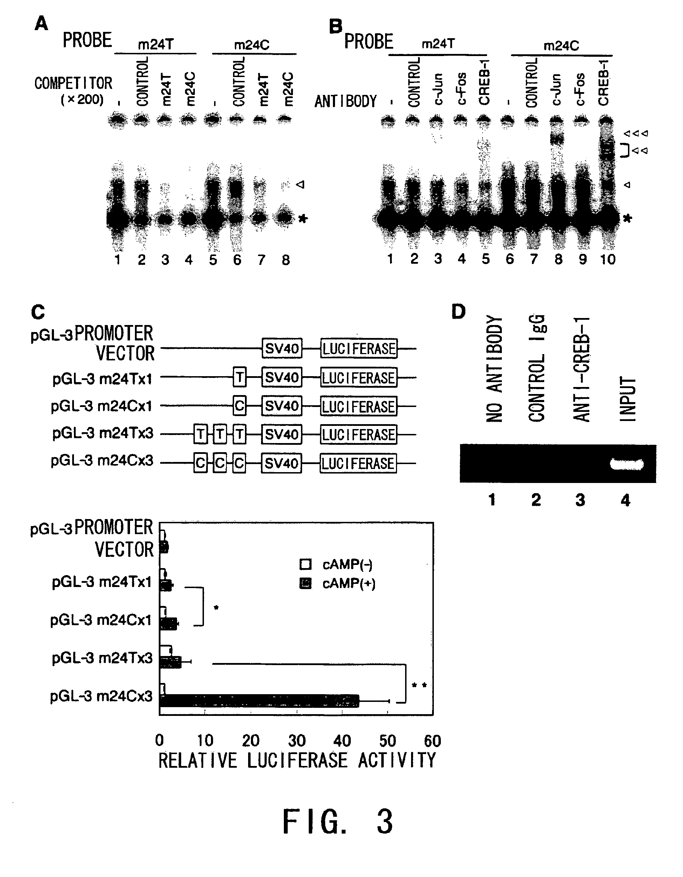

Fig. 3 is a set of photographs and diagrams demonstrating that SNP m24 located in an intron affects the transcriptional activity. The asterisks (*) in A and B indicate nonspecific bands. Fig. 3A is a photograph depicting the results of a gel-shift assay and a competitive assay on SNP m24. ³²P-labeled oligonucleotides of the T allele (m24T: lanes 1-4), which is not susceptible to the disease, and ³²P-labeled oligonucleotides the C allele (m24C: lanes 5-8), which is susceptible to the disease, were reacted with nuclear extract from SH-SY5Y cells. As competitors, 200-fold excess of nonlabeled probes were added (lanes 2-4 and 6-8). No competitor was added for lanes 1 and 5. Fig. 3B is a photograph showing a result of a supershift assay on SNP m24. A ³²P-labeled oligonucleotide of m24T (lanes 1 to 5) or m24C (lanes 6 to 10) was reacted with nuclear extract from SH-SY5Y cells. Antibodies were added for lanes 2 to 5 and lanes 7 to 10, and not for lanes 1 and 6. Anti CREB 1 antibody diminished shifted bands (arrowhead; lanes 5 and 10) and produced two supershifted bands (double arrowhead; lanes 5 and 10). Anti-c-Jun antibody also produced a supershifted band (triple arrowhead; lanes 3 and 8). The intensity of supershifted bands was higher in m24C (lanes 8 and 10) than in m24T (lanes 3 and 5). A control antibody (anti-STAT-1 antibody) did not affect the intensity of shifted bands, nor produce supershifted bands (lanes 2 and 7). Fig. 3C is a graph showing the result of luciferase assay on SNP m24. One or three copies of m24T or m24C oligonucleotide were tandemly inserted into a pGL-3 promoter vector. The vector was transferred into SH-SY5Y cells, and relative luciferase activity (RLA) was measured. No difference was observed among the five vectors in the absence of cAMP. In the presence of cAMP, one copy of m24C showed a 1.5-fold increase in RLA than one copy of m24T (p<0.05), and three copies of m24C showed a 9.7-fold increase in RLA than three copies of m24T (p<0.0001). Fig. 3D is a photograph showing the binding of CREB-1 to the m24 region in the GRK5 gene intron 2. DNA- protein complexes were immunoprecipitated. Lane 3 shows the binding of endogenous CREB-1 to the region containing m24 in SH-SY5Y cells. Lane 1: no antibody; Lane 2: unrelated antibody; Lane 3: anti-CREB-1 antibody; Lane 4: input.

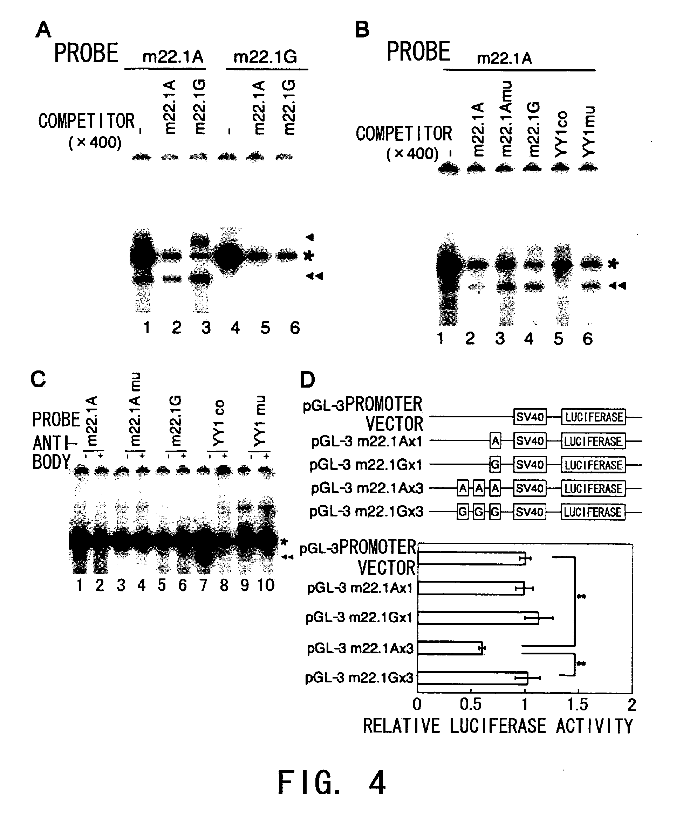

Fig. 4 is a set of photographs and diagrams demonstrating that SNP m22.1 located in an intron affects the transcriptional activity. The asterisks (*) in A, B, and C indicate nonspecific bands. Fig. 4A is a photograph depicting the results of a gel-shift assay and a competitive assay on SNP m22.1. ³²P-labeled oligonucleotides of the A allele (m22.1 A: lanes 1 to 3), which is not susceptible to the disease, or ³²P-labeled oligonucleotides of the G allele (m22.1 G: lanes 4 to 6), which is susceptible to the disease, were reacted with nuclear extract from SH-SY5Y cells. 400-fold excess of competitors were added for lanes 2, 3, 5, and 6, and not for lanes 1 and 4. Shifted bands (single and double arrowheads) were observed in m22.1A (lanes 1 to 3), but not in m22.1G (lane 3). The shift bands were diminished by addition of m22.1A competitors (unlabeled oligonucleotide probes), but not by m22.1G competitors (lane 3). Fig. 4B is a photograph depicting the results of a competitive assay on SNP m22.1 using a YY1-binding motif oligonucleotide. 400-fold excess of competitors were added for lanes 2 to 6 but not for lane 1, and the reaction was conducted. The shift band (double arrowhead) almost completely disappeared by addition of unlabeled YY1-binding sequence (YY1co; lane 5). However, it was not affected by the unlabeled mutant YY1 sequence (YY1mu; lane 6) or unlabeled mutant m22.1A (m22.1Amu; lane 3). Fig. 4C is a photograph showing the result of a supershift assay on SNP m22.1. A ³²P-labeled oligonucleotide of m22.1A (lanes 1 and 2), m22.1 Amu (lanes 3 and 4), m22.1G (lanes 5 and 6), YY1co (lanes 7 and 8), or YY1 mu (lanes 9 and 10) was reacted with nuclear extract from SH-SY5Y cells. An anti-YY1 antibody was added for lanes 2, 4, 6, 8, and 10 but was not for lanes 1, 3, 5, 7, and 9. The shifted band (double arrowhead) was produced by the ³²P-labeled oligonucleotide of m22.1 A (lane 1) or YY1co (lane 7). This band almost fully disappeared by the anti-YY1 antibody (lanes 2 and 8). Fig. 4D is a graph showing the result of a luciferase assay on SNP m22.1. One or three copies of m22.1A or m22.1 G oligonucleotide were tandemly inserted into a pGL-3 promoter vector. The vector was transferred into SH-SY5Y cells, and RLA was measured. When three copies were transferred, the m22.1A oligonucleotide showed a 42% decrease in RLA than the other four vectors (p<0.0001).

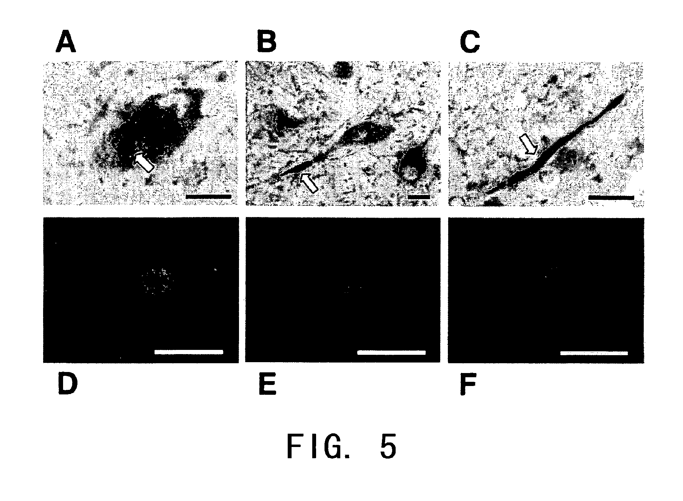

Fig. 5 is a set of photographs depicting the results of immunohistochemical staining of the mesencephalic nigra in autopsy cases of sPD patients. An anti-human GRK5 antibody (H-64) immunologically stained Lewy bodies (arrow) found in nigral dopaminergic neurons of sPD (A). Lewy neurites (arrow) were also GRK5-positive (B, C). Confocal laser microscopic images using an anti-GRK5 antibody (D) and an anti-αS (LB509) antibody (E) demonstrated that the two proteins are localized in Lewy bodies (D to F). F is a merged image of D and E. bar = 20 µm

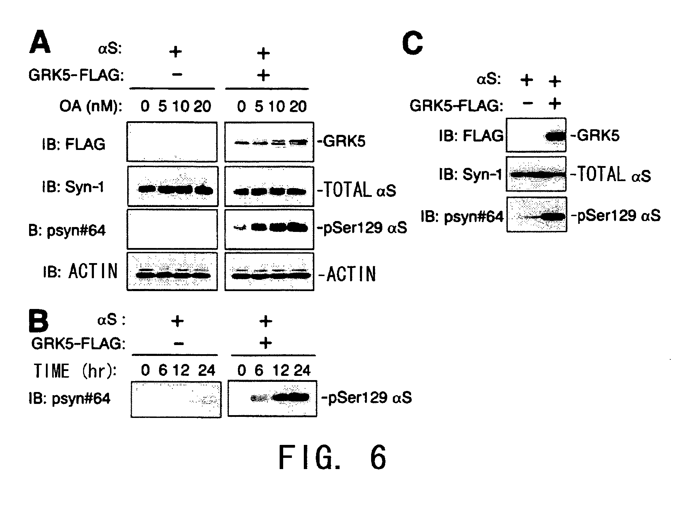

Fig. 6 is a photograph demonstrating that GRK5 phosphorylates Ser-129 of αS in HEK293 cells under the presence of okadaic acid in a dose/time dependent manner. Fig. 6A: After 30 hours of the introduction, okadaic acid was added to the culture medium at a concentration of 0, 5, 10, or 20 nM and the cells were further incubated for 16 hours. A cell lysate (20 µg) added with 1% NP-40 was loaded to each lane of a 12.5% Tris-glycine gel. The gel was electrophoresed and then transferred to a PVDF membrane. In GRK5-expressing cells, Ser-129 p-αS was detected proportionally to the concentration of okadaic acid by psyn#64 staining. Fig. 6B: After 24 hours of the introduction, 20 nM okadaic acid was added to the culture medium and incubated for 0, 6, 12, or 24 hours (up to 24 hours). Cells at 0 hour were collected as a sample at a starting point of okadaic acid treatment. In GRK5-expressing cells, the amount of Ser-129 p-αS increased with the time of culture in the presence of okadaic acid. Fig. 6C: Phosphorylation of αS by GRK5 was observed in neuroblastoma-derived SH-SY5Y cells.

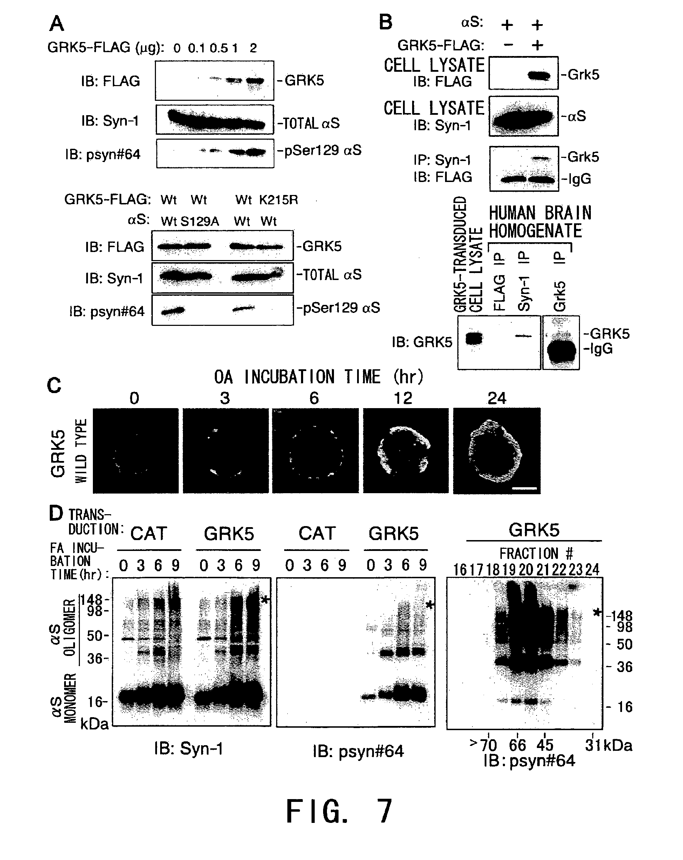

Fig. 7A is a photograph demonstrating that the phosphorylation of Ser-129 of αS correlates with the expression of GRK5 and that the phosphorylation of αS depends on the enzymatic activity of GRK5. Upper panel: The indicated amounts of GRK5-FLAG cDNA were transduced into HEK239 cells stably expressing αS. The expression level of total αS was substantially constant, but the amount of p-αS was increased depending on the amount of GRK5. Lower panel: Ser-129 phosphorylation was found in cells coexpressing wildtype GRK5 and αS. The phosphorylation did not occur by the combination of S129A mutant αS and wildtype GRK5; nor by the combination of wildtype αS and K215R mutant of GRK5. Fig. 7B is a photograph depicting the results of the experiment for immunoprecipitation of αS and GRK5 using HEK293 cells and human brain tissues. Upper panel: In the immunoprecipitate obtained from HEK293 cells expressing GRK5 and αS, the binding between GRK5 and αS was detected by Western blotting (WB) using an anti-GRK5 antibody. Lower panel: A human temporal lobe cortex tissue was treated in the same manner as above. Tissue lysate was immunoprecipitated with anti-FLAG (negative control), Syn-1, or anti-GRK5 antibody. GRK5 immunoprecipitated along with αS was detected by conducting Western blotting with an anti-GRK5 antibody. Fig. 7C is a photograph showing intracellular localization of Ser-129 p-αS catalyzed by GRK5. Cells were fixed and immunocytochemically stained with an anti-GRK5 antibody (red) and anti-p-Ser129 αS antibody (psy#64, green). Fig. 7D is a gel photograph showing that GRK5 promotes the fatty acid (FA)-induced αS oligomerization in cells through the phosphorylation. To analyze the αS oligomerization in cells over time, the cells were incubated with 20 mM okadaic acid for 16 hours, then treated with an α-linolenic acid/BSA complex, and incubated with okadaic acid for the time periods shown in the table. After centrifugation at 370,000x g, the supernatant (S370 cytosol) was incubated at 65°C for 16 hours to remove lipids, and analyzed by immunoblotting. Left and middle panels: HEK293 cells expressing αS and either CAT or GRK5-FLAG were treated with α-linolenic acid for the indicated time periods (up to 9 hours). Zero hour indicates the starting point of the treatment. The cells coexpressing αS and GRK5 were treated with okadaic acid, and the S370 cytosol (30 µg in terms of protein) was heat-treated (65°C, 16 hours). The resultant sample was applied to a gel, and immunoblotted using a Syn-1 or psyn#64 antibody. Regarding the amount of αs monomer and αS oligomer, a larger amount of αs oligomer was formed in the GRK5-expressing cells, which were abundant in phosphorylated αS, than in the CAT-expressing cells, in which unphosphorylated αS was predominant. Right panel: The S370 cytosol of cells coexpressing αS and GRK5 was applied to a gel filtration column (Superdex 75) and eluted with an ammonium acetate buffer. Each fraction was heat-treated (65°C, 16 hours) to remove lipids, and then analyzed by immunoblotting using psyn#64. The fractionation of the S370 cytosol by gel filtration was conducted under a condition without heat treatment (non-denaturing condition); however, a large amount of phosphorylated S129-αS oligomer was eluted in high molecular weight fractions. This demonstrates that αS oligomers are formed even under non-denaturing conditions without heat treatment. The asterisks (*) in the figure indicate the boundary between the stacking and separating gels.

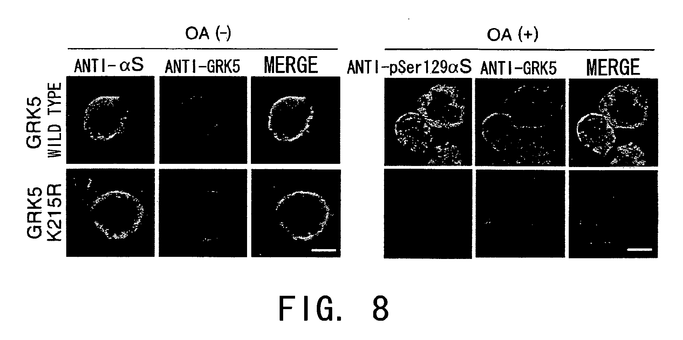

Fig. 8 is a set of photographs demonstrating that αS colocalizes with GRK5 in the cell membrane of HEK293 cells. HEK293 cells stably expressing wildtype αS were cultured on four-chamber slides, and wildtype GRK5-FLAG cDNA (upper panel) or mutant GRK5 K215R-FLAG cDNA (lower panel) was introduced to the cells and cultured in the absence of okadaic acid (left panel) or in the presence of okadaic acid (right panel). The cells were fixed and treated with a polyclonal GRK5 antibody (all panels, red), and either an anti-αS monoclonal antibody (LB509, left panel, green) or a monoclonal anti-pSer-129 αS antibody (psyn#64: right panel, green), and then subjected to direct immunofluorescence. Wildtype GRK5 was mainly localized in the cell membrane and was observed little in the perinuclear cytoplasm. Wildtype αS was mainly present in cytoplasmic regions and was colocalized with GRK5 at the inner side of the cell membrane, as shown in the merged image indicated with yellow (left panel). Ser-129 p-αS was present in the cell membrane and perinuclear cytoplasm region in wildtype GRK5-expressing cells, but was not observed in mutant GRK5 K215R-expressing cells. The localization of the mutant GRK5 was not different from that of the wildtype. bar = 10 µm.

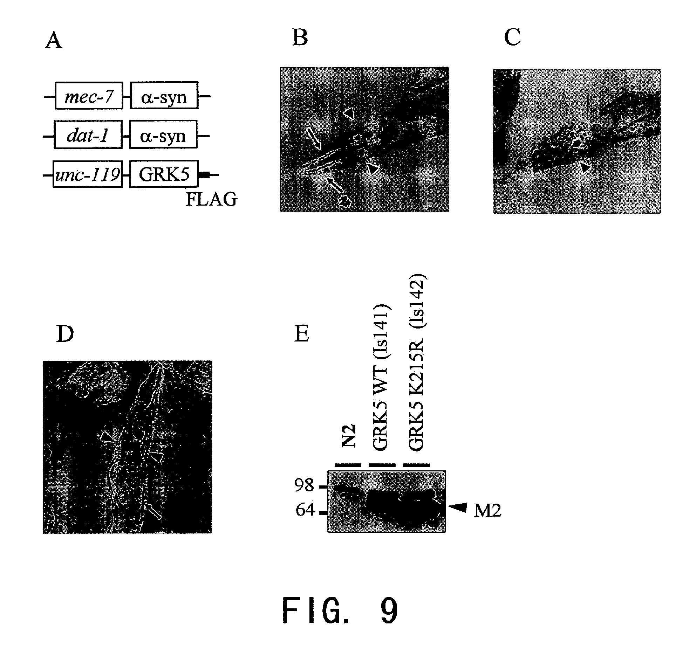

Fig. 9 is a set of diagrams and photographs that depict the configuration of plasmids for the transgenic nematode, immunoblotting, and immunohistological staining. A: The configurations of expression vectors for human α-synuclein and human GRK5 are shown. B: the expression of α-synuclein in a dopaminergic neuron is shown. The cell body (arrowhead; triangle) and dendrite (arrow →) of the dopamine neuron were positive in the immunohistochemical staining. C: The expression of α-synuclein in a posterior lateral microtubule (PLM) neuron is shown. As a result of the immunohistochemical staining, an α-synuclein-positive reaction was diffusely observed in the cytoplasm of the PLM neuron (arrowhead; triangle). D: The result of immunohistochemical staining of a transgenic nematode which expresses human GRK5 in all neurons is shown. An intense staining was observed in the nerve ring (arrowhead; triangle). The ventral nerve cord was GRK5-positive (arrow →). E: In nematodes that express wildtype GRK5 and K215R mutant GRK5, a band of a 64-kDa specific protein was detected by immunoblotting using an anti-FLAG antibody.

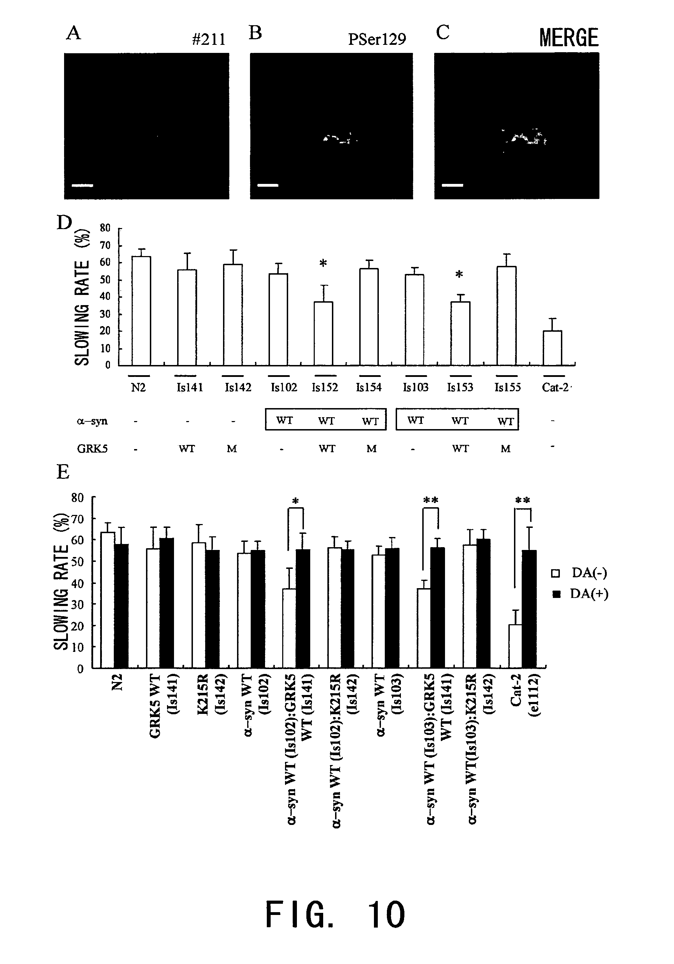

Fig. 10 is a set of diagrams and photographs demonstrating that GRK5 phosphorylates Ser-129 of α-synuclein in dopamine neurons and that the function of the dopamine neuron is lowered due to the α-synuclein phosphorylation by GRK5. A to C: The phosphorylation of α-synuclein at Ser-129 by GRK5 in dopamine neurons were observed with a confocal microscope. (A) anti-α-synuclein antibody (#211); (B) anti-phosphorylated α-synuclein antibody (PSer129); (C) a merged photograph of A and B. D: The result of a food-sensing assay is shown. Sixty animals of each strain which was independently prepared were assayed. Data are indicated by mean ± standard error. For statistical analysis, a two-sided Student's t-test was conducted. There was a significant difference (P<0.05) between transgenic nematodes coexpressing wildtype GRK5 and α-synuclein, and transgenic nematodes coexpressing K215R mutant GRK5 and α-synuclein, both of which were coincided in terms of the growth stages. * The genotypes of GRK5 and α-synuclein are indicated under the graph: WT represents the wildtype; and M represents the mutant. N2 represents a wildtype nematode (without gene transfer), and cat-2 represents a strain of nematode having a mutation causing dopamine synthesis deficiency. The numbers beginning with Is represent strains of nematodes. The difference in the numbers indicates that the strains were independently prepared even when they had the same genotype pair. E: The result of a food-sensing assay which was conducted after dopamine administration is shown.

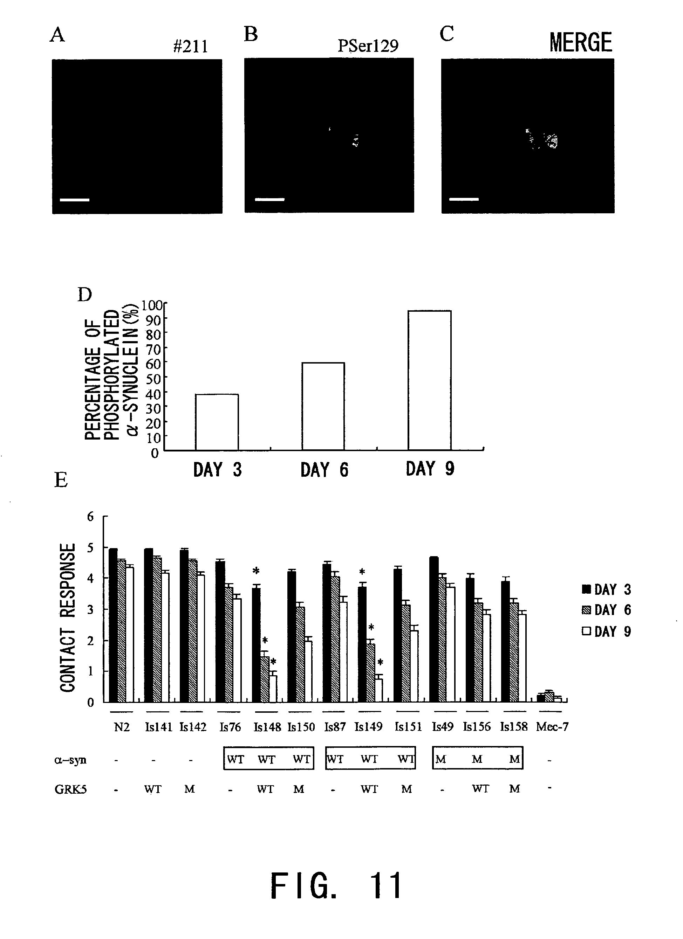

Fig. 11 is a set of diagrams and photographs demonstrating that GRK5 phosphorylates Ser-129 of α-synuclein in touch neurons and that the phosphorylation of α-synuclein by GRK5 causes mechanosensory defect. A to C: The phosphorylation of Ser-129 of α-synuclein by GRK5 in touch neurons was observed with a confocal microscope. (A) anti-α-synuclein antibody (#211); (B) anti-phosphorylated α-synuclein antibody (PSer129); (C) merged photograph of A and B
   D: Percentages of phosphorylated α-synuclein-positive neurons in PLM neurons in a strain of transgenic nematode coexpressing wildtype GRK5 and α-synuclein are shown. E: The result of a touch assay is shown. Sixty animals of each strain which was independently prepared were assayed. Data are indicated by mean ± standard error. For statistical analysis, a two-sided Student's t-test was conducted. There was a significant difference (P<0.05) between transgenic nematode coexpressing wildtype GRK5 and α-synuclein, and transgenic nematode coexpressing K215R mutant GRK5 and α-synuclein, both of which were coincided in terms of the growth stages. * The genotypes of GRK5 and α-synuclein are indicated under the graph: WT represents the wildtype; and M represents the mutant. N2 represents a wildtype nematode (without gene transfer); and cat-2 represents a strain of nematode having a mutation causing dopamine synthesis deficiency. The numbers beginning with Is represent strains of nematodes. The difference in the numbers indicates that the strains were independently prepared even when they have the same genotype pair.

### Best Mode for Carrying Out the Invention

The present inventors identified the GRK5 gene (GenBank Accession Number: NM_005308) as a gene associated with Parkinson's disease (PD). They further identified polymorphic mutations involved in the elevated expression of the gene.

Specifically, the elevated expression of the GRK5 gene was demonstrated to be strongly associated with the onset of PD. This then enables the assessment of a subject as having a predisposition to PD (a susceptibility to PD) when the expression of the GRK5 gene is enhanced in the subject.

The present invention initially provides methods of testing whether or not a subject is susceptible to PD, wherein the expression of GRK5 gene in the subject (a biological sample derived from the subject) is used as an index.

Accordingly, it is possible to determine whether or not the subject is susceptible to PD by using, as an index, the expression of GRK5 gene or the activity (function) of the protein encoded by the gene.

Information on the nucleotide sequence of the GRK5 gene in the present invention and the amino acid sequence of the protein encoded by the gene is easily accessible through the above-mentioned GenBank Accession Number. One skilled in the art can readily obtain information on the nucleotide sequence of the gene, and the amino acid sequence of the protein encoded by the gene from a public gene database or document database based on the gene notation (gene name). The nucleotide sequence of a genomic DNA region of the GRK5 gene is shown in SEQ ID NO: 1.

The term "subject" as used in the context of present invention generally refers to a human; however, the testing method of the present invention is not necessarily limited to methods which utilize only a human subjects for examination. When a subject is a non-human organism (preferably a mammal, and more preferably a vertebrate such as a mouse, rat, monkey, dog, or cat), the subject may be tested using, as an index, the expression level of an endogenous gene the subject organism corresponding to the GRK5 gene. Accordingly, the "GRK5 gene" in the present invention includes, for example, endogenous DNAs (e.g., GRK5 gene homologs) of other organisms, corresponding to the DNA having the nucleotide sequence of SEQ ID NO: 1.

Such endogenous DNAs of other organisms corresponding to the DNA having the nucleotide sequence of SEQ ID NO: 1 generally show a high homology to DNA of SEQ ID NO: 1. The term "high homology" means a homology of 50% or more, preferably 70% or more, more preferably 80% or more, furthermore preferably 90% or more (for example, 95% or more, particularly preferably 96%, 97%, 98%, or 99% or more). The homology can be determined using mBLAST algorithm (Altschul et al. (1990) Proc. Natl. Acad. Sci. USA 87:2264-8; Karlin and Altschul (1993) Proc. Natl. Acad. Sci. USA 90: 5873-7). When such DNAs are isolated from a living body, they are considered to hybridize with the DNA of SEQ ID NO: 1 under stringent conditions. The "stringent conditions" herein include, for example, "2x SSC, 0.1% SDS, 50°C", "2x SSC, 0.1% SDS, 42°C", and "1x SSC, 0.1% SDS, 37°C"; and more stringent conditions include "2x SSC, 0.1% SDS, 65°C", "0.5x SSC, 0.1% SDS, 42°C", and "0.2x SSC, 0.1% SDS, 65°C". One skilled in the art can suitably obtain endogenous genes in other organisms corresponding to the GRK5 gene, based on the nucleotide sequence of the GRK5 gene.

The present invention further provides methods of testing whether or not a subject is susceptible to PD, wherein the subject deemed to be susceptible to PD when the expression level of GRK5 gene in the subject is elevated as compared with a control.

In the above method, a biological sample derived from the subject is generally used as a test sample. The expression level of the GRK5 gene in the test sample can be suitably measured using procedures known to one skilled in the art.

The term "expression" in the context of the "gene" includes "transcription" from the gene and "translation" into a polypeptide.

When the expression level of the gene is measured using as an index the amount of a translated product (protein) of the gene, for example, a protein sample can be prepared from a test sample, and the amount of GRK5 in the protein sample can be measured. Examples of such procedures include those known to one skilled in the art, such as enzyme-linked immunosorbent assay (ELISA), double monoclonal antibody sandwich immunoassay, monoclonal polyclonal antibody sandwich assay, immunofluorescence, Western blotting, dot blotting, immunoprecipitation, protein chip analysis (Tanpakushitsu Kakusan Koso (Protein, Nucleic Acid and Enzyme) Vol. 47 No. 5 (2002); Tanpakushitsu Kakusan Koso (in Japanese; Protein, Nucleic Acid and Enzyme) Vol. 47 No. 8 (2002)), two-dimensional electrophoresis, and SDS-polyacrylamide electrophoresis, but are not limited thereto.

When the expression level of the gene is measured using the amount of a transcript (mRNA) of the gene as an index, for example, an RNA sample can be prepared from a test sample, and the amount ofGRK5-encoding RNA contained in the RNA sample can be measured. In addition, the expression level can be evaluated by preparing a cDNA sample from the test sample and measuring the amount ofGRK5-encoding cDNA contained in the cDNA sample. The RNA sample and cDNA sample from the test sample can be prepared from a subject-derived biological sample using procedures known to one skilled in the art. Examples of such procedures include those known to one skilled in the art, such as Northern blotting, RT-PCR, and DNA array techniques.

The term "control" typically refers to the expression level of a GRK5 gene in a biological sample derived from a healthy individual. The term "expression" of the GRK5 gene as used in the present invention means both the expression of mRNA transcribed from the GRK5 gene, and the expression of a protein encoded by the GRK5 gene.

The present invention further provides methods of testing whether or not a subject is susceptible to PD, which include the step of detecting a mutation in the GRK5 gene of the subject.

The phrase "testing whether or not a subject is susceptible to Parkinson's disease (PD)" in the context of the present invention includes a test to determine whether the subject is more or less likely to suffer from PD. In the method of the present invention, when a mutation in the GRK5 gene is detected, the subject deemed to be susceptible or having a predisposition of susceptibility to PD (having a predisposition to PD). Alternatively, the subject can be characterized as being not resistant or having no predisposition to being resistant to PD.

On the other hand, when no mutation is detected in the GRK5 gene, the subject is deemed to be resistant or having a resistive predisposition to PD. Alternatively, the subject can be characterized as being not susceptible or having no predisposition of susceptibility to PD.

The method of the present invention can determine whether a subject who is not affected by PD is more or less likely to suffer from PD.

The term "treatment" as used herein generally means achieving a pharmacological and/or physiological effect. The effect may be preventive in that a disease or symptom is fully or partially prevented, or may be therapeutic in that a disease or symptom is fully or partially treated. The "treatment" as used herein includes all the disease treatments in mammals, particularly in humans. Furthermore, the "treatment" also includes preventing the onset of a disease in a subject who has a predisposition of the disease but has not yet been diagnosed as being affected by the disease, suppressing the progression of the disease, alleviating the disease, and delaying its onset.

PD often occurs in elderly persons and has an aspect of senile diseases. The condition of the disease gradually develops after the onset, and patients often become bedridden. This seriously impairs the QOL of the patients and their caregivers. Accordingly, the testing method of the present invention can determine the susceptibility of a patient for PD and allow the patient thus determined as susceptible to PD to select suitable treatments in advance and prevent the development of PD. The prevention or suppression of the progression of the disease is enabled by the methods of screening for pharmaceutical agents for treating PD and such agents provided by the present invention, thereby reducing the likelihood that an elderly person developing PD will become bedridden. Thus, QOL is expected to be markedly improved in both the patients and their caregivers, particularly if the period until the patient becomes bedridden is delayed.

The DNA sequence of the GRK5 gene in the present invention specifically includes the sequence of SEQ ID NO: 1. The sequence of SEQ ID NO: 1 was produced based on the result of mapping info (NCBI build34).

In the testing method of the present invention, it is difficult to preliminarily determine the position of a "mutation". Such mutations are generally present in ORF of the above-mentioned GRK5 gene, or in regions for regulating the expression of this gene (for example, promoter regions, enhancer regions, or introns), but the position is not limited thereto. Generally, the "mutation" preferably enhances the expression level of the above-mentioned gene, improves the stability of mRNA, or increases the activity of a protein encoded by the gene. Examples of types of mutations in the present invention include addition, deletion, substitution, and insertion of nucleotides.

The present invention relates to the successful discovery of polymorphic mutations in the GRK5 gene of subjects that are significantly associated with PD. Accordingly, it is possible to test whether or not a subject is susceptible to PD by using, as an index, the presence or absence of a mutation (by determining the nucleotide type) at a polymorphic site on the GRK5 gene.

A preferred embodiment of the present invention relates to a method of testing whether or not a subject is susceptible to PD, including the step of detecting a polymorphic mutation in the GRK5 gene of the present invention.

The term "polymorphism" in genetics is generally defined as a variation of a certain nucleotide in one gene, where the variation occurs at a frequency of 1% or more in a population. However, the "polymorphism" in the present invention is not restricted by this definition. Examples of the polymorphism in the present invention include single nucleotide polymorphisms, and polymorphisms in which one to several tens of nucleotides (occasionally several thousands of nucleotides) are deleted or inserted. In addition, the number of polymorphic site is not limited to one, and two or more polymorphisms may be present.

The present invention further provides methods for testing whether or not a subject is susceptible to PD, including the step of determining the nucleotide type at a polymorphic site in the GRK5 gene of the present invention.

In the method for testing whether or not a subject is susceptible to PD according to the present invention, the "polymorphic site" is not particularly limited so long as it is a polymorphism present on the GRK5 gene of the present invention or a peripheral region thereof. Information on polymorphic sites found on the GRK5 gene is shown in List 1. The items in List 1 (chromosome, position, position in SEQ ID NO: 1, rs ID, and type) are separated with slash (/). In the list, "SNP" represents a single nucleotide polymorphism, and "INDEL" represents an insertion/deletion mutation.

### [List 1]

Chromosome/Position/Position in the sequence (SEQ ID NO: 1)/rs ID/Type
chr10/120706285/1/rs11198856/SNP
chr10/120706320/36/rs10510056/SNP
chr10/120706320/36/Affymetrix399365/SNP
chr10/120706622/338/rs11198857/SNP
chr10/120707388/1104/rs11198858/SNP
chr10/120707671/1387/rs4752271/SNP
chr10/120708336/2052/rs12269098/SNP
chr10/120708545/2261/rs1198859/SNP
chr10/120709991/3707/rs11198860/SNP
chr10/120710452/4168/rs4752272/SNP
chr10/120711288/5004/rs7393379/SNP
chr10/120711918/5634/rs4752273/SNP
chr10/120712172/5888/rs4752274/SNP
chr10/120712327/6043/rs10886438/SNP
chr10/120712357/6073/rs4751708/SNP
chr10/120713501/7217/rs12264832/SNP
chr10/120713550/7266/rs10749311/SNP
chr10/120713666/7382/rs4752275/SNP
chr10/120713840/7556/rs12250478/SNP
chr10/120714152/7868/rs10886439/SNP
chr10/120714310/8026/rs10886440/SNP
chr10/120714406/8122/rs 11198861/SNP
chr10/120714432/8148/rs7097022/SNP
chr10/120714463/8179/rs11198862/SNP
chr10/120714605/8321/rs9325563/SNP
chr10/120714842/8558/rs4752277/SNP
chr10/120714895/8611/rs7097630/SNP
chr10/120715363/9079/rs11198863/SNP
chr10/120715387/9103/rs11198864/SNP
chr10/120716711/10427/rs4752278/SNP
chr10/120716745/10461/rs4752279/SNP
chr10/120716825/10541/rs4752280/SNP
chr10/120716949/10665/rs11312254/INDEL
chr10/120716953/10669/rs10712644/INDEL
chr10/120716960/10676/rs11341250/INDEL
chr10/120717175/10891/rs6585542/SNP
chr10/120717320/11036/rs7910100/SNP
chr10/120717495/11211/rs10128498/SNP
chr10/120718729/12445/rs4752281/SNP
chr10/120718965/12681/rs10886442/SNP
chr10/120719165/12881/rs10437460/SNP
chr10/120719200/12916/rs10886443/SNP
chr10/120719688/13404/rs2168222/SNP
chr10/120719745/13461/rs2168221/SNP
chr10/120720016/13732/rs11198867/SNP
chr10/120721366/15082/rs11815427/SNP
chr10/120721920/15636/rs4752282/SNP
chr10/120723046/16762/rs10886444/SNP
chr10/120723893/17609/rs7905833/SNP
chr10/120723894/17610/rs10532699/INDEL
chr10/120723903/17619/rs5788340/INDEL
chr10/120723910/17626/rs10589661/INDEL
chr10/120723921/17637/rs3981127/INDEL
chr10/120723934/17650/rs1889743/SNP
chr10/120724932/18648/rs12252114/SNP
chr10/120726262/19978/rs7904422/SNP
chr10/120726441/20157/rs7080426/SNP
chr10/120726655/20371/rs10886445/SNP
chr10/120726680/20396/rs11198868/SNP
chr10/120727294/21010/rs1473799/SNP
chr10/120727786/21502/rs4751709/SNP
chr10/120728177/21893/rs12256695/SNP
chr10/120728359/22075/rs7913639/SNP
chr10/120728383/22099/rs10637086/INDEL
chr10/120728428/22144/rs12412957/SNP
chr10/120728458/22174/rs7913438/SNP
chr10/120728727/22443/rs12249291/SNP
chr10/120729190/22906/rs12571005/SNP
chr10/120729307/23023/rs12416019/SNP
chr10/120729490/23206/rs10886446/SNP
chr10/120729666/23382/rs2420614/SNP
chr10/120729972/23688/rs915391/SNP
chr10/120729972/23688/Affymetrix399377/SNP
chr10/120730048/23764/rs10787948/SNP
chr10/120730447/24163/rs12572560/SNP
chr10/120730527/24243/rs1563287/SNP
chr10/120731697/25413/rs4752283/SNP
chr10/120731869/25585/rs11443309/INDEL
chr10/120732548/26264/rs2061079/SNP
chr10/120732785/26501/rs2061078/SNP
chr10/120732966/26682/rs11198869/SNP
chr10/120732967/26683/rs871199/SNP
chr10/120732971/26687/rs5788341/INDEL
chr10/120733110/26826/rs871200/SNP
chr10/120733321/27037/rs915392/SNP
chr10/120733403/27119/rs871198/SNP
chr10/120733632/27348/rs871197/SNP
chr10/120733661/27377/rs871196/SNP
chr10/120734133/27849/rs10886447/SNP
chr10/120734160/27876/rs11817583/SNP
chr10/120735097/28813/rs1563286/SNP
chr10/120735284/29000/rs11818150/SNP
chr10/120736139/29855/rs11198870/SNP
chr10/120736650/30366/rs11819686/SNP
chr10/120737321/31037/rs12414327/SNP
chr10/120737990/31706/rs11198871/SNP
chr10/12073 8248/31964/rs10787953/SNP
chr10/120738826/32542/rs915394/SNP
chr10/120739007/32723/rs2420615/SNP
chr10/120739272/32988/rs915393/SNP
chr10/1 20739348/33064/rs7907259/SNP
chr10/120739902/33618/rs11198872/SNP
chr10/120740173/33889/rs11198873/SNP
chr10/120740195/33911/rs10886454/SNP
chr10/120740278/33994/rs10886455/SNP
chr10/120741083/34799/rs2420616/SNP
chr10/120741457/35173/rs5788342/INDEL
chr10/120741472/35188/rs5788344/INDEL
chr10/120741473/3 5189/rs5788345/INDEL
chr10/120741474/35190/rs5788346/INDEL
chr10/120741524/35240/rs3916271/SNP
chr10/120741568/35284/rs4620640/SNP
chr10/120741686/35402/rs1556715/SNP
chr10/120741699/35415/rs12265216/SNP
chr10/120741778/35494/rs1556714/SNP
chr10/120741806/35522/rs4751710/SNP
chr10/120742689/36405/rs4752284/SNP
chr10/120742717/36433/rs4752285/SNP
chr10/120742733/36449/rs4752286/SNP
chr10/120742830/36546/rs4752287/SNP
chr10/120743101/36817/rs11198874/SNP
chr10/120743152/36868/rs4752288/SNP
chr10/120743166/36882/rs11198875/SNP
chr10/120743288/37004/rs4531379/SNP
chr10/120743432/37148/rs4237511/SNP
chr10/120743579/37295/rs4752289/SNP
chr10/120743697/37413/rs7894210/SNP
chr10/120744006/37722/rs1556713/SNP
chr10/120744677/38393/rs10886457/SNP
chr10/120745466/39182/rs9887987/SNP
chr10/120745477/39193/rs1556712/SNP
chr10/120745585/39301/rs1556711/SNP
chr10/120745600/39316/rs1556710/SNP
chr10/120745740/39456/rs4562727/SNP
chr10/120745771/39487/rs1556709/SNP
chr10/120745834/39550/rs10886458/SNP
chr10/120745922/39638/rs12244191/SNP
chr10/120746381/40097/rs7091519/SNP
chr10/120746418/40134/rs3929218/SNP
chr10/120746477/40193/rs1563285/SNP
chr10/120747433/41149/rs11198878/SNP
chr10/120747441/41157/rs9887998/SNP
chr10/120747712/41428/rs11198879/SNP
chr10/120747782/41498/rs7096856/SNP
chr10/120747902/41618/rs11599133/SNP
chr10/120747951/41667/rs11599102/SNP
chr10/120748127/41843/rs10886459/SNP
chr10/120748648/42364/rs9325564/SNP
chr10/120748702/42418/rs6585543/SNP
chr10/120749301/43017/rs4752291/SNP
chr10/120749321/43037/rs1020672/SNP
chr10/120749474/43190/rs10886460/SNP
chr10/120750884/44600/rs11198880/SNP
chr10/120750891/44607/rs897311/SNP
chr10/120751806/45522/rs11198881/SNP
chr10/120752282/45998/rs7086215/SNP
chr10/120754568/48284/rs1378273/SNP
chr10/120756255/49971/rs11198883/SNP
chr10/120756406/50122/rs1198884/SNP
chr10/120757157/50873/rs4751711/SNP
chr10/120757747/51463/rs10886461/SNP
chr10/120759228/52944/rs10523265/INDEL
chr10/120759507/53223/rs7918145/SNP
chr10/120759681/53397/rs11814921/SNP
chr10/120759987/53703/rs3740563/SNP
chr10/120760047/53763/rs7098836/SNP
chr10/120760551/54267/rs7907709/SNP
chr10/120761032/54748/rs7069375/SNP
chr10/120761066/54782/rs11198885/SNP
chr10/120761078/54794/rs7894773/SNP
chr10/120761423/55139/rs10787955/SNP
chr10/120761741/55457/rs7070459/SNP
chr10/120761911/55627/rs7070986/SNP
chr10/120762538/56254/rs10787956/SNP
chr10/120762877/56593/rs11198886/SNP
chr10/120763622/57338/rs531344/SNP
chr10/120763890/57606/rs12219911/SNP
chr10/120763932/57648/rs11198887/SNP
chr10/120763972/57688/rs516627/SNP
chr10/120764127/57843/rs12358775/SNP
chr10/120764262/57978/rs11198888/SNP
chr10/120764351/58067/rs2252545/SNP
chr10/120764740/58456/rs4752292/SNP
chr10/120764900/58616/rs12261720/SNP
chr10/120765547/59263/rs291975/SNP
chr10/120766192/59908/rs11384740/INDEL
chr10/120766285/60001/rs4752293/SNP
chr10/120766346/60062/rs291974/SNP
chr10/120766922/60638/rs291973/SNP
chr10/120766985/60701/rs2124171/SNP
chr10/120767059/60775/rs2841339/SNP
chr10/120767289/61005/rs11198889/SNP
chr10/120767484/61200/rs915114/SNP
chr10/120767886/61602/rs2085185/SNP
chr10/120768427/62143/rs291972/SNP
chr10/120768838/62554/rs7078834/SNP
chr10/120768976/62692/rs10556176/INDEL
chr10/120769024/62740/rs10556177/INDEL
chr10/120769030/62746/rs11284334/INDEL
chr10/120769033/62749/rs10556178/INDEL
chr10/120769058/62774/rs7082781/SNP
chr10/120769336/63052/rs4752294/SNP
chr10/120769530/63246/rs11198890/SNP
chr10/120769898/63614/rs10886462/SNP
chr10/120769940/63656/rs10653418/INDEL
chr10/120769941/63657/rs11402248/INDEL
chr10/120770271/63987/rs4751712/SNP
chr10/120770668/64384/rs10082517/SNP
chr10/120770736/64452/rs184117/SNP
chr10/120770781/64497/rs864413/SNP
chr10/120770880/64596/rs7912372/SNP
chr10/120770994/64710/rs2420617/SNP
chr10/120771207/64923/rs7092272/SNP
chr10/120771927/65643/rs291966/SNP
chr10/120772034/65750/rs11198891/SNP
chr10/120772207/65923/rs11198892/SNP
chr10/120772487/66203/rs11198893/SNP
chr10/120773268/66984/rs4751713/SNP
chr10/120773282/66998/rs184116/SNP
chr10/120775282/68998/rs7095594/SNP
chr10/1207753 72/69088/rs915113/SNP
chr10/120776332/70048/rs7915016/SNP
chr10/120776914/70630/rs 12415832/SNP
chr10/120776967/70683/rs11594033/SNP
chr10/120778035/71751/rs7084292/SNP
chr10/120778495/72211/rs1671440/SNP
chr10/120778559/72275/rs2085184/SNP
chr10/120778879/72595/rs10886464/SNP
chr10/120778989/72705/rs12254227/SNP
chr10/120779882/73598/rs5788347/INDEL
chrl0/120779894/73610/rs5788348/INDEL
chr10/120780261/73977/rs11198896/SNP
chr10/120782092/75808/rs7085421/SNP
chr10/120782638/76354/rs11198897/SNP
chr10/120783079/76795/rs11198898/SNP
chr10/120783366/77082/rs7074722/SNP
chr10/120783681/77397/rs11593975/SNP
chr10/120783860/77576/rs291976/SNP
chr10/120784589/78305/rs11353327/INDEL
chr10/120785755/79471/rs7101022/SNP
chr10/120785820/79536/rs10787958/SNP
chr10/120785831/79547/rs4752295/SNP
chr10/120785882/79598/rs1248079/SNP
chr10/120785999/79715/rs4752296/SNP
chr10/120786057/79773/rs1671438/SNP
chr10/120788190/81906/rs291971/SNP
chr10/120788220/81936/rs291970/SNP
chr10/120789088/82804/rs884970/SNP

Among the above, polymorphic sites that can be used in the context of the method for testing whether or not a subject is susceptible to PD according to the present invention are those on the GRK5 gene or a region around the gene, and are preferably any one of position 27377 (m22), position 34799 (m22.1), position 54748 (m23), and position 60001 (m24) in the nucleotide sequence of SEQ ID NO: 1 (the above polymorphic sites are also simply referred to herein as "polymorphic sites of the present invention").

One skilled in the art can suitably obtain information regarding the particular nucleotides at the above sites based on the above-listed rs numbers for the dbSNP database. Regarding SNP IDs, those with "rs" at the head are registration IDs in the dbSNP database that have been uniquely assigned to respective single sequences by NCBI. The dbSNP database is publicly available on a web site (http://www.ncbi.nlm.nih.gov/SNP/index.html), and detailed information on SNPs in a nucleotide sequence (for example, position on a chromosome, nucleotide type at polymorphic site, and adjacent sequences) can be obtained by conducting search on the web site using a registration ID number stated in an SNP ID. By using the information, one skilled in the art can easily perform the test of the present invention.

Generally, one skilled in the art can easily find the actual genomic position, adjacent sequences and such of the polymorphic sites of the present invention using the registration IDs given to the polymorphisms disclosed herein, such as the rs numbers in the dbSNP database. If this information cannot be found by such means, one skilled in the art can readily find the actual genomic position corresponding to the polymorphic site based on the sequence of SEQ ID NO: 1 and information on the polymorphic site or such. For example, the genomic position of the polymorphic site of the present invention can be determined by consulting a public genome database or such. Specifically, even when the nucleotide sequences are slightly different between the nucleotide sequence in the sequence listing and the actual genomic nucleotide sequence, the actual genomic position of the polymorphic site of the present invention can be precisely identified by, for example, conducting a homology search of the genomic sequence based on the nucleotide sequence in the sequence listing. Even when the genomic position cannot be identified, the test of the present invention can be easily conducted based on the sequence listing and the information on the polymorphic sites disclosed herein.

Genomic DNA usually has a mutually complementary double-stranded DNA structure. Accordingly, even when the DNA sequence of one strand is disclosed herein for the sake of convenience, it will be naturally understood that the other sequence complementary to the above sequence (nucleotides) is also disclosed. When a DNA sequence (nucleotides) in one strand is known, the other sequence (nucleotides) complementary to the above sequence (nucleotides) is obvious to one skilled in the art. Regarding the human genome sequence, the International Human Genome Project build34, which is said to be an almost final version, has been published, and the sequences and the like described herein are based on the results of the International Human Genome Project build34.

In the methods of testing whether or not a subject is susceptible to PD according to the present invention, the following polymorphic sites are preferably tested:
(1) any of polymorphic sites on the GRK5 gene located at position 27377 (m22), position 34799 (m22.1), 54748 position (m23), and position 60001 (m24) in the nucleotide sequence of SEQ ID NO: 1 are preferable.

In a more preferred embodiment of the present invention, the polymorphic sites of (1) and/or (2) below are tested in the methods of testing whether or not a subject is susceptible to PD:
(1) a polymorphic site on the GRK5 gene located at position 34799 in the nucleotide sequence of SEQ ID NO: 1; and
(2) a polymorphic site on the GRK5 gene located at position 60001 in the nucleotide sequence of SEQ ID NO: 1.

A subject is deemed to be susceptible to PD when the nucleotide types in the above polymorphic sites (1) and (2) are (1b) and (2b) described below, respectively. The susceptibility to PD can be determined regardless of whether the subject is suffering from PD.

The testing method in which a subject deemed to be susceptible to PD when the nucleotides are (1b) and (2b).
(1b) the nucleotide at a polymorphic site on the GRK5 gene located at position 34799 in the nucleotide sequence of SEQ ID NO: 1 is G; and
(2b) the nucleotide at a polymorphic site on the GRK5 gene located at position 60001 in the nucleotide sequence of SEQ ID NO: 1 is C.

In addition to the above polymorphic sites, the susceptibility to PD can also be tested in the present invention by determining the nucleotide present at polymorphic sites near the above polymorphic sites, because the above polymorphic sites are considered to be firmly linked with adjacent DNA regions. Specifically, the nucleotide of an "adjacent polymorphic site" (for example, a polymorphic site listed in List 1 above) is determined beforehand in a human subgroup that includes PD patients in which the nucleotide at the polymorphic sites are (1b) and (2b) as above.

Next, the susceptibility of a subject to PD can be tested by determining the nucleotides of the "adjacent polymorphic site" in the subject and comparing it with the previously-determined nucleotides. When the nucleotide of the subject is identical to the previously-determined nucleotide, the subject deemed to be susceptible to PD. The testing method of the present invention can determine the susceptibility of a subject to PD, and the result can be utilized, for example, in deciding courses of treatment and agent doses.

For example, in a human subgroups including persons that has developed PD in which the nucleotide at a polymorphic site on the GRK5 gene at position 34799 in the nucleotide sequence of SEQ ID NO: 1 is G, the nucleotide at an adjacent polymorphic site, such as position 33994, is determined. When the frequency that the nucleotide of this site is A is higher in the persons with PD than in persons who do not suffer from PD, a subject is tested for the nucleotide at the polymorphic site of position 33994, and determined as susceptible (having a predisposition of susceptibility) to PD if the nucleotide type of this site is also A.

As described above, the discovery of genetic regions associated with PD by the present invention allows those skilled in the art to test the susceptibility to PD without undue burden.

As the human genome analysis progresses, information on whole nucleotide sequences and polymorphisms such as SNPs, microsatellites, VNTRs, RFLPs has been enriched. While the genomic nucleotide sequences are now being revealed in detail, the current top concern is to analyze a relationship between a gene or a specific sequence and a function (phenotype such as disease or disease progression). One of promising solutions to this is a genetic statistical analysis using haplotypes.

Human chromosomes are present in pairs and each pair is derived from mother and father. The term "haplotype" refers to a combination of the genotypes in either one of the pair of an individual, and shows how gene loci are arranged on a paternally- or maternally-derived chromosome. A child inherits one chromosome each from the father and mother. Thus, if no recombination occurred at the time of gametogenesis, , the genes on each chromosome would inevitably be transferred together to the child, namely, they would be linked to each other. However, since recombination does in fact occur upon meiosis, genes carried on one chromosome are not necessarily linked. Inversely, however, even when genetic recombination occurs, gene loci closely located in one chromosome are strongly linked.

When allele dependency is found by observing this phenomenon in a population, this is referred to as "linkage disequilibrium". For example, when three gene loci are observed and no linkage disequilibrium is seen among them, there expected to be 2³ different haplotypes, and the frequency of each haplotype is predicted from the frequencies of the gene loci. However, when a linkage disequilibrium is seen, there are only less than 2³ haplotypes, and their frequencies are different from those predicted.

It has recently been demonstrated that haplotypes are useful for analyzing linkage disequilibrium (Genetic Epidemiology 23:221-233), and further studies have been made. As a result, it has been revealed that the genome has portions susceptible or insusceptible to recombination, and that the portions that are inherited from ancestry to progeny as single regions (regions specified by haplotypes) are common to human races (Science 226, 5576:2225-2229). Specifically, there are tightly linked DNA regions, and these regions are generally called DNA blocks. In the present invention, the susceptibility to PD can also be tested by detecting the presence or absence of a DNA block that includes a polymorphic site of the present invention.

Namely, a preferred embodiment of the present invention provides a method of testing whether or not a subject is susceptible to PD, wherein the detection of the presence of a DNA block showing the following haplotype indicates that a subject is susceptible to PD:
(1') a haplotype in which nucleotides at polymorphic sites of positions 27377, 54748, and 60001 in the nucleotide sequence of SEQ ID NO: 1 are G, A, and C, respectively, wherein the polymorphic sites are located in the GRK5 gene.

In the present invention, the term "DNA block" refers to a portion (region) which shows an intense linkage disequilibrium among gene loci. If a DNA block associated with PD is found, the susceptibility to PD can be tested by detecting the DNA block.

If (a DNA block showing) a haplotype associated with PD is found, the susceptibility to PD can be tested by detecting the haplotype. As a result of dedicated research, haplotypes associated with the susceptibility to PD were successfully discovered and disclosed herein.

Accordingly, the present invention provides methods of testing a subject for the susceptibility to PD, which includes the step of detecting (a DNA block showing) a haplotype which is associated with PD and is present on the GRK5 gene.

The present method can determine whether or not a subject is susceptible to PD by detecting a "haplotype associated with PD" in the subject. These determinations can be used, for example, in deciding courses of treatment.

The "(DNA block showing) haplotype associated with PD" includes, for example, the following (DNA block showing) haplotype:
(1') a haplotype in which nucleotides at polymorphic sites of positions 27377, 54748, and 60001 in the nucleotide sequence of SEQ ID NO: 1 are G, A, and C, respectively, wherein the polymorphic sites are located in the GRK5 gene.

A preferred embodiment of the above-mentioned method is a method of testing a subject for the susceptibility to PD, including the following steps (a) and (b):
(a) determining the nucleotide at a polymorphic site in the GRK5 gene of a subject; and
(b) determining the subject to be susceptible to PD when the nucleotide determined in step (a) is the same as the nucleotide at the above polymorphic site on the GRK5 gene that shows the haplotype of (1'):
   (1') a haplotype in which nucleotides at polymorphic sites on the GRK5 gene located at positions 27377, 54748, and 60001 of the nucleotide sequence of SEQ ID NO: 1 are G, A, and C, respectively.

Examples of the polymorphic site in step (a) include the polymorphic sites listed in List 1 above. Preferred polymorphic sites are as follows:
(1) any polymorphic sites of positions 27377, 27849, 27876, 28813, 29000, 29855, 30366, 31037, 31706, 31964, 32542, 32723, 32988, 33064, 33618, 33889, 33911, 33994, 34799, 35173, 35188, 35189, 35190, 35240, 35284, 35402, 35415, 35494, 35522, 36405, 36433, 36449, 36546, 36817, 36868, 36882, 37004, 37148, 37295, 37413, 37722, 38393, 39182, 39193, 39301, 39316, 39456, 39487, 39550, 39638, 40097, 40134, 40193, 41149, 41157, 41428, 41498, 41618, 41667, 41843, 42364, 42418, 43017, 43037, 43190, 44600, 44607, 45522, 45998, 48284, 49971, 50122, 50873, 51463, 52944, 53223, 53397, 53703, 53763, 54267, 54748, 54782, 54794, 55139, 55457, 55627, 56254, 56593, 57338, 57606, 57648, 57688, 57843, 57978, 58067, 58456, 58616, 59263, 59908, and 60001.

Preferably, the polymorphic site in step (a) above includes, for example, the following polymorphic site (1) or (2):
(1) a polymorphic site on the GRK5 gene located at position 34799 in the nucleotide sequence of SEQ ID NO: 1; and
(2) a polymorphic site on the GRK5 gene located at position 60001 in the nucleotide sequence of SEQ ID NO: 1.

The nucleotide at a polymorphic site of the present invention can be determined by one skilled in the art using various methods. For example, it can be determined by directly determining the nucleotide sequence of a DNA having a polymorphic site of the present invention.

Typically, a test sample to be subjected to the testing methods of the present invention is preferably a biological sample collected from a subject beforehand. The biological sample may include, for example, a DNA sample. In the present invention, such DNA samples can be prepared based on chromosomal DNA or RNA extracted from, for example, the blood, skin, oral mucosa, a tissue or cell collected or excised by surgery, or a body fluid collected for testing or such, of the subject.

Thus, in the methods of the present invention, a subject-derived biological sample (a biological sample obtained from the subject beforehand) is subjected as a test sample to the test.

One skilled in the art can prepare a biological sample suitably using known techniques. For example, DNA samples can be prepared, for example, by PCR using chromosomal DNA or RNA as a template and primers that hybridize to a DNA having a polymorphic site of the present invention.

Next, the nucleotide sequence of isolated DNA is determined. The nucleotide sequence of the isolated DNA can be easily determined by one skilled in the art using, for example, a DNA sequencer.

Variations in the nucleotides at the polymorphic sites of the present invention are normally already known. The phrase "determining the nucleotide" in the present invention does not necessarily mean determining whether the nucleotide at a certain polymorphic site is A, G, T, or C. For example, when the nucleotide variations at a certain polymorphic site are known to be A or G, it is only necessary to find that the nucleotide of the site is "not A" or "not G".

There are a variety of known methods for determining the nucleotide of a polymorphic site whose nucleotide variations are known. The method for determining the nucleotide in the present invention is not particularly limited. For example, TaqMan PCR, AcycloPrime, and MALDI-TOF/MS are in practical use as analysis methods using PCR. In addition, the Invader method and RCA method are known as non-PCR-dependent methods for determining nucleotide types. The nucleotide can also be determined using DNA arrays. These methods will be briefly illustrated below. Any of these processes can be applied to the determination of the nucleotide of a polymorphic site in the present invention.

### [TaqMan PCR]

The principle of TaqMan PCR is as follows. TaqMan PCR is an analysis method using a TaqMan probe and a set of primers that can amplify a region containing an allele. The TaqMan probe is designed to hybridize with the region containing the allele, which is amplified by the set of primers.

When the TaqMan probe is allowed to hybridize with a target nucleotide sequence under a condition near the Tm of the TaqMan probe, the hybridization efficiency of the TaqMan probe is significantly lowered due to the difference in a single nucleotide. When PCR is conducted in the presence of the TaqMan probe, elongation from the primers reaches the hybridized TaqMan probe in due course. Then, the TaqMan probe is decomposed from its 5' end by the 5'-3' exonuclease activity of DNA polymerase. By labeling the TaqMan probe with a reporter dye and a quencher, the decomposition of the TaqMan probe can be traced as a change in fluorescent signal. Specifically, when the TaqMan probe is decomposed, the reporter dye is released away from the quencher and thereby generates a fluorescence signal. When the hybridization of the TaqMan probe is reduced due to the difference in a single nucleotide, the decomposition of the TaqMan probe does not proceed, and a fluorescence signal is not generated.

Multiple nucleotide types can be determined simultaneously by designing TaqMan probes corresponding to a polymorphism and further modifying them so that the decomposition of each probe produces a different signal. For example, 6-carboxy-fluorescein (FAM) and VIC are used as reporter dyes for TaqMan probes for allele A and allele B, respectively, in a given allele. The generation of fluorescence signals by the reporter dyes are inhibited by a quencher when the probes are not decomposed. When each probe hybridizes with the corresponding allele, a fluorescence signal is observed upon the hybridization. Specifically, when the signal of either FAM or VIC is more intense than the other, the allele is found to be a homozygote of allele A or allele B. On the other hand, when the allele is a heterozygote of allele A and allele B, the two signals are to be detected at substantially identical levels. By using the TaqMan PCR, PCR and the determination of nucleotide type can be simultaneously conducted using a genome as an analysis target without a time-consuming step like separation on a gel. Accordingly, the TaqMan PCR is useful as a method capable of determining nucleotide types of many subjects.

### [AcycloPrime]

AcycloPrime is also in practice use as a method of determining a nucleotide using PCR. AcycloPrime uses one pair of primers for genome amplification, and one primer for polymorphism detection. Initially, a genomic region containing a polymorphic site is amplified by PCR. This step is the same as a regular genomic PCR. Next, the resultant PCR product is annealed with a primer for detecting SNPs, and an elongation reaction is conducted. The primer for detecting SNPs is so designed as to be annealed with a region adjacent to the polymorphic site to be detected.

In this step, a nucleotide derivative (terminator), which is labeled with a fluorescence polarization dye and is blocked at its 3'-OH, is used as a nucleotide substrate for elongation. As a result, only one complementary nucleotide is incorporated at the nucleotide at a position corresponding to the polymorphic site, and the elongation reaction is terminated. The incorporation of the nucleotide derivative to the primer can be detected by an increase of fluorescence polarization (FP) due to the increase of molecular weight. When two labels having different wavelengths are used as fluorescence polarization dyes, it is possible to determine whether a particular SNP is either of two nucleotides. Since the level of fluorescence polarization can be quantified, a single analysis can also determine whether an allele is a homozygote or heterozygote.

### [MALDI-TOF/MS]

The nucleotide type can also be determined by analyzing a PCR product through MALDI-TOF/MS. The MALDI-TOF/MS can quantify molecular weights very accurately. Thus, it is used in a variety of fields as an analysis method that can distinguish a slight difference in the amino acid sequence of a protein and the nucleotide sequence of a DNA. To determine a nucleotide through MALDI-TOF/MS, a region containing an allele to be analyzed is initially amplified by PCR. Next, an amplified product is isolated, and the molecular weight thereof is measured using MALDI-TOF/MS. Since the nucleotide sequence of the allele is already known, the nucleotide sequence of the amplified product is uniquely determined based on the molecular weight.

The determination of a nucleotide using MALDI-TOF/MS requires the separation of a PCR product and such. However, this technique is expected to enable accurate determination of a nucleotide without using labeled primers and probes. This technique can also be applied to simultaneous detection of polymorphisms at multiple sites.

### [SNP-specific Labeling Method Using Type IIs Restriction Enzymes]

Methods that can determine a nucleotide type more rapidly using PCR have been reported. For example, the nucleotide at a polymorphic site can be determined using a type IIs restriction enzyme. This method uses a primer having a type IIs restriction enzyme-recognition sequence in PCR. Common restriction enzymes (type II), which are used in gene recombination, recognize a specific nucleotide sequence and cleaves a specific site in the nucleotide sequence. In contrast, type Ils restriction enzymes recognize a specific nucleotide sequence and cleaves a site away from the recognized nucleotide sequence. The number of nucleotides between the recognized sequence and the cleaving site depends on each enzyme. Accordingly, the amplified product can be cleaved exactly at the polymorphic site by a type IIs restriction enzyme when a primer that contains a recognition sequence of the type IIs restriction enzyme is allowed to anneal at a position away from the polymorphic site by the specific number of nucleotides.

A cohesive end containing a SNP nucleotide is formed at an end of the amplified product cleaved by the type Ils restriction enzyme. Then, adaptors having a nucleotide sequence corresponding to the cohesive end of the amplified product are ligated. The adaptors include different nucleotide sequences containing nucleotides corresponding to polymorphic mutations, and they can be labeled with different fluorescent dyes in advance. Finally, the amplified product is labeled with a fluorescent dye corresponding to the nucleotide of the polymorphic site.

When PCR is conducted using a primer having a type IIs restriction enzyme-recognition sequence in combination with a capture primer, the amplified product can be fluorescently labeled and then immobilized to a solid phase using the capture primer. For example, when a biotin-labeled primer is used as the capture primer, the amplified product can be captured by avidin-linked beads. The nucleotide can be determined by tracing the fluorescence dye of the amplified product thus captured.

### [Determination of Nucleotide Type at Polymorphic Site Using Magnetic Fluorescence Beads]

There are also known techniques capable of analyzing plural alleles in parallel in a single reaction system. Analyzing multiple alleles in parallel is called "multiplexing". In typing methods using fluorescent signals, fluorescent elements having different fluorescence wavelengths are necessary for multiplexing. However, not so many fluorescent elements are available in actual analyses. In contrast, when multiple fluorescent elements are mixed with resins or such, even limited kinds of fluorescent elements can yield various fluorescence signals distinguishable from each other. In addition, it is possible to prepare magnetically-separable beads that emit fluorescence by adding a magnetically-adsorbable component to the resins. Multiplex polymorphism typing using such magnetic fluorescent beads has been developed (Baiosaiensu To Baioindasutorii (Bioscience & Bioindustry), Vol. 60 No. 12, 821-824).

In the multiplex polymorphism typing using magnetic fluorescent beads, probes having at their end a nucleotide complementary to a polymorphic site of each allele are immobilized to the magnetic fluorescent beads. The two components are combined so that each allele corresponds to each magnetic fluorescent bead with a unique fluorescence signal. On the other hand, fluorescently-labeled oligoDNA having a nucleotide sequence complementary to an region on the allele that is adjacent to the complementary sequence hybridized by the probe immobilized on the magnetic fluorescent bead, is prepared.

A region containing the allele is amplified by asymmetric PCR, and hybridized with the magnetic fluorescent bead-immobilized probe and the fluorescently-labeled oligoDNA, and the two are then ligated. When the end of the magnetic fluorescent bead-immobilized probe has a nucleotide sequence complementary to the nucleotide at the polymorphic site, they are efficiently ligated. Inversely, when the terminal nucleotide is different due to a polymorphism, The efficiency of the ligation between the two is lowered. As a result, the fluorescently-labeled oligoDNA binds with each magnetic fluorescent bead only when the sample has a nucleotide complementary to that of the magnetic fluorescent bead.

The nucleotide is determined by magnetically recovering the magnetic fluorescent beads, and detecting the presence of fluorescently-labeled oligoDNA on the magnetic fluorescent beads. The fluorescence signal can be analyzed for each one of the magnetic fluorescent beads using a flow cytometer. Thus, when a number of different magnetic fluorescent beads are mixed, the signals can be easily separated. Namely, the "multiplexing", in which a number of different polymorphic sites are analyzed in parallel in a single reaction vessel, is achieved.

### [Invader Method]

Non-PCR-dependent genotyping methods have also been in practical use. For example, the invader method achieves the determination of nucleotide types using only a special nuclease called "cleavase" and three different oligonucleotides: allele probe, invader probe, and FRET probe. Of these probes, only the FRET probe needs to be labeled.

The allele probe is designed to hybridize with a region adjacent to an allele to be detected. The 5'-side of the allele probe is linked with a flap having a nucleotide sequence not involved in hybridization. The allele probe has a structure such that it is hybridized with the 3'-side of a polymorphic site and is linked to the flap on the polymorphic site.

On the other hand, the invader probe includes a nucleotide sequence which hybridizes with the 5'-side of the polymorphic site. The nucleotide sequence of the invader probe is designed so that its 3'-end corresponds to the polymorphic site as a result of hybridization. The invader probe may have an arbitrary nucleotide at the position corresponding to the polymorphic site. Namely, the nucleotide sequences of the invader probe and the allele probe are designed so that they become adjacent to each other across the polymorphic site upon hybridization.

When the polymorphic site has a nucleotide complementary to the nucleotide sequence of the allele probe, both the invader probe and the allele probe hybridize with the allele and then form a structure in which the invader probe invades the nucleotide of the allele probe corresponding to the polymorphic site. In the oligonucleotides which have formed the invasion structure as above, the cleavase cleaves the strand that has been invaded. Since the cleavage occurs on the invasion structure, it results in removal of the flap of the allele probe. On the other hand, when the nucleotide of the polymorphic site is not complementary to the nucleotide of the allele probe, no competition occurs between the invader probe and the allele probe, and no invasion structure is formed. Accordingly, the flap is not cleaved by the cleavase.

The FRET probe is for detecting the flap thus separated. The FRET probe has a self-complementary sequence on its 5'-end side and forms a hairpin loop in which a single-stranded portion is present in its 3'-end side. The single-stranded portion in the 3'-end side of the FRET probe has a nucleotide sequence complementary to the flap, and the flap can hybridize with this portion. The nucleotide sequences of the flap and the FRET probe are designed so that the flap hybridizes with the FRET probe and forms a structure in which the 3'-end of the flap invades the 5'-end portion of the self-complementary sequence of the FRET probe. The cleavase recognizes and cleaves the invasion structure. By labeling the FRET probe with a reporter dye and a quencher, which are similar to those in the TaqMan PCR, at the positions that sandwich the region to be cleaved by the cleavase, the cleavage of the FRET probe can be detected as a change in fluorescence signal.

Theoretically, uncleaved flaps should also hybridize with the FRET probe. However, in fact, the FRET-binding efficiency is largely different between cleaved flaps and flaps in the form of allele probes. Accordingly, cleaved flaps can be specifically detected by using FRET probes.

To determine nucleotides based on the invader method, two different allele probes having nucleotide sequences complementary to allele A and allele B, respectively, may be prepared. In this case, the flaps of the two have different nucleotide sequences. By preparing two different FRET probes for detecting the flaps with distinguishable reporter dyes, nucleotides can be determined in the same manner as in TaqMan PCR.

An advantage of the invader process is that the labeling is necessary only to the oligonucleotide of the FRET probe. The oligonucleotide of the FRET probe may be the same regardless of nucleotide sequences to be detected. Accordingly, mass production is possible. On the other hand, the allele probe and the invader probe do not need to be labeled. After all, the reagents for genotyping can be produced at low cost.

### [RCA]

Non-PCR-dependent methods for determining nucleotide sequences include rolling circle amplification (RCA). The rolling circle amplification (RCA) is a process of amplifying DNA based on a reaction in which a DNA polymerase having a strand displacing activity synthesizes a long complementary strand using a cyclic single-stranded DNA as a template (Lizardri PM et al., Nature Genetics 19, 225, 1998). In the RCA, an amplification reaction is constituted using a primer which anneals to a cyclic DNA to initiate complementary strand synthesis, and a second primer which anneals to a long complementary strand formed by the former primer.

The RCA uses a DNA polymerase having a strand displacing activity. Accordingly, a double-stranded portion formed by the complementary strand synthesis is displaced as a result of a complementary strand synthesis reaction initiated by another primer annealed to a further 5' region. For example, a complementary strand synthesis reaction using cyclic DNA as a template does not complete by one cycle. The complementary strand synthesis continues while displacing a previously synthesized complementary strand, and produces a long single-stranded DNA. Meanwhile, the second primer anneals to the long single-stranded DNA produced from the template cyclic DNA, and initiates a complementary strand synthesis. In the RCA method, since the single-stranded DNA is produced using a cyclic DNA as a template, it has repeated nucleotide sequence of the identical nucleotide sequence. Accordingly, the continuous production of a long single strand leads to continuous annealing of the second primer. As a result, single-stranded portions to which the primer can anneal are continuously produced without a degeneration step. DNA amplification is thus achieved.

When cyclic single-stranded DNAs necessary for RCA are prepared depending on the nucleotide types of polymorphic sites, the nucleotide types can be determined using RCA. To this end, a padlock probe, which is a single linear strand, is used. The padlock probe has at its 5'- and 3'-ends nucleotide sequences that are complementary to both sides of a polymorphic site to be detected. These nucleotide sequences are linked by a portion called "backbone", which is composed of a special nucleotide sequence. If the polymorphic site has a nucleotide sequence complementary to the ends of the padlock probe, the ends that have hybridized to the allele can be ligated by a DNA ligase. As a result, the linear padlock probe is cyclized, and an RCA reaction is triggered. The efficiency of the DNA ligase reaction is significantly reduced when the ends to be ligated is not completely complementary. Accordingly, the nucleotide can be determined by detecting the presence or absence of the ligation using the RCA method.

The RCA method can amplify DNA, but does not yield a signal as it is. In addition, when only the presence or absence of amplification is used as an index, the reaction must be conducted for every allele to determine the nucleotide type. There are known methods in which these points are improved for nucleotide determination. For example, molecular beacons can be used to determine nucleotide types in a single tube based on the RCA method. The molecular beacon is a signal-generating probe using a fluorescent dye and a quencher as in the TaqMan method. The molecular beacon includes complementary nucleotide sequences at the 5'-end and 3'-end and forms a hairpin structure by itself. When the vicinities of the two ends are labeled with a fluorescent dye and a quencher, a fluorescence signal is not detectable from the molecular beacon forming a hairpin structure. The molecular beacon in which a part thereof is designed as a nucleotide sequence complementary to an RCA amplified product hybridizes to the RCA amplified product. The hairpin structure is resolved as a result of hybridization, and a fluorescence signal is produced.

An advantage of such molecular beacons is that a common nucleotide sequence can be used in the molecular beacons, regardless of subjects to be detected, by using the nucleotide sequence of the backbone portion of the padlock probe. When different backbone nucleotide sequences are used for different alleles, and two molecular beacons having different fluorescence wavelengths are used in combination, nucleotide types can be determined in a single tube. Since the cost for synthesizing fluorescently-labeled probes is high, it is an economical advantage that a common probe can be used regardless of subjects to be assayed.

These methods have been developed for rapid genotyping of a large quantity of samples. All methods but MALDI-TOF/MS generally require the preparation of labeled probes and such in any way. In contrast, nucleotide typing methods that do not depend on labeled probes and such have long been performed. Examples of such methods include methods based on restriction fragment length polymorphisms (RFLP) and the PCR-RFLP method.

The RFLP method is based on the fact that a mutation in a recognition site of a restriction enzyme, or an insertion or deletion of nucleotides in a DNA fragment yielded by restriction enzyme treatment, can be detected as a change in the size of the fragment formed after the restriction enzyme treatment. If there is a restriction enzyme that recognizes a nucleotide sequence having a polymorphism to be detected, the nucleotide at the polymorphic site can be identified according to the principle of RFLP.

Methods of detecting a difference in nucleotides using a change in the secondary structure of a DNA as an index are also known as methods requiring no labeled probe. PCR-SSCP is based on the fact that the secondary structures of single-stranded DNAs reflect differences in their nucleotide sequence (Cloning and polymerase chain reaction-single-strand conformation polymorphism analysis of anonymous Alu repeats on chromosome 11. Genomics. 1992 Jan 1; 12(1): 139-146., Detection of p53 gene mutations in human brain tumors by single-strand conformation polymorphism analysis of polymerase chain reaction products. Oncogene. 1991 Aug 1; 6(8): 1313-1318., Multiple fluorescence-based PCR-SSCP analysis with postlabeling., PCR Methods Appl. 1995 Apr 1; 4(5): 275-282.). The PCR-SSCP method is conducted by the steps of dissociating a PCR product into single-stranded DNAs, and separating them on a non-denaturing gel. Since the mobility of DNA on the gel varies depending on the secondary structure of single-stranded DNA, a nucleotide difference at the polymorphic site can be detected as a difference in mobility.

Another example of the methods requiring no labeled probe is denaturant gradient gel electrophoresis (DGGE). DGGE is a method in which a mixture of DNA fragments is electrophoresed in a polyacrylamide gel with a gradient of denaturant concentration, and the DNA fragments are separated depending on the difference in their instability. When an unstable DNA fragment having a mismatch moves to a position at a certain denaturant concentration in the gel, a DNA sequence around the mismatch is partially dissociated to single strands due to its instability. The mobility of the partially denatured DNA fragment is much slower and differs from that of a complete double-stranded DNA having no dissociated portion. Thus, the two fragments can be separated from each other.

Specifically, a region having a polymorphic site is initially amplified by PCR or such. A probe DNA with a known nucleotide sequence is allowed to hybridize with the amplified product to form a double-strand. This is electrophoresed in a polyacrylamide gel with a gradually increasing concentration of a denaturant such as urea, and is compared with a control. A DNA fragment that has a mismatch as a result of hybridization with the probe DNA is dissociated to single strands in a portion at a lower concentration of the denaturant and then shows a markedly slow mobility. The presence or absence of the mismatch can be detected by detecting the difference in mobility thus occurred.

In addition, nucleotides can also be determined by using DNA arrays (Saibo Kogaku Bessatsu "DNA Maikuroarei To Saishin PCR-ho" (Cell Technology Suppl., "DNA Microarray and Latest PCR Techniques"), Shujunsha Co., Ltd., published on April 20, 2000, pp.97-103 "OrigoDNA Chippu Niyoru SNP No Bunseki (SNP Analyses with OligoDNA Chips)", Shin-ichi Kajie). In a DNA array, a sample DNA (or RNA) is allowed to hybridize with many probes arrayed in one plate, and the plate is then scanned to detect the hybridizations with the probes. Since reactions on many probes can be observed simultaneously, such DNA arrays are useful, for example, to analyze many polymorphic sites simultaneously.

DNA arrays generally are generally composed of thousands of nucleotides densely printed on a substrate. Normally, these DNAs are printed on a surface layer of a non-porous substrate. The surface layer of the substrate is generally made of glass, but a porous membrane such as a nitrocellulose membrane can also be used.

In the present invention, an example of techniques for immobilizing (arraying) nucleotides is oligonucleotide-based arrays developed by Affymetrix, Inc. In oligonucleotide arrays, the oligonucleotides are generally synthesized *in vitro.* For example, *in situ* oligonucleotide synthesis methods are known, such as photolithographic techniques (Affymetrix, Inc.), and inkjet techniques for immobilizing chemical compounds (Rosetta Inpharmatics LLC). Any of these techniques can be used for preparing substrates used in the present invention.

The oligonucleotides are composed of nucleotide sequences complementary to regions containing SNPs to be detected. The length of nucleotide probes to bind with the substrate is, when they are oligonucleotides, generally 10 to 100 bases, preferably 10 to 50 bases, and more preferably 15 to 25 bases. In addition, the DNA array method generally uses a mismatch (MM) probe to avoid errors due to cross-hybridization (non-specific hybridization). The mismatch probe constitutes a pair with an oligonucleotide having a nucleotide sequence completely complementary to a target nucleotide sequence. The oligonucleotide that is composed of a nucleotide sequence completely complementary to a target nucleotide sequence is called a perfect match (PM) probe. The influence of cross-hybridization can be reduced by erasing signals observed with the mismatch probe in data analyzing processes.

Samples for genotyping by the DNA array method can be prepared based on biological samples collected from subjects, according to known methods to one skilled in the art. The biological samples are not particularly limited. For example, DNA samples can be prepared from chromosomal DNA extracted from tissues or cells such as blood, peripheral blood leucocytes, skin, or oral mucosa; tears, saliva, urine, faeces, or hair, of the subjects. A particular region of the chromosomal DNA is amplified using primers for amplifying a region having a polymorphic site to be determined. In this step, multiple regions can be amplified simultaneously by using multiplex PCR. The multiplex PCR is a PCR method in which multiple sets of primers are used in one reaction solution. The multiplex PCR is useful to analyze multiple polymorphic sites.

In the DNA array method, a DNA sample is amplified by PCR and the amplified product is labeled. Labeled primers are used for labeling the amplified product. For example, initially, a genomic DNA is amplified by PCR with a set of primers specific to a region containing a polymorphic site. Next, a biotin-labeled DNA is synthesized by labeling PCR using a biotin-labeled primer. The biotin-labeled DNA thus synthesized is allowed to hybridize with oligonucleotide probes on a chip. The reaction solution and conditions of hybridization can be suitably adjusted according to conditions such as the length of nucleotide probes immobilized on the substrate, and reaction temperature. One skilled in the art can appropriately design hybridization conditions. Fluorescent dye-labeled avidin is added to detect a hybridized DNA. The array is analyzed with a scanner, and the presence or absence of hybridization is detected using fluorescence as an index.

The above-mentioned process will be illustrated more specifically. Initially, DNA containing a polymorphic site of the present invention is prepared, and a solid phase to which nucleotide probes are immobilized is obtained. Next, the DNA is contacted with the solid phase. Further, DNA hybridized with the nucleotide probes immobilized on the solid phase is detected to determine the nucleotide type at the polymorphic site of the present invention.

The term "solid phase" as used in the context of the present invention refers to a material to which a nucleotide can be immobilized. The solid phase used in the present invention is not particularly limited, so long as a nucleotide can be immobilized thereto. Specific examples thereof include solid phases including microplate wells, plastic beads, magnetic particles, and substrates. In the present invention, a substrate generally used in DNA array techniques is preferably used as a solid phase. The term "substrate" in the present invention means a plate-like material to which a nucleotide can be immobilized. In the present invention, the nucleotide includes oligonucleotides and polynucleotides.

In addition to the above-mentioned methods, the allele-specific oligonucleotide (ASO) hybridization method can be used to detect a nucleotide at a specific site. The allele-specific oligonucleotide (ASO) is composed of a nucleotide sequence that hybridizes with a region containing a polymorphic site to be detected. When the ASO is hybridized with a sample DNA and a mismatch occurs at the polymorphic site due to a polymorphism, the hybridization efficiency is lowered. The mismatch can be detected by Southern blotting or a method using a special fluorescent reagent which is quenched when intercalating to a gap in a hybrid. The mismatch can also be detected by the ribonuclease A mismatch cleavage method.

Of the above-mentioned oligonucleotides, oligonucleotides that hybridize with DNA having any of the polymorphic sites described in (1) and have at least a 15-nucleotide chain length are usable as a reagent (testing agent) for testing whether a subject is susceptible to PD. These are used in tests in which the gene expression is used as an index, or tests using gene polymorphism as an index.

The oligonucleotides specifically hybridize with DNA having any of the above-mentioned polymorphic sites of (1) according to the present invention. The phrase "specifically hybridizes" as used herein means that significant cross-hybridizations do not occur with DNAs encoding other proteins under usual hybridization conditions, preferably under stringent hybridization conditions (for example, conditions stated by Sambrook et al., Molecular Cloning, Cold Spring Harbour Laboratory Press, New York, USA, 2nd Ed. 1989). When specific hybridization is possible, the oligonucleotide does not need to be completely complementary to the above-mentioned nucleotide sequence of (1) in a gene to be detected or in an adjacent DNA region of the gene.

Examples of hybridization conditions in the present invention include "2x SSC, 0.1 % SDS, 50°C", "2x SSC, 0.1% SDS, 42°C", and "1x SSC, 0.1% SDS, 37°C"; and as more stringent conditions, "2x SSC, 0.1% SDS, 65°C", "0.5x SSC, 0.1% SDS, 42°C", and "0.2x SSC, 0.1% SDS, 65°C". More specifically, as a process using the Rapid-hyb Buffer (Amersham Life Science), hybridization can be carried out by conducting prehybridization at 68°C for 30 minutes or more; adding probes to form hybrids while maintaining at 68°C for one hour or more; thereafter carrying out three times of washing in 2x SSC, 0.1 % SDS at room temperature for 20 minutes; subsequently carrying out three times of washing in 1x SSC, 0.1% SDS at 37°C for 20 minutes; and finally carrying out two times of washing in 1x SSC, 0.1% SDS at 50°C for 20 minutes. Hybridization can also be conducted, for example, by carrying out prehybridization in the Expresshyb Hybridization Solution (CLONTECH) at 55°C for 30 minutes or more; adding labeled probes and incubating at 37°C to 55°C for one hour or more; carrying out three times of washing in 2x SSC, 0.1% SDS at room temperature for 20 minutes; and carrying out washing once in 1x SSC, 0.1% SDS at 37°C for 20 minutes. More stringent conditions are available, for example, by setting the temperatures of prehybridization, hybridization and/or the second washing at higher levels. For example, the temperatures of prehybridization and hybridization can be set to 60°C, and, as more stringent conditions, to 68°C. One skilled in the art can set the conditions in consideration of, in addition to these conditions such as salt concentrations of buffers and temperatures, other conditions such as concentrations, lengths, and nucleotide sequence structures of probes, and reaction times.

The oligonucleotide can be used as a probe or primer in the above testing method according to the present invention. The length of the oligonucleotide, if used as a primer, is generally 15 bp to 100 bp, and preferably 17 bp to 30 bp. The primer is not particularly limited, so long as it can amplify at least a part of a DNA having any of the above-mentioned polymorphic sites of (1) of the present invention.

The present invention provides a primer for amplifying a region having a polymorphic site of the present invention, and a probe that hybridizes with a DNA region containing the polymorphic site.

Such primers for amplifying a region having the polymorphic site of the present invention also include primers that can initiate complementary strand synthesis toward the polymorphic site using as a template a DNA containing the polymorphic site. The primers can be described as primers for imparting an origin of replication to the 3' side of a polymorphic site in a DNA containing the polymorphic site. The distance between the polymorphic site and the region with which the primer hybridizes is arbitrary. As the distance between them, a suitable number of nucleotides can be selected according to the technique for analyzing the nucleotide at the polymorphic site. For example, when the primer is a primer for analysis using DNA chips, it is possible to design the primer to yield an amplification product having a length of 20 to 500, generally 50 to 200 nucleotides as a region that includes the polymorphic site. One skilled in the art can design a primer according to the analysis technique based on nucleotide sequence information on an adjacent DNA region containing the polymorphic site. The nucleotide sequence constituting the primer according to the present invention can be not only a nucleotide sequence completely complementary to a genomic nucleotide sequence but also suitable modifications thereof.

The primer according to the present invention can be added with arbitrary nucleotide sequences, in addition to nucleotide sequences complementary to a genomic nucleotide sequence. For example, primers added with a type IIs restriction enzyme-recognition sequence are used in primers for a method of analyzing polymorphisms using a type IIs restriction enzyme. Such primers with modified nucleotide sequences are also included in the primers for use in the present invention. In addition, the primers for use in the present invention can be modified. For example, primers labeled with fluorescent substance or substance with binding affinity, such as biotin or digoxin, are used in various genotyping methods. These modified primers are also included within the present invention.

On the other hand, the phrase "probe that hybridizes with a region containing a polymorphic site" in the present invention refers to probes that can hybridize with a polynucleotide that has a nucleotide sequence of a region containing a polymorphic site. More specifically, a probe having a polymorphic site in its nucleotide sequence is a preferably probe for use in the present invention. Alternatively, a probe may be designed to have an end corresponding to a nucleotide (base) adjacent to a polymorphic site in some methods of analyzing the nucleotide at the polymorphic site. Accordingly, a preferred probe in the present invention includes a probe which does not contain a polymorphic site in its nucleotide sequence but contains a nucleotide sequence complementary to a neighboring region to the polymorphic site.

In other words, a probe that can hybridize with a polymorphic site of the present invention on a genomic DNA or with a neighboring site to the polymorphic site is preferable as a probe for use in the present invention. Such probes according to the present invention can be subjected to alternations in nucleotide sequence, addition of nucleotide sequence, or modification, as in the primers. For example, probes for use in the Invader process are added with a nucleotide sequence which constitutes a flap and does not relate to the genome. Such probes are also included in the probe of the present invention, so long as they hybridize with a region containing a polymorphic site. The nucleotide sequence constituting a probe according to the present invention can be designed according to the analysis method based on nucleotide sequence of a DNA region neighboring the present invention's polymorphic site on the genome.

Primers or probes according to the present invention can be synthesized by arbitrary methods based on the nucleotide sequence constituting the same. In a primer or probe according to the present invention, the length of a nucleotide sequence complementary to a genomic DNA is usually 15 to 100, generally 15 to 50, and usually 15 to 30. Procedures of synthesizing oligonucleotides having a given nucleotide sequence, based on the given nucleotide sequence, have been known. In addition, it is possible in the synthesis of oligonucleotides to introduce arbitrary modifications to the oligonucleotides using nucleotide derivatives modified with, for example, fluorescent dye or biotin. Procedures of binding synthesized oligonucleotides with, for example, fluorescent dye has also been known.

The oligonucleotides according to the present invention, when used as a probe, may be suitably labeled with, for example, a radioisotope or a nonradioactive compound. When used as a primer, it is possible, for example, to design its structure so that the 3'-end region of the oligonucleotide is complementary to a target sequence and so that a restriction enzyme-recognition sequence, a tag, or the like is added to the 5'-end of the oligonucleotide. Such a polynucleotide having a nucleotide sequence composed of at least 15 successive nucleotides can hybridize with a GRK5 mRNA.

The oligonucleotides according to the present invention may contain a nucleotide (base) other than naturally-occurring nucleotides, according to necessity, the examples of which include 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, β-D-galactosylqueuosine, 2'-O-methylguanosine, inosine, N6-isopentenyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, β-D-mannosylqueuosine, 5-methoxycarbonylmethyl-2-thiouridine, 5-methoxycarbonylmethyluridine, 5-methoxyuridine, 2-methylthio-N6-isopentenyladenosine, N-((9-β-D-ribofuranosyl-2-methylthiopurine-6-yl)carbamoyl)threonine, N-((9-β-D-ribofuranosylpurine-6-yl)N-methylcarbamoyl)threonine, uridine-5-oxyacetic acid-methylester, uridine-5-oxyacetic acid, wybutoxosine, pseudouridine, queuosine, 2-thiocytidine, 5-methyl-2-thiouridine, 2-thiouridine, 4-thiouridine, 5-methyluridine, N-((9-β-D-ribofuranosylpurine-6-yl)carbamoyl)threonine, 2'-O-methyl-5-methyluridine, 2'-O-methyluridine, wybutosine, and 3-(3-amino-3-carboxypropyl)uridine.

The present invention also provides a reagent (testing agent) for use in a method of testing whether the subject is susceptible to PD. The reagent according to the present invention includes the primer and/or probe according to the present invention as described above. In testing the subject's susceptibility to PD, a primer and/or a probe for amplifying a DNA containing any of the polymorphic site listed in (1) (preferably the above (1) and (2)) is used.

The reagent according to the present invention can be combined with, for example, various enzymes, enzyme substrates, and buffers according to the nucleotide typing process. Examples of the enzymes include enzymes necessary for the various analysis processes exemplified as the nucleotide typing process, such as DNA polymerase, DNA ligase, or Ils restriction enzyme. As the buffer, suitable buffers for maintaining the activity of the enzymes used in the analysis is suitably selected. As the enzyme substrate, for example, a substrate for complementary strand synthesis is used.

A control composed of a known nucleotide at the polymorphic site may be attached to the reagent of the present invention. The control can be a genome or a genomic fragment whose nucleotide type at the polymorphic site is already identified. The genome may be an extract from a cell; and a cell or cell fraction can also be used. When cells are used as the control, it is possible to prove that the extraction procedure of a genomic DNA is conducted suitably, based on the result of the control. Alternatively, the DNA having a nucleotide sequence that includes the polymorphic site may be used as the control. Specifically, a YAC vector and a BAC vector containing a DNA derived from a genome and having a known nucleotide type at the polymorphic site of the present invention are useful as the control. It is also possible to use, as the control, a vector prepared by splicing only several hundreds of bases corresponding to the polymorphic site and inserting the bases into a vector.

Another embodiment of the reagent of the present invention is a reagent for testing whether the subject is susceptible to PD, which includes a nucleotide probe immobilized on the solid phase, in which the nucleotide probe hybridizes with a DNA comprising any of the polymorphic sites described in (1) of the present invention.

These reagents find utility in the context of tests that use an index the polymorphic site of the present invention. Methods for preparing these are as mentioned above.

A preferred embodiment of the present invention relates to a method for testing whether the subject is susceptible to PD (a predisposition of PD), which includes the step of detecting a transcription or translation product of the GRK5 gene.

Accordingly, oligonucleotides usable as a probe in the detection of a transcription product of the GRK5 gene in the testing method, such as oligonucleotides that hybridize with the transcription product of the GRK5 gene, is one example of testing reagents according to the present invention.

In addition, antibodies that recognize a GRK5 protein (anti-GRK5 protein antibody) and is usable in the detection of a translation product of the GRK5 gene in the testing method is also a preferred example of testing reagents according to the present invention.

It is considered that the phosphorylation of α-synuclein (αS) is promoted due to enhancement of GRK5 gene, and as a result, the formation of soluble α-synuclein oligomer is promoted, thereby leading to PD. Accordingly, substances that suppress the function (activity) of GRK5 gene or of a protein encoded by the gene may be agents for treating or preventing PD.

The present invention provides agents for treating PD that include, as an active ingredient, substances that inhibit the expression of GRK5 gene or GRK5 protein, or substances that inhibit the function (activity) of a protein encoded by GRK5 gene (GRK5 protein).

In a preferred embodiment, the present invention initially provides agents for treating PD (agent and/or pharmaceutical composition for treating or preventing PD), which includes, as an active ingredient, an expression inhibitor for the expression of GRK5 gene.

The GRK5 gene expression inhibitor in the present invention includes, for example, substances that inhibit the transcription of GRK5 or the translation of the transcription product. Examples of preferred embodiments of the above expression inhibitors in the present invention include compounds (nucleic acids) selected from the group consisting of (a) to (c) below:
(a) an antisense nucleic acid to a transcription product of the GRK5 gene or a part thereof;
(b) a nucleic acid having ribozyme activity of specifically cleaving a transcription product of the GRK5 gene; and
(c) a nucleic acid having an action of inhibiting expression of the GRK5 gene through an RNA interference effect.

The term "nucleic acid" in the present invention means an RNA or a DNA. The "nucleic acid" in the present invention also includes chemically synthesized nucleic acid analogues such as a so-called PNA (peptide nucleic acid). PNA has a three-dimensional structure closely resembling that of a nucleic acid, and has a polyamide skeleton having glycine units instead of the pentose-phosphate skeleton of the nucleic acid as a basic skeleton structure.

As methods for inhibiting the expression of specific endogenous genes, a method using an antisense technique is well known to one skilled in the art. As actions for an antisense nucleic acid to inhibit the expression of a target gene, there are multiple factors as mentioned below. Specifically, examples of such actions include: inhibition of transcription initiation due to triplex formation; transcription inhibition due to hybridization with a site in which a local open loop structure is formed by RNA polymerase; transcription inhibition due to hybridization with RNA which is under synthesis; inhibition of splicing due to hybridization with an intron-exon junction; inhibition of splicing due to hybridization with a spliceosome-forming site; inhibition of translocation from the nucleus to the cytoplasm due to hybridization with mRNA; inhibition of splicing due to hybridization with a capping site or a poly(A) addition site; inhibition of translation initiation due to hybridization with translation initiation factor-binding site; inhibition of translation due to hybridization with a ribosome-binding site in the vicinity of initiation codon; inhibition of peptide chain elongation due to hybridization with a coding region or polysome-binding site of mRNA; and inhibition of gene expression due to hybridization with an interaction site between nucleic acid and protein. Thus, antisense nucleic acid inhibits the expression of a target gene by inhibiting various processes, such as transcription, splicing, or translation (Hirashima and Inoue, Shin Seikagaku Jikken Koza 2 Kakusan IV Idenshi No Fukusei To Hatsugen (Experimental Biochemistry, New Ed., 2, Nucleic Acid IV, Replication and Expression of Genes), edited by The Japanese Biochemical Society, TOKYO KAGAKU DOJIN CO., LTD., 1993, 319-347.).

The antisense nucleic acid for use in the present invention may inhibit the expression of GRK5 gene by any of the above-mentioned actions. As one embodiment, it may be effective for inhibiting gene translation to design an antisense sequence to be complementary to a noncoding region adjacent to the 5'-end of the mRNA of the GRK5 gene. In addition, sequences complementary to a coding region or to a noncoding region at the 3' side. Thus, antisense nucleic acids for use in the present invention also include nucleic acids having an antisense sequence to not only a sequence of a coding region of the GRK 5 gene, but also to a sequence of a noncoding region of the GRK5 gene. The antisense nucleic acid to be used is linked downstream of a suitable promoter, and a sequence containing a transcription terminator signal is preferably linked to the 3' side. Nucleic acids thus prepared can be used to transform a desired animal according to known methods. The sequence of the antisense nucleic acid is preferably a sequence complementary to endogenous GRK5 gene of the animal to be transformed, or a part thereof. However, the sequence may not be completely complementary, so long as the gene expression can be effectively inhibited. A transcribed RNA has a complementarity of preferably 90% or more, and most preferably 95% or more to a transcription product of the target gene. To effectively inhibit the expression of the target gene (GRK5) using an antisense nucleic acid, the length of the antisense nucleic acid is preferably at least 15 nucleotides or more and less than 25 nucleotides, but the length of the antisense nucleic acid for use in the present invention is not necessarily limited thereto.

The antisense for use in the present invention is not particularly limited; however, it can be prepared, for example, based on the nucleotide sequence of SEQ ID NO: 1.

Expression of GRK5 gene may be inhibited by using ribozymes, or the DNA encoding ribozymes. The term "ribozyme" refers to RNA molecules having catalytic activity. There are ribozymes having a variety of activities. As a result of studies focusing, of such ribozymes, on ribozymes as enzymes that cleave RNA, it becomes possible to design ribozymes that site-specifically cleave RNA. Of ribozymes, some have a size of 400 nucleotides or more, such as Group I intron-type ribozymes and M1 RNA belonging to RNase P, others have an active domain of about 40 nucleotides, called hammerhead or hairpin ribozymes (Makoto Koizumi and Eiko Otsuka, Tanpakushitsu, Kakusan Koso (PROTEIN, NUCLEIC ACID, AND ENZYME), 1990, 35, 2191.).

For example, self-cleaving domain of the hammerhead ribozyme cleaves the 3'-side of C 15 in a sequence of G13U14C15; the formation of a base pair of U14 and A9 is considered to be important to its activity; and cleavage can occur at A15 or U15 instead of C15 (Koizumi, M. et al., FEBS Lett, 1988, 228, 228.). By designing a ribozyme having a substrate-binding site complementary to an RNA sequence adjacent to a target site, an RNA-cleaving ribozyme that recognizes UC, UU, or UA in the target RNA and acts in a restriction enzyme-like manner can be prepared (Koizumi, M. et al., FEBS Lett, 1988, 239, 285., Makoto Koizumi and Eiko Otsuka, Tanpakushitsu, Kakusan Koso (PROTEIN, NUCLEIC ACID, AND ENZYME), 1990, 35, 2191., Koizumi, M. et al., Nucl Acids Res, 1989, 17, 7059.).

The hairpin ribozyme is also useful for the objects of the present invention. This ribozyme is found, for example, in the minus strand of satellite RNA of tobacco ringspot virus (Buzayan, JM., Nature, 1986, 323, 349.). There has been shown that target-specific RNA-cleaving ribozyme can also be prepared from hairpin ribozyme (Kikuchi, Y & Sasaki, N., Nucl Acids Res, 1991, 19, 6751., Yo Kikuchi, Kagaku To Seibutsu (Chemistry and Biology), 1992, 30, 112.). Thus, by specifically cleaving transcription products of the GRK5 gene in the present invention using ribozymes, expression of the gene can be suppressed.

In addition, suppression of the expression of endogenous gene can also be conducted through RNA interference (RNAi) using double-stranded RNA containing a sequence identical or similar to the sequence of a target gene. Nucleic acids having inhibitory action due to RNAi effect for use in the present invention is generally also called as siRNA. RNAi is a phenomenon in which a double-stranded RNA is introduced into, for example, a cell to induce the destruction of a mRNA of the target gene to thereby suppress the expression of the target gene, in which the double-stranded RNA contains a sense RNA having a sequence identical to the mRNA of the target gene and an antisense RNA having a sequence complementary thereto. Since RNAi can thus suppress the expression of the target gene, it receives attention as an easy and convenient gene knock-out method as an alternative to conventional complicated, inefficient gene destroying techniques through homologous recombination; or as a method applicable to gene therapies. RNA for use in RNAi does not necessarily need to be completely identical to GRK5 gene or a partial region of the gene, but preferably has complete homology.

A preferred embodiment of the above nucleic acid (c) in the present invention includes double-stranded RNA (siRNA) having an RNAi (RNA interference) effect to GRK5 gene. More specifically, it includes double-stranded RNA (siRNA) composed of a sense RNA and an antisense RNA to a partial sequence of the nucleotide sequence of SEQ ID NO: 1.

Although the details of the RNAi mechanism remains unrevealed, it is considered that an enzyme called DICER (one type of the RNase III nuclease family) comes in contact with the double-stranded RNA, and the double-stranded RNA is decomposed into small fragments called small interfering RNAs or siRNAs. Double-stranded RNAs having RNAi effect in the present invention also include double-stranded RNA before the decomposition by DICER. Namely, even RNAs with such a long strand as not to exhibit RNAi effect in its original length is expected to be decomposed in a cell into siRNAs having RNAi effect, and thus, the length of the double-stranded RNA in the present invention is not particularly limited.

For example, a long double-stranded RNA corresponding to a region of the full-length or substantially full-length of the mRNA of GRK5 gene of the present invention may be decomposed beforehand with DICER, and the decomposition product thereof may be used as a therapeutic agent for PD. The decomposition product is expected to contain double-stranded RNA molecules having RNAi effect (siRNA). Following to this method, there is no need to particularly select a region on mRNA expected to have RNAi effect. Thus, it is not always necessary to precisely specify a region on the mRNA of GRK5 gene of the present invention which has RNAi effect.

Of the above described RNA molecules, those that have a conformation in which one end is closed, such as an siRNA having hairpin structure(shRNA), is also included within the present invention. Namely, single stranded RNA molecules that can intramolecularly form a double-stranded RNA structure is also included within the present invention.

The above "double-stranded RNA that can be suppressed through RNAi effect" for use in the present invention can be appropriately produced by one skilled in the art based on the nucleotide sequence of the GRK5 gene of the present invention as a target for the double-stranded RNA. For example, the double-stranded RNA for use in the present invention can be produced based on the nucleotide sequence of SEQ ID NO: 1. Namely, based on the nucleotide sequence of SEQ ID NO: 1, it is within the range of usual trials for one skilled in the art to suitably select an arbitrary consecutive RNA region of the mRNA, which is the transcription product of the sequence, and prepare a double-stranded RNA corresponding to the selected region. In addition, the selection of siRNA sequence having stronger RNAi effect from among the mRNA sequences, which are the transcription products of the sequence, can be suitably conducted by one skilled in the art according to known methods. If one of the two strands (for example, the nucleotide sequence of SEQ ID NO: 1) has been identified, one skilled in the art can easily know the nucleotide sequence of the other strand (complementary strand). The siRNA can be suitably produced by one skilled in the art using commercially available nucleic acid synthesizers. In addition, a general contract synthesis for customers can be used for the synthesis of desired RNA.

A DNA (vector) that can express the above-mentioned RNA of the present invention is also included in the preferred embodiments of the compounds that can suppress the expression of GRK5 gene of the present invention. For example, a DNA (vector) that can express the above double-stranded RNA according to the present invention is a DNA having a structure in which a DNA encoding one strand of the double-stranded RNA, and a DNA encoding the other strand of the double-stranded RNA are linked with promoters so that they can each be expressed. The above mentioned DNA of the present invention can be suitably produced by one skilled in the art according to general genetic engineering techniques. More specifically, an expression vector for use in the present invention can be prepared by suitably inserting a DNA encoding the RNA of the present invention into various known expression vectors.

The expression inhibitors of present invention further include compounds that suppress the expression of GRK5 by binding with, for example, the expression regulatory region (for example, a promoter region) of GRK5. The compound may be obtained, for example, by a screening method using a promoter DNA fragment of GRK5 and using the binding activity with the DNA fragment as an index. One skilled in the art can suitably conduct determination on a desired compound whether the compound suppresses the expression of GRK5 of the present invention according to known methods, such as a reporter assay.

As demonstrated herein, the expression of GRK5 gene is enhanced by polymorphic variations "m22.1" and "m24" in transcriptional regulatory regions of GRK5.

"m22.1" is a polymorphism present in a sequence recognized by YY1 (Yin Yang-1) which is a transcriptional regulator (YY1-recognition sequence) (Fig. 1). YY1 is a factor that regulates to suppress the transcription of GRK5. GRK5 gene expression is thought to be enhanced because the "m22.1" polymorphic variation lowers the binding ability (interaction) between YY1 and "YY1-recognition sequence", and a transcriptional repression effect of YY1 is thereby lowered.

Accordingly, a compound that elevates the binding ability between YY 1 and "YY1-recognition sequence" is considered to suppress GRK5 gene transcription and can be said as one of the preferred embodiments of the above described expression inhibitors of the present invention. The wildtype nucleotide sequence of a DNA having the "YY 1-recognition sequence" is shown in SEQ ID NO: 4; and the nucleotide sequence of a DNA having the polymorphic variation "m22.1" and having a varied binding activity with the YY1 transcription factor is shown in SEQ ID NO: 5.

"m24" is a polymorphism present in a sequence recognized by CRE-binding protein 1 (CREB-1) which is a transcriptional regulator (CREB-recognition sequence) (Fig. 1). CREB-1 is a factor that regulates to promote GRK5 transcription. GRK5 gene expression is thought to be enhanced because the "m24" polymorphic variation increases the binding ability (interaction) between CREB-1 and "CREB-recognition sequence", and the transcription promotion effect of CREB-1 thereby increases.

Accordingly, a compound that lowers the binding ability between CREB-1 and "CREB-recognition sequence" is considered to suppress GRK5 gene transcription and can be one of the preferred embodiments of the above described expression inhibitor of the present invention. In addition, anti-CREB-1 antibody is also one example of the above described expression inhibitors for use in the present invention. The wildtype nucleotide sequence of a DNA having the "CREB-recognition sequence" is shown in SEQ ID NO: 2; and the nucleotide sequence of a DNA having the polymorphic variation "m24" and having a varied binding activity with the CREB-1 transcription factor is shown in SEQ ID NO: 3.

As is described above, the "YY1-recognition sequence" and "CREB-recognition sequence" having polymorphic variations of the present invention have varied binding abilities with the YY1 transcription factor and the CREB-1 transcription factor, respectively, and polynucleotides having the sequences can be suitably used and are useful in the after-mentioned method of screening for an agent for treating or preventing PD. The present invention provides a polynucleotide of following (a) or (b):
(a) a polynucleotide having the nucleotide sequence of SEQ ID NO: 4 or a nucleotide sequence with an addition, deletion, or a substitution of one or more nucleotides in the nucleotide sequence, in which the polynucleotide has a lowered binding ability with YY1 transcription factor; and
(b) a polynucleotide having the nucleotide sequence of SEQ ID NO: 2 or a nucleotide sequence with an addition, deletion, or a substitution of one or more nucleotides in the nucleotide sequence, in which the polynucleotide has an elevated binding ability with a CREB-1 transcription factor.

The present invention further provides a therapeutic agent for PD, comprising a GRK5 protein function inhibitor as an active ingredient.

Examples of the GRK5 protein function inhibitor in the present invention include compounds of following (a) and (b):
(a) an antibody that binds with GRK5 protein; and
(b) a low-molecular-weight compound that binds with GRK5 protein.

An antibody that binds with a GRK5 protein (anti-GRK5 antibody) can be prepared according to methods known to one skilled in the art. When the antibody is a polyclonal antibody, it can be obtained, for example, in the following manner. Small animals such as rabbit are immunized with natural GRK5 protein, or recombinant (recombination) GRK5 protein expressed as fusion protein with GST in microorganisms such as *Escherichia coli,* or partial peptides thereof, and the serum is collected from the small animal. Serum is purified, for example, through precipitation with ammonium sulfate, a protein A or protein G column, DEAE ion exchange chromatography, or an affinity column coupled with GRK5 protein or synthetic peptide to yield the antibody. When the antibody is a monoclonal antibody, it can be prepared, for example, in the following manner. Small animals such as mice are immunized with GRK5 protein or partial peptides thereof; the spleen is extirpated from the mice, and is ground to separate cells; the cells and mouse myeloma cells are fused using reagents such as polyethylene glycol; and clones that produce antibodies that bind with GRK5 protein are selected from the resulting fused cells (hybridomas). Next, the obtained hybridomas are intraperitoneally transplanted to a mouse; the ascites is recovered from the mouse; and the obtained monoclonal antibodies are purified, for example, through precipitation with ammonium sulfate, a protein A or protein G column, DEAE ion exchange chromatography, or an affinity column coupled with GRK5 protein or synthetic peptides to yield the antibody.

The antibody of the present invention is not particularly limited in its form and includes, in addition to the above-mentioned polyclonal antibody and monoclonal antibody, human antibody, humanized antibody obtained by gene recombination, antibody fragment, and modified antibody thereof, so long as it binds with GRK5 protein of the present invention.

The GRK5 protein for use as sensitizing antigen for obtaining the antibody in the present invention is not limited in animal species as its origin; however, it is preferably a protein derived from mammals such as mice or humans and is particularly preferably a human-derived protein. Such a human-derived protein can be suitably obtained by one skilled in the art using the gene sequence or amino acid sequence disclosed in the present specification.

The protein for use as sensitizing antigen in the present invention may be an entire protein or a partial peptide of the protein. Examples of the partial peptide of proteins include amino (N) terminal fragment, carboxy (C) terminal fragment, or kinase activity site at a center part, of a protein. The term "antibody" in the present specification means refers to antibodies that react with a full-length protein or a fragment of the protein.

Examples of the antibodies for use in the present invention include polyclonal antibodies, monoclonal antibodies, chimeric antibodies, single stranded antibodies (scFv) (Huston et al. (1988) Proc. Natl. Acad. Sci. USA 85: 5879-83; The Pharmacology of Monoclonal Antibody, Vol. 113, Rosenburg and Moore ed., Springer Verlag (1994) pp. 269-315), humanized antibodies, polyspecific antibodies (LeDoussal et al. (1992) Int. J. Cancer Suppl. 7: 58-62; Paulus (1985) Behring Inst. Mitt. 78: 118-32; Millstein and Cuello (1983) Nature 305: 537-9; Zimmermann (1986) Rev. Physiol. Biochem. Pharmacol. 105: 176-260; VanDijk et al. (1989) Int. J. Cancer 43: 944-9), and antibody fragments such as Fab, Fab', F(ab') 2, Fc, and Fv. These antibodies may be modified, for example, with PEG according to necessity. Antibodies can be configured to be detectable without using a secondary antibody, by preparing a fusion protein with, for example, β-galactosidase, maltose binding protein, glutathione S-transferase (GST), or a green fluorescent protein (GFP). Antibodies can be altered to be detectable and recoverable using, for example, avidin or streptavidin by labeling the antibody with, for example, biotin.

Besides obtaining the above described hybridomas by immunizing animals other than humans with antigens, hybridomas that produce desired human antibodies having binding activity with a protein can be obtained by sensitizing human lymphocytes, such as human lymphocytes infected by EB virus, *in vitro* with the protein, cells expressing the protein, or the lysate thereof; and fusing the sensitized lymphocytes with human-derived myeloma cells having a permanent division potential, such as U266.

Antibodies against the GRK5 protein of the present invention is expected to suppress the function of GRK5 protein by binding with GRK5 protein and to have, for example, a therapeutic or improving effect on PD. When the obtained antibody is used for administration to humans (antibody therapy), it is preferably a human antibody or a human-type antibody for lowering immunogenicity. Substances that can inhibit the function of GRK5 protein in the present invention further include low molecular weight substances (low molecular weight compounds) that bind with the GRK5 protein. The low molecular weight substances that bind with the GRK5 protein in the present invention may be natural or artificial compounds. The compounds can be generally prepared or obtained according to methods known to one skilled in the art. The compound of the present invention can also be obtained by screening methods mentioned below.

The above-mentioned low -molecular weight compound that binds with a GRK5 protein of (b) includes, for example, compounds having a high affinity for GRK5.

Substances that can inhibit the function of GRK5 protein of the present invention include mutant GRK5 protein having dominant-negative property to the GRK5 protein. The phrase "mutant GRK5 protein having dominant-negative property to the GRK5 protein" refers to proteins having the function of causing the activity of endogenous wildtype protein to disappear or reducing the activity of endogenous wildtype protein by expressing the gene encoding the protein.

Substances (compounds) known to inhibit the function of GRK5 protein can be a suitable and specific example of "substances that can suppress the function of GRK5 protein" in the present invention.

Following compounds have been reported as compounds known to inhibit the function of GRK5 protein. It was discovered for the first time that GRK5 is associated with PD. As a result, novel uses of the following compounds as agents for preventing or treating PD have been discovered.
(1) 2-[1-(3-dimethylaminopropyl)-1H-indol-3-yl]-3-(1H-indol-3-yl)-maleimide) (GF 109203X) (see Zhou H. et al., J Pharmacol Exp Ther. 2001 Sep;298(3):1243-51).
(2) 2-(8-[(dimethylamino)methyl]-6,7,8, 9-tetrahydropyrido[1,2-a]indol-3-yl)-3-(1-methylindol-3-yl)maleimide hydrochloride (Ro 32-0432) (see Aiyar N. et al., Eur J Pharmacol. 2000 Sep 1;403(1-2):1-7).
(3) a GRK inhibitor including a compound represented by the following formula: 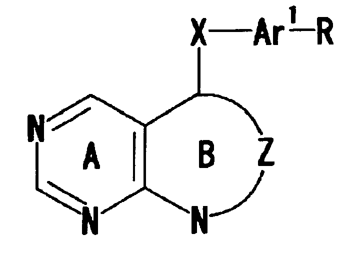 [wherein, Ring A is an optionally substituted pyrimidine ring; Z is an optionally substituted divalent to tetravalent linear C1-3 hydrocarbon group; Ring B is further optionally substituted; X is an optionally substituted C1-4 alkylene or such; Ar1 is an aromatic hydrocarbon group which is optionally substituted with substituents other than R or an aromatic heterocyclic group which is optionally substituted with substituents other than R; and R is a hydrogen atom or -Y Ar2 (Y is a bond or a spacer in which the number of atoms constituting the linear portion is one to six; and Ar2 is an optionally substituted aromatic hydrocarbon group or an optionally substituted aromatic heterocyclic group), a salt thereof, or a prodrug thereof (see Japanese Patent Application Kokai Publication No. (JP-A) 2003-321472 (unexamined, published Japanese patent application)).
(4) a GRK inhibitor including a compound represented by the following formula: 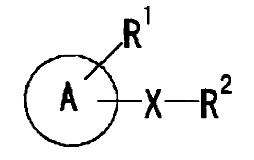 [wherein, Ring A is a further optionally substituted nitrogen-containing heterocyclic ring; R1 and R2 are each optionally substituted amino group; and X is a spacer in which the number of atoms constituting the linear portion is one to four, in which R1 may bind with R2 and/or X to form a ring], a salt thereof, or a prodrug thereof (see JP-A 2002-145778).
(5) 2-(2'-amino-3'-methoxyphenyl)oxanaphthalen-4-one) and U0126 (1,4-diamino-2,3-dicyano-1,4-bis (aminophenylthio) butadiene) (PD98059) (see Trincavelli ML et al., Biochim Biophys Acta. 2002 Aug 19;1591(1-3):55-62).
(6) a peptide composed of the 14 amino acids of the GRK5 protein N terminal (see Noble B. et al., J Biol Chem. 2003 Nov 28; 278(48):47466-76. Epub 2003 Sep 24)
(7) Heparin, Dextran sulfate (see Kunapuli P et al., J Biol Chem. 1994 Jan 14; 269(2):1099-105)
(8) Calmodulin (see Pronin AN et al., J Biol Chem. 1997 Jul 18;272(29):18273-80)
(9) Zn²⁺ (see Shayo C. et al., Mol Pharmacol. 2001 Nov;60(5):1049-56)

The function inhibitor according to the present invention can be suitably obtained by screening methods according to the present invention using the GRK5 activity as an index.

Substances that suppress the formation of soluble α-synuclein (αS) oligomers are also useful as a therapeutic agent for PD according to the present invention. Accordingly, the present invention provides a therapeutic agent for PD, such an agent containing as an active ingredient a substance that inhibits the formation of a soluble α-synuclein oligomer.

The present invention further provides methods of screening for an agent for treating or preventing PD (candidate compound), including the step of selecting compounds that lower the expression level of GRK5 gene or the activity of the protein encoded by the gene.

One embodiment of the screening methods of the present invention is a method using the expression level of GRK5 gene as an index. Compounds that lower the expression level of GRK5 gene are expected to serve as agents for the prevention and treatment of PD.

The above described method of the present invention is, for example, a method of screening for an agent for treating or preventing PD, which includes the following steps (a) to (c):
(a) contacting a test compound with cells that express GRK5 gene;
(b) measuring the expression level of GRK5 gene; and
(c) selecting the compound that lowers the expression level as compared to the expression level measured in the absence of the test compound.

In the present method, initially a test compound is contacted with cells that express GRK5 gene. The "cells" for use herein, regarding its origin, can be cells derived from humans, mic, cats, dogs, cattle, sheep, birds, or another pets or livestock, but is not limited thereto. Cells expressing an endogenous GRK5 gene, or cells expressing an exogenous GRK5 gene which has been introduced thereto can be used as the "cells that express GRK5 gene". Cells expressing an exogenous GRK5 gene can be generally produced by introducing to a host cell, expression vectors into which GRK5 gene has been inserted. The expression vector can be produced according to general genetic engineering techniques.

Test compounds for use in the method are not particularly limited. Examples thereof include single compounds such as natural compounds, organic compounds, inorganic compounds, proteins, and peptides; and compound libraries, expression products of gene libraries, cell extracts, cell culture supernatants, products of fermenting microorganisms, marine organism extracts, and vegetable extracts, but are not limited thereto.

Test compounds may be "contacted" with cells that expresses GRK5 gene generally by adding the test compounds to the culture medium of the cells that express the GRK5 gene, but is not limited thereto. When the test compound is, for example, proteins, "contacting" can be carried out by introducing DNA vectors that express the protein into the cells.

In the present method, next, the expression level of the GRK5 gene is measured. The term " gene expression" as used herein includes both transcription and translation. Gene expression levels can be measured according to methods known to one skilled in the art. The transcription level of the gene can be measured, for example, by extracting mRNA from cells that express the GRK5 gene according to common procedures, and carrying out northern hybridization or RT-PCR using the extracted mRNA as the template. The gene translation level can be measured by recovering protein fractions from cells that expresses the GRK5 gene, and detecting the expression ofGRK5 protein by electrophoresis such as sodium dodecyl sulfate-polyacrylamide gel electrophoresis (SDS-PAGE). In addition, the gene translation level can also be measured by carrying out Western blotting using antibodies against GRK5 protein to detect the expression of the protein. Antibodies for use in the detection of GRK5 protein is not particularly limited, so long as they are detectable antibodies, and for example, both monoclonal antibodies and polyclonal antibodies can be used.

In the present method, next, compounds that lower the expression level as compared to the expression level when the test compound is not contacted (control) is selected. Such compounds that lower the expression level can be an agent for treating or preventing PD.

Another embodiment of the screening methods according to the present invention is a method of identifying compounds that lower the expression level of the GRK5 gene of the present invention, using the expression of a reporter gene as an index.

The above-mentioned method of the present invention is, for example, a method of screening for an agent for treating or preventing PD, which includes the following steps (a) to (c):
(a) contacting a test compound with cells containing DNA having a structure in which a transcriptional regulatory region of GRK5 gene and a reporter gene are operably linked with each other;
(b) measuring the expression level of the reporter gene; and
(c) selecting the compound that lowers the expression level as compared to the expression level measured in the absence of the test compound.

In the present method, initially a test compound is contacted with cells or cell extracts that include a DNA having a structure in which a transcriptional regulatory region of the GRK5 gene and a reporter gene are operably linked with each other. The phrase "operably linked" herein means that a transcriptional regulatory region of the GRK5 gene and a reporter gene bind with each other so that the expression of the reporter gene is induced as a result that a transcription factor binds with the transcriptional regulatory region of the GRK5 gene. Accordingly, even when the reporter gene is linked with another gene and forms a fused protein with another gene product, one is included within the meaning of "operably linked", so long as the expression of the fused protein is induced as a result of binding of a transcription factor with a transcriptional regulatory region of the GRK5 gene. A transcriptional regulatory region of the GRK5 gene in the genome can be obtained according to known methods based on the cDNA nucleotide sequence of the GRK5 gene by one skilled in the art.

The reporter gene for use in the present method is not particularly limited, so long as its expression is detectable, and includes, for example, the CAT gene, lacZ gene, luciferase gene, and GFP gene. The "cells containing DNA having a structure in which a transcriptional regulatory region of a GRK5 gene and a reporter gene are operably linked with each other" can be, for example, cells introduced with vectors into which such structure is inserted. Such vectors can be prepared according to methods well known to one skilled in the art. The vectors can be introduced into cells according to general methods such as calcium phosphate precipitation, electroporation, a Lipofectamine method, or microinjection. The "cells containing DNA that has a structure in which a transcriptional regulatory region of a GRK5 gene and a reporter gene are operably linked with each other" further include cells in which the structure has been inserted into their chromosome. The DNA structure can be inserted to the chromosome according to methods generally used by one skilled in the art, such as gene transfer technique using homologous recombination.

The "cell extracts containing DNA that has a structure in which a transcriptional regulatory region of a GRK5 gene and a reporter gene are operably linked with each other" can be, for example, cell extracts that are contained in commercially available *in vitro* transcription/translation kit and are added with DNA that has a structure in which a transcriptional regulatory region of a GRK5 gene and a reporter gene are operably linked with each other.

The "contact" in the present method can be conducted by adding a test compound to a culture medium of the "cells containing DNA having a structure in which a transcriptional regulatory region of a GRK5 gene and a reporter gene are operably linked with each other", or by adding a test compound to the above described commercially available cell extracts containing the DNA. When the test compound is a protein, the contact can also be conducted by introducing DNA vectors that express the protein into the cells.

Next, the expression level of the reporter gene is measured in the present method. The expression level of the reporter gene can be measured by methods known to one skilled in the art according to the type of the reporter gene. When the reporter gene is, for example, the CAT gene, the expression level of the reporter gene can be measured by detecting acetylation of chloramphenicol by the gene product. The expression level of the reporter gene can be measured by detecting coloring of a dye compound catalyzed by an expression product of the lacZ gene when the reporter gene is the lacZ gene; by detecting fluorescence of a fluorescent compound catalyzed by an expression product of the luciferase gene when the reporter gene is the luciferase gene; or by detecting fluorescence of the GFP protein when the reporter gene is the GFP gene.

Next, in the present method, compounds that lower (suppress) the measured expression level of the reporter gene as compared to the expression level measured in the absence of the test compound is selected. The compounds that lower (suppress) the expression level can be an agent for treating or preventing PD.

As the GRK5 gene for use in the above-mentioned screening method of the present invention, a wildtype gene can be generally used, and a GRK5 gene (mutant GRK5 gene) containing a polymorphic variation ("m22.1" and/or "m24") which is involved in elevated expression and discovered herein can also be suitably used.

This mutant GRK5 gene originally shows enhanced gene expression and is suitable for screening a substance that suppresses (lowers) the expression of the gene. In addition, a substance that suppresses (lowers) the enhanced gene expression of a mutant GRK5 gene found in patients actually suffering from PD is expected to be a suitable agent for preventing or treating PD.

Another embodiment of the screening methods of the present invention is a method in which an interaction activity between GRK5 protein and α-synuclein is used as an index.

The above-mentioned method of the present invention is, for example, a method of screening for an agent for treating or preventing PD, which includes the following steps (a) to (c):
(a) contacting a test compound with GRK5 protein and α-synuclein;
(b) measuring an interaction activity between GRK5 protein and α-synuclein; and
(c) selecting a compound that lowers the interaction activity as compared to the interaction activity measured in the absence of the test compound.

Initially, a test compound is contacted with GRK5 protein and α-synuclein in the present method.

Next, an interaction activity (binding activity) between GRK5 protein and α-synuclein is measured. The interaction activity between arbitrary proteins can be evaluated by one skilled in the art using various known methods.

For example, the interaction activity can be evaluated using immunoprecipitation.

Specifically, the evaluation can be conducted in the following manner, but not necessarily limited thereto. DNA encoding GRK5 is inserted into a vector for exogenous gene expression, and the gene is expressed, for example, in cells. Promoters for use in gene expression are not particularly limited, as long they are commonly used promoters.

GRK5 can be expressed as a fused polypeptide having a monoclonal antibody-recognition site by introducing a monoclonal antibody-recognition site (epitope), whose specificity has been revealed, into the N-terminus or C-terminus of GRK5. Commercially available epitope-antibody system can be used herein (Jikken Igaku (Experimental Medicine) 13, 85-90 (1995)). There are commercially available vectors that can express a fusion polypeptide with, for example, β-galactosidase, maltose binding protein, glutathione S-transferase, or green fluorescent protein (GFP) through a multicloning site. In addition, methods of preparing fusion polypeptides by introducing small epitope portions alone, which include several to several tens of amino acids, to minimize changes in properties of GRK5 due formation of fusion polypeptide have been reported. Examples of epitope- antibody system usable for screening for a polypeptide that binds with GRK5 include epitopes such as polyhistidine (His-tag), influenza agglutinin HA, human c-myc, FLAG, Vesicular stomatitis virus glycoprotein (VSV-GP), T7 gene 10 protein (T7-tag), human herpes simplex virus glycoprotein (HSV-tag), and E-tag (epitope on a monoclonal phage) in combination with a monoclonal antibody that recognizes the epitope (Jikken Igaku (Experimental Medicine) 13, 85-90 (1995)).

In immunoprecipitation, immunocomplex is formed by adding such antibodies to cell lysates which have been prepared by using suitable surfactants. This immunocomplex includes GRK5, a polypeptide having binding ability with GRK5 (for example, α-synuclein in the above-mentioned screening), and antibody. Instead of using the antibodies against the above described epitopes, immunoprecipitation can also be conducted using antibodies against GRK5. The antibody against GRK5 can be prepared, for example, by introducing the gene encoding GRK5 into suitable *Escherichia coli* expression vectors, allowing the gene to express in *Escherichia coli,* purifying the expressed polypeptide, and immunizing, for example, rabbits, mice, rats, goats, or chicken with the purified polypeptide. It can also be prepared by immunizing the above described animals with synthesized partial peptides of GRK5.

When the antibody is, for example, a murine IgG antibody, an immunocomplex can be precipitated using Protein A Sepharose or Protein G Sepharose. When GRK5 is prepared, for example, as a fusion polypeptide with an epitope such as GST, an immunocomplex can be formed using a substance that specifically binds with the epitope, such as glutathione-Sepharose 4B, as in the use of an antibody against GRK5.

Immunoprecipitation may be conducted according to or pursuant to common methods such as the method stated in the document (Harlow, E. and Lane, D.: Antibodies, pp. 511-552, Cold Spring Harbor Laboratory publications, New York (1988)).

SDS-PAGE or Western blotting is used to analyze the immunoprecipitated polypeptides. When SDS-PAGE is used, bound polypeptides can be analyzed based on the molecular weight of the polypeptides using gel in a suitable concentration. In this case, since polypeptides bound to GRK5 are generally difficult to be detected according to usual polypeptide staining methods such as Coomassie staining or silver staining, the detection sensitivity can be improved by cultivating cells in culture medium containing 35S-methionine or 35S-cysteine, which are radioisotopes, to label the polypeptides in the cells, and detecting the labeled polypeptides.

The interaction (binding) activity between GRK5 and α-synuclein can be evaluated by analyzing the amount of α-synuclein that interacts with GRK5 according to the above-mentioned method.

Next, compounds that lower (suppress) the interaction activity as compared to the interaction activity measured in the absence of the test compound is selected. The compounds that lower (suppress) the interaction activity can be an agent for treating PD.

Yet another embodiment of the screening methods of the present invention is a method in which the phosphorylation activity of GRK5 protein in which α-synuclein is the substrate is used as an index. Compounds that lower the phosphorylation activity suppress the formation of soluble α-synuclein oligomers to thereby decrease the soluble oligomers in the brain and are expected to have a therapeutic effect on PD.

The above-mentioned method according to the present invention is, for example, a method of screening for an agent for treating or preventing PD, which includes the following steps (a) to (c):
(a) contacting a test compound with GRK5 protein and α-synuclein;
(b) measuring the phosphorylation activity of GRK5 protein in which α-synuclein is the substrate; and
(c) selecting the compound that lowers the phosphorylation activity as compared to the phosphorylation activity measured in the absence of the test compound.

Initially, a test compound is contacted with GRK5 protein and α-synuclein in the present method.

This "contact" can be conducted, for example, by contacting a test compound with cells (for example, HEK293 cells) which is allowed to co-express GRK5 and α-synuclein cDNA.

Next, the phosphorylation activity of GRK5 protein using α-synuclein as a substrate is measured. The GRK5 protein for use in the above-mentioned method is preferably a wildtype protein including no mutation; however, it may be a protein (polypeptide) in which a part of the amino acid sequence of GRK5 protein is substituted and/or deleted, so long as it has α-synuclein phosphorylation activity. In addition, α-synuclein serving as a substrate is also preferably a wildtype protein including no mutation; however, it may be a partial polypeptide that includes the site to be phosphorylated by GRK5 protein, or a mutant polypeptide including the site.

According to past studies, α-synuclein is thought to be phosphorylated on serine at position 129 (Ser-129). Accordingly, the α-synuclein to be subjected to the above described screening method of the present invention may also be a polypeptide fragment that includes Ser-129.

More specifically, the phosphorylation activity can be measured according to the method described in the after-mentioned Examples.

These activities can be measured according to methods known to one skilled in the art, such as Western blotting using phosphorylation-specific antibodies.

Compounds that lower the phosphorylation activity as compared to the phosphorylation activity measured in the absence of the test compound is selected in the above-mentioned method. The thus-selected compound is expected to suppress the phosphorylation activity of GRK5 protein using α-synuclein as a substrate to thereby suppress soluble α-synuclein oligomerization, and to have an effect of treating or preventing PD.

The present invention further provides a method of screening for agents for treating or preventing PD, in which the solubility of α-synuclein oligomers is used as an index.

One embodiment of the screening methods of the present invention is a method in which the amount of soluble α-synuclein oligomers is used as an index. Compounds that lower the amount of soluble α-synuclein oligomers is expected to serve as an agent for treating PD.

The method of the present invention mentioned above is, for example, a method of screening for an agent for treating or preventing PD, which includes the following steps (a) to (c):
(a) contacting a test compound with GRK5 protein and insoluble α-synuclein oligomer;
(b) measuring the amount of a soluble α-synuclein oligomer; and
(c) selecting the compound that lowers the amount of soluble α-synuclein oligomer as compared to the amount of soluble α-synuclein oligomer measured in the absence of the test compound.

In the present method, initially, a test compound is contacted with GRK5 protein and insoluble α-synuclein oligomer. Next, the amount of soluble α-synuclein oligomer is measured. The measurement can be conducted according to techniques known to one skilled in the art, such as Western blotting using anti-α-synuclein antibodies. For example, the amount of soluble oligomers can be measured by subjecting the soluble fraction of the above described HEK293 cells to gel filtration.

Further, in the above-mentioned method, compounds that lower the amount of the soluble α-synuclein oligomer as compared to the amount of soluble α-synuclein oligomer measured in the absence of the test compound is selected. The thus-selected compounds are thought to have an effect of treating or preventing PD.

The present invention further relates to a method of screening for an agent for treating or preventing PD, which uses, as an index, binding between a transcriptional regulator and a DNA transcriptional regulatory region containing the polymorphic site of the present invention.

As is described above, the present invention demonstrates that polymorphic variations "m22.1" and "m24" that are present in transcriptional regulatory regions ofGRK5 enhance the GRK5 gene expression.

Accordingly, a transcriptional regulatory region containing the polymorphic site(s) is associated with GRK5 gene expression, and a substance that alters the binding activity between the transcriptional regulatory region and a transcription factor is expected to be able to alter GRK5 gene expression.

The transcriptional regulatory region DNA containing the polymorphic variation of the present invention has altered binding ability with a transcription factor as compared to the wildtype transcriptional regulatory region DNA. Accordingly, the DNA can be suitably used in a method of screening for substances that alter the binding activity with a transcription factor.

A preferred embodiment of the above-mentioned screening method of the present invention is a method of screening for an agent for treating or preventing PD, which includes the following steps (a) to (c):
(a) contacting a test compound with a YY1 transcription factor and a polynucleotide containing a YY1-recognition sequence or a polynucleotide having the polymorphic variation "m22.1" of the present invention and having a lowered binding activity with the YY1 transcription factor;
(b) measuring the binding activity between the polynucleotide and the transcription factor; and
(c) selecting the compound that elevates the binding activity as compared to the binding activity measured in the absence of the test compound.

As another embodiment, the present invention includes a method of screening for an agent for treating or preventing PD, which includes the following steps (a) to (c):
(a) contacting a test compound with CREB-1 transcription factor, and a polynucleotide having the CREB-recognition sequence or a polynucleotide having the polymorphic variation "m24" of the present invention and having an elevated binding activity with the CREB-1 transcription factor;
(b) measuring the binding activity between the polynucleotide and the transcription factor; and
(c) selecting the compound that lowers the binding activity as compared to the binding activity measured in the absence of the test compound.

In the context of the present invention, the "Parkinson's disease (PD)" is preferably sporadic Parkinson's disease (sPD).

Compounds for use in the above-mentioned screening methods are also useful as reagents for screening for an agent for treating or preventing PD.

Specific examples of the reagents of the present invention include a reagent for screening for an agent for treating or preventing PD, which includes any of following (a) to (d) as an active ingredient:
(a) an oligonucleotide that hybridizes with a transcription product of GRK5 gene;
(b) an antibody that recognizes GRK5 protein;
(c) an antibody that recognizes α-synuclein protein in which a serine residue at position 129 is phosphorylated; and
(d) (d-1) a polynucleotide having the nucleotide sequence of SEQ ID NO: 5 or a nucleotide sequence with an addition, deletion, or a substitution of one or more nucleotides in the nucleotide sequence, in which the polynucleotide has a lowered binding ability with a YY1 transcription factor, or (d-2) a polynucleotide having the nucleotide sequence of SEQ ID NO: 3 or a nucleotide sequence with an addition, deletion, or a substitution of one or more nucleotides in the nucleotide sequence, in which the polynucleotide has an elevated binding ability with a CREB-1 transcription factor.

The present invention further provides kits which include, for example, various agents and/or reagents used for carrying out the testing methods or screening methods of the present invention.

The kit of the present invention can include, for example, a reagent suitably selected from among the reagents of the present invention, according to the testing method or screening method to be conducted. For example, the kit of the present invention may include, as a constitutional component, the oligonucleotides and antibodies for use in the present invention. More specific examples include (1) primer oligonucleotides for use in the present invention, and PCR reaction reagents (such as Taq polymerase and buffer); (2) probe oligonucleotides for use in the present invention, and hybridization buffer; and (3) anti-GRK5 antibody or anti-phosphorylated α-synuclein antibody and ELISA reagent.

The kit of the present invention may further suitably include, for example, control samples, buffer, and directions for use.

The present invention further provides transgenic non-human animals in which exogenous GRK5 protein and α-synuclein are expressed. The present invention demonstrates that the animals show a behavior caused by neurotoxicity. Specifically, the present invention succeeded in actually producing animals showing a phenotype easily distinguishable from that of a wildtype, by allowing GRK5 protein and α-synuclein protein to be expressed artificially in the animals.

The animals of the present invention can be suitably used for screening for a therapeutic agent for PD by using its easily distinguishable phenotype as an index. Substances (compounds) obtained (identified) by the screening methods of the present invention are substances that can actually alter (suppress) the phenotype of the animal of the present invention, and they can be said as substances with a high probability of having a therapeutic effect on nerve diseases, particularly on PD.

In a preferred embodiment, the above-mentioned method of the present invention is a method of screening for an agent for treating or preventing PD, which includes the following steps (a) to (c):
(a) administering a test compound to a transgenic non-human animal of the present invention;
(b) monitoring the animal for the presence of a behavior caused by neurotoxicity in the animal; and
(c) selecting the compound that suppresses the occurrence of the behavior.

The transgenic animals of the present invention can be suitably produced using common genetic engineering techniques. They can be generally produced by linking the genes encoding GRK5 protein and α-synuclein with the expression vectors, and introducing the respective vectors into animal cells. Specifically, transgenic animals of the present invention can be produced by the method stated in the after-mentioned Examples. One skilled in the art can produce the transgenic animals of the present invention from a desired animal, by suitably altering the method stated in Examples.

The transgenic animals of the present invention preferably have a structure in which the exogenous GRK5 gene and the gene encoding α-synuclein are inserted into the genome in an expressible manner.

The transgenic animals of the present invention are not particularly limited, so long as a GRK5 protein and α-synuclein protein, or proteins corresponding to the proteins, such as homologues, can substantially function in the animals when expressed in their cells. The transgenic animal is preferably a nematode.

When the animal is a nematode, examples of the behavior caused by neurotoxicity in step (b) described above include "number of bending", "behavior caused by a contact stimulus in the anterior part of the body", and "food-detecting behavior" as shown in the after-mentioned Examples.

In the method according to the present invention, candidate compounds for treating or preventing PD can be obtained by selecting, from among test compounds, compounds that recover the above-mentioned behavior to a normal level (the various behavior in wildtype animals).

Test compounds can be administered either orally or parenterally. Specific examples thereof include injection, transnasal administration, transpulmonary administration, transdermal administration, and oral administration. As an example of injection, the administration can be conducted systemically or locally through intravenous injection, intramuscular injection, intraperitoneal injection, or hypodermic injection. When the test compound is a DNA and when it is administered to a living body, viral vectors such as retrovirus, adenovirus, or Sendai virus, or non-viral vectors such as liposome can be used.

The present invention further relates to a method of treating or preventing PD, which includes the step of administering to an individual, for example a patient, an agent of the present invention.

A preferred embodiment of the present invention is a method of preventing and/or treating PD, which includes the step of administering to an individual, such as a patient, an inhibitor of GRK5 gene expression, or an inhibitor of the function of GRK5 protein.

In the context of the treating method of the present invention, the term "individual" generally refers to a patient suffering from the disease, and is not particularly limited, but is preferably a human.

Administration to patients can be generally conducted according to methods known to one skilled in the art, such as intraarterial injection, intravenous injection, or hypodermic injection. Although the dose varies depending on, for example, the body weight and age of the patient and the administration method, one skilled in the art can suitably select a suitable dose. When the compound is one that can be encoded by a DNA, it may be possible to integrate the DNA into a vector for gene therapy and to conduct gene therapy.

Examples of vectors for gene therapy include viral vectors such as retrovirus vectors, adenovirus vectors, and adeno-associated virus vectors; and non-viral vectors such as liposome. Subject DNA can be administered to a patient using the vectors according to, for example, an *ex vivo* method or an *in vivo* method.

The present invention further relates to use of an inhibitor of GRK5 gene expression or an inhibitor of the function of GRK5 protein in the production of a therapeutic agent for PD.

All the prior art documents cited in the present specification are incorporated herein by reference.

### [Examples]

Herein below, the present invention will be more specifically described with reference to Examples, but it is not to be construed as being limited thereto. Reagents and plasmids used in Examples were obtained in the following manner.

Sodium a-linolenate and fatty acid-free bovine serum albumin were purchased from Sigma. Okadaic acid was purchased from Wako Pure Chemical Industries, Ltd. The following antibodies were used in the present invention: mouse monoclonal anti-αS antibody (Syn-1, BD Transduction Laboratories), mouse monoclonal anti-phosphorylated Ser-129 αS antibody (psyn#64), a mouse monoclonal anti-α-actin antibody (AC-15, Sigma), and rabbit polyclonal antibody against anti-GRK5 (H-64, Santa Cruz Biotechnology, Inc.). Full-length human αS cDNA (IMAGE clone #3604532) and subcloned vector pcDNA3.1 were supplied from Open Biosystems and Invitrogen Corp., respectively. Human GRK5 cDNA was prepared from RNA of human brains through reverse transcription PCR. GRK5 cDNA was fused at the C-terminus of a FLAG tag and subcloned into pcDNA3.1 vector.

A mutant cDNA in which serine at position 129 was substituted with alanine, and a mutant cDNA in which lysine at position 215 was substituted with arginine as a mutant of GRK5, were verified using the QuickChange Site-Directed Mutagenesis Kit (Stratagene), and the sequences of all cDNAs were identified by DNA sequencing before use.

### [Example 1] SNPs Analysis

The presence or absence of haplotype association was screened for by scanning 5632 SNPs in 520 candidate genes including GRK5 gene according to the sliding window approach, using 286 PD patients and 496 normal controls in Yamagata Pref., Japan, obtained in the above manner.

As a result, it was discovered a low frequency haplotype in the GRK5 gene, which increases the PD morbidity risk. The haplotype G-A-C containing three intron SNPs of rs871196 (m22), rs7069375 (m23), and rs4752293 (m24) in the vicinity of exon 2 and was a low frequency haplotype that increases the PD risk about two fold (haplotype frequency = PD 9.3%, control 5.1%, OR = 1.91, p value = 0.0002) (Table 1A).

In addition, analyses were conducted on linkage disequilibrium and haplotype block in the GRK5 gene to find that a haplotype block containing three SNPs of m22, m23, and m24 is significantly associated with sPD (Table 1A). The results in HTR analyses are also shown. Empirical p values (individual-p) of the respective haplotype-based association studies were obtained by 10,000 permutations. There were found significant empirical p values (P = 0.0152 in 10,000 permutations) in all haplotype-based association studies.

In addition, the D' distribution in this region was investigated. D' scores between SNP m24 and other SNPs are shown in Fig. 2A. The names and positions of genotype SNPs in the GRK5 gene are also shown in Fig. 2B. Of the 15 SNPs covering the GRK5 gene, SNPs covering a region from m20 to m25 showed significant empirical individual p values. These haplotypes had the same allele sequence G-A-C in common in SNPs of m22-m23-m24.

On the other hand, there was no significant difference in frequency in a single SNP analysis (Table 1B). No high frequency SNP that alters an amino acid coding was found, except for exon 9, and this SNP was not associated with sPD.

**[Table 1A]**

| HAPLOTYPE | | | HAPLOTYPE FREQUENCY | | | |
|---|---|---|---|---|---|---|
| **m22,m23,m24** | | | **sPD** | **control** | **Individual-p** | **OR(9S % CI)** |
| G | A | T | 0.462 | 0.506 | 0.0689 | 0.83 (0.67-1.01) |
| A | A | C | 0.198 | 0.196 | 0.9849 | 1.01 (0.77-1.30) |
| A | G | T | 0.131 | 0.134 | 0.8523 | 0.98 (0.71-1.31) |
| G | A | C | 0.093 | 0.051 | 0.0002 | 1.91 (1.25-2.79) |
| G | G | T | 0.070 | 0.060 | 0.4753 | 1.16 (0.77-1.75) |
| A | A | T | 0.052 | 0.050 | 0.9371 | 1.03 (0.65-1.65) |

**[Table 1B]**

| marker | | 2 | rs | pchr | pbp(build35) | int | type | freqcase | 1 Freqcont | ap | OR | 95%Cl | dp | OR | rp | OR | hp-cont | null rate |
|---|---|---|---|---|---|---|---|---|---|---|---|---|---|---|---|---|---|---|
| m19 | A | G | rs10886424 | 10 | 120967753 | 63935 | Intron 1 | 0.516 | 0.5662 | 0.0632 | 0.82 | 0.66-1.01 | 0.269 | 0.82 | 0.0513 | 0.72 | 0.633 | 0.0128 |
| m20 | A | C | rs11198856 | 10 | 121031688 | 21009 | Intron 1 | 0.6426 | 0.6194 | 0.3836 | 1.1 | 0.89-1.37 | 0.514 | 1.17 | 0.4465 | 1.12 | 0.7679 | 0.0064 |
| m21 | C | G | rs1473799 | 10 | 121052697 | 6367 | Intron1 | 0.1937 | 0.1832 | 0.6366 | 1.07 | 0.82-1.39 | 0.6915 | 1.07 | 0.731 | 1.14 | 0.0535 | 0.0064 |
| m22 | A | G | rs871196 | 10 | 121059064 | 7422 | Intron1 | 0.3811 | 0.3821 | 1 | 1 | 0.81-1.23 | 0.7608 | 0.95 | 0.7558 | 1.08 | 0.9091 | 0.0013 |
| m22.1 | A | G | rs2420616 | 10 | 121066486 | 19949 | Intron 1 | 0.2727 | 0.2626 | 0.6777 | 1.05 | 0.83-1.33 | 0.6027 | 1.09 | 1 | 1 | 0.7911 | 0.0013 |
| m23 | A | G | rs7069375 | 10 | 121086435 | 5253 | lntron2 | 0.799 | 0.8047 | 0.7921 | 0.96 | 0.75-1.25 | 0.5705 | 0.79 | 1 | 0.99 | 0.8076 | 0.0038 |
| m24 | C | T | rs4752293 | 10 | 121091688 | 22803 | lntron2 | 0.2875 | 0.2465 | 0.0832 | 1.23 | 0.98-1.56 | 0.0307 | 1.39 | 1 | 1 | 0.7978 | 0.0038 |
| m25 | A | G | rs884970 | 10 | 121114491 | 5296 | Intron2 | 0.7045 | 0.7302 | 0.2918 | 0.88 | 0.7-1.11 | 0.779 | 0.9 | 0.2646 | 0.84 | 0.9782 | 0.0051 |
| m26 | A | G | rs291979 | 10 | 121119787 | 10524 | Intron2 | 0.1626 | 0.1599 | 0.8865 | 1.02 | 0.77-1.35 | 0.8089 | 1.04 | 1 | 0.86 | 0.3778 | 0.0038 |
| m27 | C | T | rs2275036 | 10 | 121130311 | 17309 | Intron2 | 0.6351 | 0.6066 | 0.2783 | 1.13 | 0.91-1.4 | 0.4524 | 1.2 | 0.3552 | 1.16 | 0.5018 | 0.0128 |
| m28 | A | G | rs11198907 | 10 | 121147620 | 27079 | Intron4 | 0.2281 | 0.2196 | 0.7049 | 1.05 | 0.82-1.34 | 1 | 1 | 0.2825 | 1.49 | 0.2048 | 0.0051 |
| m29 | A | T | rs3740564 | 10 | 121174699 | 3955 | Intron6 | 0.1578 | 0.1636 | 0.8296 | 0.96 | 0.72-1.27 | 0.8706 | 0.97 | 0.8108 | 0.8 | 0.955 | 0.0115 |
| m30 | C | T | rs933048 | 10 | 121178654 | 7307 | Intron6 | 0.3794 | 0.3804 | 1 | 1 | 0.81-1.23 | 0.9391 | 1.01 | 0.9155 | 0.96 | 0.9615 | 0.0102 |
| m31 | A | C | rs11198922 | 10 | 121185961 | 364 | Intron8 | 0.8059 | 0.8164 | 0.6375 | 0.93 | 0.72-1.21 | 0.2526 i | 0.65 | 0.9373 | 0.98 | 0.8538 | 0.0051 |
| m32 | G | A | rs12718341 (rs2230349 in build33) | 10 | 121186325 | 20726 | exon9(R304H) | 0.7228 | 0.7388 | 0.513 | 0.92 | 0.73-1.16 | 0.4904 | 0.82 | 0.6543 | 0.93 | 0.5689 | 0.0077 |
| m33 | A | T | rs1999628 | 10 | 121207051 | | Intergenic | 0.3127 | 0.3014 | 0.6471 | 1.05 | 0.84-1.32 | 1 | 0.99 | 0.2978 | 1.34 | 0.323 | 0.0115 |

Gel-shift assays and luciferase assays were conducted using a dopaminergic human neuronal cell line SH-SY5Y to investigate whether there is a functional intron SNP among registered SNPs in these haplotypes. As a result, two SNPs were found. One was m22.1 (rs2420616) which had not yet been typed, and the other was m24 which had been typed. Haplotype scanning was conducted on 16 SNPs further including the newly typed m22.1 according to a sliding window approach in the same way as above. The same results as in the results of SNPs not including m22.1 were obtained. Haplotypes by functional SNPs m22.1 and m24 were analyzed, and a susceptibility haplotype G-C with a very low frequency of OR = 3.434 was identified (PD group 4%, control group 1.2%, OR = 3.43, 95% confidence limit 1.69-6.94, p = 0.00012). Haplotype blocks are shown in Table 2.

**[Table 2]**

| D' | | | | | | | | | | | | | | | | |
|---|---|---|---|---|---|---|---|---|---|---|---|---|---|---|---|---|
| | m19 | m20 | m21 | m22 | m22.1 | m23 | m24 | m25 | m26 | m27 | m28 | m29 | m30 | m31 | m32 | m33 |
| m19 | | 0.039 | **0.307** | 0.054 | **0.178** | 0.121 | 0.177 | 0.189 | 0.134 | 0.105 | 0.061 | 0.172 | 0.052 | 0.054 | 0.069 | 0.119 |
| m20 | | | **0.501** | **0.703** | **0.618** | 0.268 | **0.513** | 0.075 | 0.035 | 0.284 | **0.356** | **0.325** | 0.161 | **0.336** | 0.257 | 0.129 |
| m21 | | | | **0.897** | **0.539** | **0.644** | **0.562** | **0.522** | **0.706** | **0.493** | 0.296 | 0.184 | **0.32** | 0.149 | 0.033 | 0.145 |
| m22 | | | | | | **0.497** | **0.594** | 0.156 | 0.14 | **0.433** | **0.341** | 0.025 | 0.22 | **0.32** | 0.282 | **0.109** |
| m22.1 | | | | | | **0.97** | **0.885** | **0.715** | **0.562** | **0.342** | 0.046 | **0.516** | 0.02 | 0.096 | **0.328** | 0.221 |
| m23 | | | | | | | **1.002** | **0.603** | **0.889** | **0.702** | **0.359** | 0.037 | 0.226 | 0.202 | 0.057 | 0.073 |
| m24 | | | | | | | | **0.039** | **0.709** | **0.444** | 0.027 | **0.609** | 0.18 | 0.07 | **0.301** | 0.269 |
| m25 | | | | | | | | | **0.997** | **0.88** | **0.314** | **0.751** | **0.839** | 0.204 | **0.856** | |
| m26 | | | | | | | | | | **0.799** | **0.688** | **0.652** | **0.582** | **0.454** | 0.226 | 0.168 |
| m27 | | | | | | | | | | | **0.908** | **0.759** | **0.667** | **0.578** | 0.18 | 0.004 |
| m28 | | | | | | | | | | | | **0.921** | **0.531** | **0.549** | 0.165 | 0.206 |
| m29 | | | | | | | | | | | | | **0.866** | **1.003** | **0.826** | **0.469** |
| m30 | | | | | | | | | | | | | | **1.008** | **0.639** | **0.592** |
| m31 | | | | | | | | | | | | | | | **0.999** | **0.906** |
| m32 | | | | | | | | | | | | | | | | **0.804** |

| p-value | | | | | | | | | | | | | | | | |
|---|---|---|---|---|---|---|---|---|---|---|---|---|---|---|---|---|
| | m19 | m20 | m21 | r n22 | m22.1 | m23 | m24 | m25 | m26 | m27 | m28 | m29 | m30 | m31 | m32 | m33 |
| m19 | | 0.43104 | **0** | 0.18468 | **0.00731** | 0.06606 | **0.00836** | **0.00024** | 0.06685 | **0.03056** | 0.29878 | **0.0172** | 0.20598 | 0.41663 | 0.25426 | **0.05071** |
| m20 | | | **0** | **0** | **0** | **0** | **0** | 0.07932 | 0.57159 | **0** | **0** | **0.00202** | **0.00001** | **0** | **0** | **0.0023** |
| m21 | | | | **0** | **0** | **0** | **0** | **0** | **0.00001** | **0** | **0** | **0.00001** | **0** | **0,00004** | 0.72919 | 0.15945 |
| m22 | | | | | **0** | **0** | **0** | **0.0003** | **0.03316** | **0** | **0** | 0.65478 | **0** | **0** | **0** | **0.01058** |
| m22.1 | | | | | | **0** | **0** | **0** | **0** | **0.00001** | 0.23347 | **0.00003** | **0.63908** | **0.03021** | **0** | **0** |
| m23 | | | | | | | **0** | **0** | **0** | **0** | **0** | **0.35942** | **0.00006** | **0** | 0.16593 | 0.47087 |
| m24 | | | | | | | | **0** | **0** | **0** | 0.47087 | **0** | **0.02041** | **0.11247** | **0** | **0** |
| m25 | | | | | | | | | **0** | **0** | **0** | **0** | **0** | **0.00001** | **0** | **0** |
| m26 | | | | | | | | | | **0** | **0** | **0.00019** | **0** | **0.0134** | **0.00001** | **0.00283** |
| m27 | | | | | | | | | | | **0** | **0** | **0** | **0** | **0.01625** | 1 |
| m28 | | | | | | | | | | | | **0** | **0** | **0** | 0.1278 | **0.04** |
| m29 | | | | | | | | | | | | | **0** | **0** | **0** | **0.00003** |
| m30 | | | | | | | | | | | | | | **0** | **0** | **0** |
| m31 | | | | | | | | | | | | | | | **0** | **0** |
| m32 | | | | | | | | | | | | | | | | **0** |

Interestingly, m22.1 and m24 are in a relationship close to absolute linkage disequilibrium (D' = 89, Δ = 0.88), and it was found that there is an intense linkage between the alleles. In fact, both the two alleles of the susceptibility haplotype G-C were alleles acting to increase luciferase activity (G in m22.1, and C in m24). Further detailed investigations revealed that the presence or absence of this susceptibility haplotype G-C nearly completely agreed with the presence or absence of the susceptibility haplotype G-A-C (m22m23m24) first identified. Namely, the m22.1m24 haplotype with a lower frequency was considered to be derived from the original haplotype m22m23m24.

### [Example 2] Functional Analysis

Gel-shift assays, supershift assays, and luciferase assays were carried out to verify the transcriptional activity of SNP m24 in intron 2.

### (2-1) Gel-shift Assay on SNP m24

Initially, gel-shift assays and competitive assays on SNP m24 were conducted. Nuclear extract of SH-SYSY cells was reacted with ³²P-labeled oligonucleotides of disease-insusceptible T allele (m24T: 5' cagaagactcTgtcatcaggc/SEQ ID NO: 2) and ³²P-labeled oligonucleotides of disease-susceptible C allele (m24C: 5'cagaagactcCgtcatcaggc/SEQ ID NO: 3). As a competitor (competitive substance), unlabeled m24C probes were added in excess of 200 folds.

As a result, as is shown in Fig. 3A, shift bands (arrowhead) were seen in lanes 1 and 5, and the band intensity was higher in m24C (lane 5) than in m24T (lane 1), indicating that the disease-susceptible C allele has a higher affinity to nucleoproteins than the disease-insusceptible T allele. This was also indicated from data that inhibition by the competitor (unlabeled oligonucleotide probe) more strongly reduces the shift band in m24C (lanes 4 and 8) than in m24T (lanes 3 and 7). No inhibition of shift bands occurred in the control (lanes 2 and 6). (2-2) Supershift Assay on SNP m24

The nucleotide sequence of this site had a high homology to CRE (cAMP response element) (Gonzalez GA, Yamamoto KK, Fischer WH, Karr D, Menzel P, Biggs W 3rd, Vale WW, Montminy MR. A cluster of phosphorylated sites on the cyclic AMP-regulated nuclear factor CREB predicted by its sequence. Nature 337, 749-752 (1989).). Accordingly, supershift assays were conducted on SNP m24. ³²P-labeled oligonucleotides of m24T or m24C were reacted with the nuclear extract of SH-SY5Y cells. Antibodies against CRE-binding protein 1 (CREB-1), anti-c-JUN antibodies, and control antibodies (anti-STAT 1 antibody) were used.

As a result, antibodies against CREB1 diminished the shift bands (arrowhead: lanes 5 and 10) and produced two supershift bands (double arrowhead: lanes 5 and 10) which were further shifted than the shift band (Fig. 3B). These bands were more strongly observed in the C allele than in the T allele. This indicates that CREB-1 binds with the C allele more firmly than with the T allele. Anti-c-JUN antibody also produced supershift bands (triple arrowhead: lanes 3 and 8). The intensity of the supershift bands were higher in m24C (lanes 8 and 10) than in m24T (lanes 3 and 5). Control antibodies (anti-STAT-1 antibody) did not affect the intensity of shift bands and did not produce supershift bands (lanes 2 and 7). (2-3) Luciferase Assay on SNP m24

To determine the transcriptional activities of these alleles, a sequence containing SNP m24 was inserted into a pGL-3 promoter vector to measure the expression level of luciferase. Specifically, one copy or three copies of m24T or m24C oligonucleotides were inserted in tandem into the pGL-3 promoter vector, the vector was introduced into SH-SY5Y cells, and the relative luciferase activity (RLA) was measured.

As a result, as is shown in Fig. 3C, there was no difference in RLA in the absence of cAMP among the five vectors. In the presence of cAMP, the insertion of one copy of C allele (m24C) showed a 1.5-fold increase in RLA (p<0.05) compared with the insertion of one copy of T allele (m24T). Insertion of three copies of C allele (m24C) showed 9.7-fold increase in RLA (p<0.0001) compared with the insertion of three copies of T allele (m24T).

In addition, chromatin immunoprecipitation (ChIP) revealed that endogenous CREB-1 binds with the m24 region in SH-SY5Y cells (Fig. 3D).

ChIP was conducted according to the kit manufacturer's manual (Upstate). SH-SY5Y cells were fixed in 1% formaldehyde at 37°C for 10 minutes. The cells were fractionated by the application of ultrasound with ice-cooling to yield soluble chromatin complexes having DNA fragment lengths of < 2 kb. Samples each containing 1 x 10⁶ cells were subjected to immunoprecipitation using no antibody, 5 µg of unrelated antibodies, or 5 µg of anti-CREB-1 antibody (C-21). After removing the proteins, DNA was amplified using a set of primers (5'-tgccggagttcgactggtcag-3' (SEQ ID NO: 10) and 5'-tagcagctcaatggctttgtc-3' (SEQ ID NO: 11)).

### (2-4) Gel-Shift Assay and Competitive Assay on SNP m22.1

The present invention also verified the function of m22.1 present in intron 1 through gel-shift assay. ³²P-labeled oligonucleotides of disease-insusceptible A allele (m22.1A: 5'taaactcagatAtggcttcaggg/SEQ ID NO: 4) or ³²P-labeled oligonucleotides of disease-susceptible G allele (3 m22.1G: 5'taaactcagatGtggcttcaggg/SEQ ID NO: 5) were reacted with nuclear extract from SH-SY5Y cells.

As a result, shift bands of DNA-protein complexes (arrowhead and double arrowhead) were observed in m22.1 A lanes (1 to 3) using the disease-insusceptible A allele as a probe, but not found in m22.1 G (lane 3) using the disease-susceptible G allele as a probe (Fig. 4A). Addition of m22.1A competitors (unlabeled oligonucleotide probes) diminished the shift bands, but addition of m22.1G competitors did not eliminate the shift band (lane 3). These indicate that nucleoproteins bind with m22.1 A.

### (2-5) Competitive Assay on SNP m22.1 Using YY1 (Yin Yang-1)-binding Motif Oligonucleotides

The nucleotide sequence of this site has a homology to the binding site with YY- 1 which is a transcriptional regulator that sometimes activate and sometimes suppress transcription (Shrivastava A, Calame K. An analysis of genes regulated by the multi-functional transcriptional regulator Yin Yang-1. Nucleic Acids Res 22, 5151-5155 (1994).). Accordingly, an inhibition assay was conducted by an oligonucleotide containing a YY-1-binding site. ³²P-labeled oligonucleotide of m22.1A has a YY1-binding site (5'taaactcagatAtggcttcaggg/SEQ ID NO: 6). This was reacted with the nuclear extract from SH-SY5Y cells. Unlabeled YY1-binding sequence (YY1co: 5'cagccgccaagatggccggggag/SEQ ID NO: 7), unlabeled mutant YY1 sequence (YY1mu: 5'cagccgccaagataatcgcggag/SEQ ID NO: 8), and unlabeled mutant m22.1A (m22.1Amu: 5'taaactcagatAcacctcaggg/SEQ ID NO: 9) were each added.

The results show that addition of unlabeled YY1-binding sequence nearly completely eliminated a shift band (triple arrowhead) (YY1co: lane 5). However, addition of unlabeled mutant YY1 sequence (YY1mu: lane 6), unlabeled mutant m22.1A (m22.1Amu: lane 3), and unlabeled m22.1G (lane 4) had no effect (Fig. 4B).

### (2-6) Supershift Assay on SNP m22.1

Supershift assays using anti-YY 1 antibodies were conducted. Nuclear extract from SH-SY5Y cells was reacted with ³²P-labeled oligonucleotide of m22.1A, m22.1Amu, m22.1G, YY1co, or YY1mu.

As is shown in Fig. 4C, shift bands (double arrowhead) occurred in the m22.1A (lane 1) and YY1co (lane 7) ³²P-labeled oligonucleotides. This band nearly completely disappeared by the addition of anti-YY1 antibodies (lanes 2 and 8). These results indicate that YY1 binds with m22.1A (A allele), as with YY1co.

### (2-7) Luciferase Assay on SNP m22.1

One copy or three copies of m22.1A or m22.1G oligonucleotides were inserted in tandem into a pGL-3 promoter vector, the vector was introduced into SH-SY5Y cells, and RLA was measured through luciferase assays.

As a result, when three copies were introduced, m22.1A oligonucleotide showed 42% reduction in RLA as compared to the other four vectors (p<0.0001) (Fig. 4D). This demonstrates that YY1 suppresses transcriptional activity. (2-8) Immuno-histochemical Staining of Nigras of sPD Patients

To verify the relation between GRK5 and sPD, immuno-histochemical studies were conducted using the brains of PD patients (humans).

Specifically, formalin-fixed, paraffin-embedded tissue sections (6 µm thickness) of the midbrain extirpated from six autopsied patients with sPD were deparaffinized and heated in citric acid solution of pH 6.0 by using a microwave oven to expose the antigen. The sections were reacted with a 1 % hydrogen peroxide solution for 10 minutes to block endogenous peroxidase activity and reacted with PBS added with 5% normal goat serum and 0.03% TritonX for 30 minutes, to block unspecific reactions. The sections were incubated with polyclonal antibodies against human GRK1, GRK2, GRK3, GRK4, GRK5, and GRK6 (Santa Cruz Biotechnology, Inc.) at 4°C overnight, and reacted with biotinylated secondary antibodies and then with peroxidase-labeled avidin-biotin complex. The sections were color developed with 3,3'-diaminobenzidine tetrahydrochloride and ammonium nickel sulfate. Tissue sections of the brains from patients with multiple system atrophy and patients with Alzheimer disease were also subjected to immuno-histochemical staining in the same manner.

As a result, it was discovered that GRK5 accumulates in Lewy bodies which is the pathological characteristics of PD (Fig. 5A). Lewy neurites were also GRK5-positive (Figs. 5B and C). Confocal laser microscopic images showed that GRK5 colocalized αS in Lewy bodies (Figs. 5D to F). Lewy bodies were not stained with respect to GRK1, GRK2, GRK3, GRK4, and GRK6. GRK5 was not detected in αS-positive glial cell cytoplasmic inclusions (GCI) which is the pathological characteristics of multiple system atrophy. Neurofibrillary tangle (NFT) and senile plaque (SP) for Alzheimer disease were also GRK5 negative. The result that GRK5 was present in Lewy bodies but not present in GCI, NFT, and SP indicates that GRK5 is specifically involved in the pathology of sPD.

### [Example 3] Biological Effect of GRK5 on αS

The relation between GRK5 and sPD was revealed in the present invention as described in Example 2 above. The biological role of GRK5 in the onset of sPD was investigated using HEK293 cells in which GRK5 and αS cDNA were coexpressed, while focusing on GRK5 and on phosphorylation of αS.

Since p-αS is immediately dephosphorylated, cells were treated with okadaic acid (Wako Pure Chemical Industries, Ltd.). Okadaic acid is a specific inhibitor of PP1 and PP2A which are isozymes of type I protein phosphatases (Okochi M, Walter J, Koyama A, Nakajo S, Baba M, Iwatsubo T, Meijer L, Kahle PJ, Haass C. Constitutive phosphorylation of the Parkinson's disease associated alpha-synuclein. J Biol Chem. 275, 390-397 (2000).). Phosphorylated αS (p-αS) was detected in GRK5-expressing cells treated with okadaic acid dependently on the dose of okadaic acid and the time by antibodies against p-αS in which serine at position 129 is phosphorylated (Saito Y, Kawashima A, Ruberu NN, Fujiwara H, Koyama S, Sawabe M, Arai T, Nagura H, Yamanouchi H, Hasegawa M, Iwatsubo T, Murayama S. Accumulation of phosphorylated alpha-synuclein in aging human brain. J. Neuropathol. Exp. Neurol. 62, 644-654 (2003).) (Fig. 6).

Specifically, vehicle vectors or GRK5-FLAG cDNA was introduced into HEK293 cells stably expressing human wildtype αS. 30 hours after introduction, okadaic acid in concentrations of 0, 5, 10, and 20 nM was added to the medium, and the cells were further cultured for 16 hours. Cell lysates added with 20 µg of 1% NP-40 were added to respective lanes of a 12.5% Tris-glycine gel, electrophoresed, and transferred to a PVDF membrane. Western blotting was conducted using FLAG, mouse monoclonal anti-αS antibody Syn-1 (BD Transduction Laboratories), mouse monoclonal anti-phosphorylated Ser-129 αS antibody psyn#64, or mouse monoclonal anti-β-actin antibody AC-15 (Sigma), which are antibodies recognizing GRK5-FLAG, total αS, Ser-129 phosphorylated αS, and β-actin, respectively.

The Western blotting was conducted in the following manner. All protein samples were boiled in a Laemmli sample buffer containing 2.5% 2-mercaptoethanol for 5 minutes before electrophoresis. The proteins in equivalent amounts were electrophoresed on a 12.5% polyacrylamide gel and then transferred to PVDF membranes (Millipore Corporation). The membranes were reacted with primary antibodies at 4°C overnight and then reacted with secondary antibodies labeled with horseradish peroxidase. Color development was conducted using ECL plus or ECL advance (Amersham Pharmacia Biotech).

As a result, Ser-129 p-αS was detected in GRK5-expressing cells in proportion to the concentration of okadaic acid by staining with psyn#64 (Fig. 6A).

In addition, a vehicle vector or GRK5-FLAG cDNA was introduced into HEK293 cells stably expressing human wildtype αS, 20 nM of okadaic acid was added to the medium 24 hours after introduction, and the cells were cultured for 0, 6, 12, and 24 hours (up to 24 hours). The cells at 0 hour were sampled as a starting point of okadaic acid treatment.

As a result, the amount of Ser-129 p-αS (Ser-129 αS) in GRK5-expressing cells increased as the culture time increased in the presence of okadaic acid (Fig. 6B). A trace signal of Ser-129 p-αS was observed in cells which had been cultured in the presence of okadaic acid for 24 hours after introduction of a vehicle vector. Unidentified kinase is estimated to be involved in the phosphorylation of Ser-129 αS.

### [Example 4] Effects of Varying Expression Level and Varying Enzymatic Activity of GRK5 on the Phosphorylation of αS

To search a biological relation between phosphorylation of Ser at position 129 of αS and GRK5, Western blotting was conducted using FLAG, Syn-1, and psyn#64 antibodies recognizing GRK5-FLAG, total αS, and Ser-129 p-αS, respectively, and the following study was performed.

HEK293 cells and human neuroblastoma-derived cells SH-SY5Y were cultured in Dulbecco's Modified Eagle Medium added with 10% fetal bovine serum, and in a 1:1 mixed culture medium of DMEM containing 10% fetal bovine serum and Ham's F-12 (Invitrogen Corp.), respectively, under humidified incubation conditions of 37°C, 5% CO₂. HEK239 cell line stably expressing αS was selected using 1 mg/ml of G418 (Invitrogen Corp.), with neomycin resistance as an index.

GRK5-FLAG cDNA (0, 0.1, 0.5, 1, and 2 µg) was introduced into HEK239 cell line stably expressing αS, using the Lipofectamine PLUS Reagent (Invitrogen Corp.). 30 hours after transformation, 20 nM of okadaic acid was added, and the cells were further cultured for 16 hours. As a result, the amount of total αS remained almost the same, but the amount of p-αS increased with an increasing amount of GRK5 (Fig. 7A, upper panel).

In addition, phosphorylation of αS at Ser-129 was observed in cells transiently co-expressing wildtype GRK5 and αS (Fig. 7A, lower panel). In contrast, no phosphorylated αS was observed in cells transiently co-expressing wildtype GRK5 and S129A mutant in which the serine residue at position 129 of αS was substituted by alanine. No phosphorylation of αS was observed in cells transiently co-expressing wildtype αS and mutant GRK5 in which the 215^{th} lysine residue in the enzyme active center of GRK5 was substituted by arginine.

Namely, it was demonstrated that the expression level of GRK5 protein shows a positive correlation with the expression level of p-αS stabilized by okadaic acid (Fig. 7A). In addition, phosphorylation of αS by GRK5 was observed in SH-SY5Y cells, which are human neuroblastoma-derived cells.

### [Example 5] Investigation of Binding Between GRK5 and αS Through Immunoprecipitation

To search whether GRK5 binds with αS, immunoprecipitation was conducted. No binding between GRK5 and αS was observed in a common immunoprecipitation method using cell lysates prepared by extracting HEK293 cells with surfactant-containing buffer (the immunoprecipitation conditions were the same as with the after-mentioned method, except for not using a crosslinker). In contrast, binding between GRK5 and αS could be detected by treating cultured cells or human brain homogenate with membrane-permeable crosslinkers (crosslinking agents).

Specifically, a vehicle vector or GRK5-FLAG cDNA was transiently introduced into HEK293 cells stably expressing αS. The transformed HEK293 cells were cultured on a 100-mm plate, washed twice with cooled PBS buffer, the buffer was replaced with 6 ml of crosslinking buffer (10 mM HEPES pH 7.4, PBS containing 2.5 mM dithiobis(succinimidyl propionate)) (DSP, Pierce, Rockford, Illinois, USA), and reacted at room temperature for 30 minutes. The crosslinking reaction was completed by quickly removing the crosslinking buffer. The cells were added with 1 ml of ice-cooled lysis buffer (50 mM Tris-HCl, pH 7.6, 150 mM NaCl, 1 mM EDTA, 10% glycerol, 1 % Nonidet P-40, protease inhibitor), centrifuged at 12000x g for 30 minutes, and the supernatants were used in immunoprecipitation experiments. The cell lysates were pretreated with protein G agarose beads for 30 minutes to remove unspecific bindings, added with 2 µl of Syn-1 antibodies, and reacted at 4°C overnight. They were reacted with protein G agarose beads for 2 hours, thereafter the beads were washed three times with the lysis buffer, and the immunoprecipitated proteins were dissolved out with Laemmli sample buffer. Samples in equivalent amounts were analyzed by Western blotting.

As a result, binding between GRK5 and αS was observed in cells expressing GRK5 by Western blotting using anti-GRK5 antibodies (Fig. 7B, upper panel).

A further verification was conducted using human temporal lobe cortex tissues. The cortex of the temporal lobe of human brain was homogenized with PBS added with protease inhibitor in a Teflon homogenizer, and centrifuged at 800x g for 5 minutes. The supernatant was added with DSP to a final concentration of 2.5 mM and gently shaken at room temperature for 30 minutes. The crosslinking reaction was quenched with Tris (pH 7.6) in a final concentration in the reaction mixture of 50 mM. The brain homogenate was centrifuged at 100,000x g for 30 minutes to yield pellets, the pellets were added with ice-cooled lysis buffer at a ratio of 1:5 (weight/volume), and extracted using a 27-gauge injection needle. The extract was centrifuged at 12,000x g for 30 minutes, and the supernatant was subjected to immunoprecipitation in the same way as described above. The tissue lysate was subjected to immunoprecipitation with anti-FLAG (negative control), Syn-1, or anti-GRK5 antibodies.

As a result, GRK5 coprecipitated with αS was detected by Western blotting with anti-GRK5 antibodies (Fig. 7B, lower panel).

This indicates that GRK5 and αS are present in a relation very close in distance, but binding between them is weak or transient (Fig. 7B).

### [Example 6] Immunocytochemical Analysis of Intracellular Localization of GRK5 and αS

To morphologically verify the relation between GRK5 and αS, cells were observed with a confocal laser microscope.

HEK293 cells stably expressing αS were cultured on a 4-chamber slide (Nalge Nunc International). Wildtype GRK5-FLAG or mutant GRK5 K215R-FLAG cDNA was transiently transduced into the cells, and the cells were cultured in the absence or presence of okadaic acid. 48 hours after transduction, the cells were washed twice with PBS buffer and fixed in a 4% formaldehyde solution at room temperature for 15 minutes. The fixed cells were then washed three times with PBS, subjected to blocking with PBS containing 5% skimmed milk and 0.05% TritonX-100 for 1 hour, and reacted with rabbit polyclonal anti-GRK5 antibodies (H-64), mouse monoclonal anti-αS antibodies (LB509), or psyn#64 at 4°C overnight. After washing, the cells were reacted with a mixture of Alexa 488-labeled anti-mouse IgG (green) and Alexa 568-labeled anti-rabbit IgG (red) (Molecular Probes) at 37°C for 1 hour. Images were obtained with Zeiss LSM 510 META Laser Scanning Microscope.

As a result, wildtype GRK5 was mainly localized in the cell membrane and was hardly observed in the perikaryon (Fig. 8). Wildtype αS was mainly present in the perikaryon cytoplasmic domain and co-localized with GRK5 in the cell membrane region. The co-localization site is a site indicated by yellow in the merged images (Fig. 8, left panel). Ser-129 p-αS was present in the cell membrane and the perikaryon cytoplasmic domain in cells expressing wildtype GRK5 and was not observed in cells expressing mutant GRK5 K215R. Intracellular localization of mutant GRK K215R5 was the same as with the wildtype. In the presence of okadaic acid, Ser-129-p-αS was observed only in cells expressing wildtype GRK5, and was not observed in cells expressing K215R which is the mutant GRK5.

### [Example 7] Changes in Intracellular Localization of Phosphorylated αS Associated with Okadaic Acid Treatment

Changes in intracellular localization of Ser-129 p-αS catalyzed by GRK5 was further verified. Wildtype GRK5-FLAG cDNA was transiently introduced into HEK293 cells stably expressing wildtype αS. The cells were treated with okadaic acid for predetermined time periods (0, 3, 6, 12, and 24 hours). The cells were fixed and subjected to immunochemical staining with anti-GRK5 antibodies (red) or anti-p-Ser-129 αS antibodies (psy#64, green).

As a result, p-αS was formed in the cell membrane region, where GRK5 and αS colocalized, after a three-hour reaction in the presence of okadaic acid (Fig. 7C). Thereafter, p-αS left the cell membrane region where GRK5 localized, and migrated to the perikaryon cytoplasmic domain.

### [Example 8] Effects of GRK5 on αS Oligomerization

To study the effects of GRK5 on the αS oligomerization under non-denaturing conditions, GRK5 cDNA was introduced into HEK293 cells stably expressing wildtype αS and treated the cells with α-linolenic acid and okadaic acid for a predetermined time. Soluble fraction (supernatant after 370,000x g centrifugation) (Sharon R, Goldberg MS, Bar-Joseph I, Betensky RA, Shen J, Selkoe DJ. α-Synuclein occurs in lipid-rich high molecular weight complexes, binds fatty acids, and shows homology to the fatty acid-binding proteins. Proc Natl Acad Sci USA 98, 9110-9115 (2001).) was extracted from the obtained cells, and analyzed through gel filtration. Specifically, this was conducted in the following manner.

Chloramphenicol acetyltransferase (CAT) cDNA as a control, or GRK5 cDNA was transiently introduced into HEK293 cells stably expressing wildtype αS. 24 hours after transduction, 20 nM okadaic acid was added to the cell culture. After further culturing for 8-hours, α-linolenic acid (unsaturated fatty acid) and BSA in a molar ratio of 5:1 were added to the buffer (10 mM Tris-HCl, pH 8.0, 150 mM NaCl) and reacted at 37°C for 30 minutes to form α-linolenic acid/BSA complexes. The culture medium was replaced with a fresh culture medium added with 20 nM okadaic acid and the α-linolenic acid/BSA complex to final concentrations of 500 µM α-linolenic acid and 100 µM BSA, and the cells were further cultured for 16 hours.

Protein fractionation of the cells was conducted according to the Sharon's method (Sharon et al, 2001) with partial modifications. All operation processes were conducted at 4°C. The cells were recovered, suspended in homogenization buffer (20 mM HEPES, pH 7.4, 1 mM MgCl₂, 0.32 M sucrose, 43 mM 2-mercaptoethanol, 1x protease inhibitor cocktail (Roche Diagnostics) in a ratio of 1: 3 (weight/volume), and homogenized in a Teflon homogenizer while repeating vertical motion twenty times. The homogenate was centrifuged at 200x g for 10 minutes, and the supernatant was further centrifuged at 8000x g for 15 minutes. The resulting supernatant was further centrifuged at 370,000x g for 1 hour (S370 cytosol). Protein concentrations were measured according to the Bradford assay (Bio-Rad Laboratories).

For gel filtration, molecular weight markers or 300 µg of S370 cytosol obtained from the cells was added to the Superdex 75 10/300 Column (bed volume 24 ml, Amersham Pharmacia Biotech). Elution was conducted with 50 mM ammonium acetate (pH 7.4) containing 20 nM okadaic acid at a flow rate of 6 ml/h using the FPLC System (Amersham Pharmacia Biotech). Fractions 0.5 ml each were fractionated, of which 0.25 ml was lyophilized, and resuspended in a homogenization buffer. This sample was subjected to heat treatment at 65°C for 16 hours according to the Sharon's method (Sharon et al, 2001) to delipidate, added with Laemmli sample buffer containing 2.5% 2-mercaptoethanol, subjected to SDS-PAGE, transferred to PVDF membrane, and analyzed using anti-αS antibodies (Syn-1) or anti-p-Ser129αS antibodies (psyn#64).

In addition, to observe αS oligomerization associated with the treatment time by unsaturated fatty acid, HEK293 cells were allowed to coexpress αS with CAT, or αS with GRK5, and the α-linolenic acid/BSA complex was added to the culture medium for 0, 3, 6, or 9 hours. Okadaic acid was also added to the cells coexpressing αS and GRK5. S370 cytosols were prepared from these cells, delipidated by heat treatment at 65°C for 16 hours, and analyzed by Western blotting.

As a result, the amount of αS oligomerization induced by α-linolenic acid was enhanced in αS containing phosphorylated molecules observed in the HEK293 cells coexpressing GRK5, as compared to the non-phosphorylated αS observed in the HEK293 cells coexpressing CAT (Fig. 7D, left and middle panels). The αS oligomerization was also seen in non-denaturing conditions, namely when the S370 cytosol was fractionated through gel filtration column before heat treatment (Fig. 7D, right panel).

### [Example 9] Plasmid Production

Full-length cDNAs of wildtype and kinase-inactive (K215R) GRK5 both added with FLAG tag at the C-terminus were amplified by PCR. The set of primers used herein was as follows. Sense primer (with Spe-I-end): 5'-CCCACTAGTATGGAGCTGGAAAACATCGT-3' (SEQ ID NO: 12), and antisense primer (with NdeI-end): 5'-CCCATATGCTACTTGTCATCGTCGTCCTTG-3' (SEQ ID NO: 13). The PCR products were inserted into the Spe-I and Nde-I sites of vector pFX_UNC-119 (Madulo and pilgrim, 141(3):977-988, Genetics). Thus, Punc-119::GRK5 (wildtype) and Punc-119::GRK5 (K215R, kinase-inactive) were produced, in which the GRK5 gene was linked with Punc-119, which is a promoter of the unc-119 gene that is expressed in all neurons of nematodes.

The full-length cDNA of α-synuclein was supplied from Dr. S. Nakajo, Department of Biological Chemistry, Showa University School of Pharmaceutical Sciences, and cDNA of S129A (which is the phosphorylation site of α-synuclein) mutant α-synuclein was prepared as previously reported (Fujiwara H. et al., Nat Cell Biol. 4(2):160-164, 2002). The α-synuclein gene (S129A) was linked to Pdat-1, which is a promoter of the dat-1 gene which is expressed in dopamine neurons, to yield Pdat-1::α-synuclein (Kuwahara T. et al., J Biol Chem 281(1):334-340, 2006). Pmec-7, which is a promoter of the mec-7 gene which is expressed in touch neurons, was obtained from the genome of a wildtype nematode strain N2 by amplification through PCR (Savage c. et al., J Cell Sci. 107(Pt8):2165-2175). Next, the PCR product was inserted into a pFXneEGFP vector, and enhanced green fluorescent protein (EGFP) was linked with Pmec-7 to produce Pmec-7::EGFP. In addition, the EGFP region of Pmec-7::EGFP was replaced with α-synuclein to produce Pmec-7::α-synuclein.

Sequences of all plasmids were identified with an automatic sequencer (LI-COR Biosciences, Lincolin, NE) using Thermo Sequence^{™} (Amersham Biosciences).

### [Example 10] Production of Transgenic Nematode (C. elegans)

Nematodes were raised under previously reported standard conditions (Brenner s. et al., 77(1):71-94, Genetics, 1974). More specifically, the nematodes were raised on NGM (nematodes growth medium) agar plate at 20°C and fed with bacteria (*Escherichia coli* OP50 strain) as nutrients. A wildtype N2 Bristol strain was used for the production of transgenic nematodes. The prepared plasmids and marker plasmids were injected into the genital gland of the N2 strain monoecious nematode to prepare transgenic nematodes.

180 µg/ml of the prepared plasmid and, as a marker, Pmyo-2::dsRed (20 µg/ml) presenting red fluorescence in the throat were injected to the strain to be introduced with Punc-119::GRK5. 100 µg/ml of the prepared plasmid and, as a marker, pRF4 (rol-6) (100 µg/ml) which is a marker showing a roller phenotype were injected to the strain to be introduced with Pmec-7::α-synuclein. 100 µg/ml of the prepared plasmid and, as a marker, Pges-1::RFP (100 µg/ml) presenting red fluorescence in the small intestine were injected to the strain to be introduced with Pdat-1::α-synuclein,. The marker genes were supplied from Dr. S. Mitani, Tokyo Women's Medical University.

To obtain stable recombinant strains, the transgenes were incorporated into the chromosomes by ultraviolet irradiation (Mitani S. et al., Dev. Growth & Diff. 37:551-557, 1995). The strains in which the transgenes were incorporated into the chromosomes were subjected to outcrossing two to four times for stabilization.

To obtain a strain in which GRK5 and α-synuclein were coexpressed and the transgene was incorporated into the chromosomes, a strain overexpressing GRK5 (wildtype or mutant (K215R)) was crossed with a strain overexpressing α-synuclein.

### [Example 11] Age Culture

A population in uniform growth stage was obtained by treating adults in the ovipositional stage with a hypochlorite (Lewis JA. et al., Methods Cell Biol, 48:3-29, 1995). The population was cultured in M9 buffer at 20°C for 16 to 24 hours to yield L1 in uniform growth stage. The nematodes were plated on NGM agar plate and cultured at 20°C for 3 days. To obtain a nematode group in a later growth stage, the nematodes cultured for three days were cultured in M9 buffer containing 0.2 mg/ml of 5'-fluoro-2'-deoxyuridine (Wako Pure Chemical Industries, Ltd.) and NGM agar plate to suppress the development of a next generation (Honda S. et al., J Gelontol. 48(2):B57-61, 1993).

### [Example 12] Behavior Assay <Food-sensing Behavior Assay>

To evaluate the function of dopamine neurons of nematodes, Food-sensing Behavior Assay was conducted as previously reported (Kuwahara T. et al., J Biol Chem 281(1):334-340, 2006). When a normal nematode encounters food, the number of bending of the head decreases. However, a nematode of the cat-2 strain which lacks dopamine synthetase shows an abnormality that the number of bending does not decrease even when it encounters food. The deletion strain of cat-2 is equivalent to the wildtype N2, except that it hardly produces dopamine. Accordingly, the function of dopamine neurons of nematodes can be evaluated by food-sensing behavior assay in which the change in number of bending upon encounter with food is ethologically assayed. Concretely, nematodes in a uniform growth stage and supplied with sufficient nutrition were washed with M9 buffer and transferred to the center of a plate with bacteria plated as food or of a plate without bacteria. Five minutes after transfer, numbers of bending of respective nematodes at intervals of 20 seconds were measured. Slowing rates were calculated by subtracting the number of bending with bacteria from the number of bending without bacteria, dividing the result by the number of bending without bacteria, and expressing the result in unit of percent ({the number of bending without bacteria - the number of bending with bacteria}/the number of bending without bacteria x 100 (%)).

Five nematodes in each strain were subjected to the test, and the test was conducted seven times independently.

In addition, dopamine was administered as previously reported ( Kuwahara T. et al., J Biol Chem 281 (1):334-340, 2006) and motilities of respective nematodes were measured as noted previously.

Upon behavior assays, a measurer conducted measurements without being noticed the strains (genotypes) of nematodes.

### [Example 13] Touch Assay

Touch assays were conducted on sixty each of 3, 6, or 9-day old transgenic nematodes of each strain in a uniform growth stage. Nematodes were touched in the body five times with human eyelash, and their responses were scored. In a normal response, a nematode moves backward when it is lightly contacted at its anterior side, and moves forward when it is lightly contacted at its posterior side. If a nematode has abnormalities in touch neurons, it keeps on moving forward even when it receives contact stimulus. Scores of respective individuals were recorded in such a manner that a score was added when an individual showed normal response.

### [Example 14] Immunoblotting and Immunohistochemical Staining

Nematodes were washed three times with M9 buffer and recovered (Brenner s. et al., 77(1):71-94, Genetics, 1974). Samples for immunoblotting were prepared as follows. The nematodes were crushed with an equivalent amount of sample buffer (160 mM Tris-HCl, 4% SDS, 30% glycerol) by applying ultrasound, centrifuged at 10,000 rpm for 15 minutes, and supernatants were collected. Protein concentrations were measured according to BCA (Bicinchoninic Acid) method, samples were weighed so as to have equal protein amounts, and then electrophoresed in SDS-polyacrylamide gel. Next, they were transferred to polyvinylidene difluoride (PVDF) membranes (Millipore Corporation). After blocking in TBS buffer added with 5% skimmed milk and 0.1 % tween-20 for 1 hour, the membranes were incubated with anti-FLAG M2 monoclonal antibodies (1:1000) (Sigma) as a primary antibody at 4°C overnight. Next, the membranes were incubated with horseradish peroxidase-labeled anti-mouse IgG antibodies (Amersham Biosciences) as a secondary antibody. Staining was conducted according to a fluorescence chemiluminescence method in which specific reactions were enhanced.

For immuno-histochemical analyses, nematodes were fixed in 4% paraformaldehyde at 4°C overnight. They were embedded in paraffin and sliced into sections 3 µm thick. In immunochemical staining, anti-human GRK5 antibody C-20 (1:500) (Santa Cruz Biotechnology, Inc.), anti-human α-synuclein monoclonal antibody (LB509 (epitope position 115-121/122 amino acids) (Baba M. et al., Am J Pathol. 152, 879-884, 1998) (1:1000), SYN211 (1:1000) (provided from Dr. Fujiwara, Faculty of Pharmaceutical Sciences, The University of Tokyo), and anti-Ser-129 phosphorylated α-synuclein (pSer129, 1:1000) (Fujiwara H. et al., Nat Cell Biol. 4, 160-164, 2002) were respectively used as primary antibodies. The sections were deparaffinized and subjected to blocking with phosphate buffered saline (PBS; pH 7.4) containing 10% calf serum. Next, the sections were incubated with primary antibodies at room temperature overnight and incubated with biotin-labeled secondary antibodies (1:500) (Vector Laboratories) for 2 hours. After incubating with avidin-biotin-peroxidase complex for 1 hour, the peroxidase label of the sections were visualized in 0.05 M Tris buffered saline added with 0.05% 3,3'-diaminobenzidine tetrahydrochloride (DAB) and 0.012% hydrogen peroxide (H₂O₂) to yield brown coloring. Subsequently, counter staining with hematoxylin was carried out. As fluorescence staining, Alexa 488 or Alexa 546 as secondary antibodies (1:500) (Molecular Probes, Inc.) were reacted for 5 hours, and fluorescence was observed with the FLUOVIEW FV300 Confocal Microscope (OLYMPUS CORPORATION).

### [Example 15]

### «Results»

### <Production of Transgenic C. elegans Strain>

To study the role ofGRK5 in the phosphorylation of α-synuclein, transgenic nematode strains were produced coexpressing GRK5 and α-synuclein. Initially, strains specifically expressing α-synuclein in two different neurons were produced respectively. dat-1 promoter provides dopamine neuron-specific expression, and mec-7 provides touch neuron-specific expression. The structures of the plasmids are shown in Fig. 9A. Human GRK5 was configured to be expressed in all neurons of nematodes by arranging it under the regulation of unc-119 promoter. Kinase-inactive mutant gene (K215R) of GRK5 was used as a negative control. A strain in which a transgene overexpressing GRK5 had been incorporated into the chromosome was crossed with nematodes specifically overexpressing α-synuclein in dopamine neurons or touch neurons. Thus, transgenic nematode strains coexpressing GRK5 and α-synuclein were obtained. The expression of α-synuclein was confirmed by immunohistochemical staining. In one strain, α-synuclein was expressed using the mec-7 promoter. The mec-7 gene is expressed in six touch neurons positioned in the vicinity of the tail of nematodes, namely, ALML/R (anterior lateral microtubule cell left/right), AVM (anterior ventral microtubule cell), PVM (posterior ventral microtubule cell), and PLML/R (posterior lateral microtubule cell left/right). α-synuclein-positive immunoreaction in a cell body of a neuron was observed diffusely in the cytoplasm (Fig. 9C). When a promoter of the dat-1 gene which is expressed in dopamine neurons was used, α-synuclein was expressed in eight dopamine neurons, namely, two pairs of CEPs (cephalic sencilla), one pair of ADEs (anterior deirids), and one pair of PDEs (posterior deirids) (Fig. 9B). Human GRK5 protein expressed in the nematodes was detected through immunohistochemical staining and immunoblotting. Immunohistochemical staining results revealed that human GRK5 was expressed in the entire nerve cell of the nematodes, namely, in the nerve cell body and neurites. A particularly strong GRK5-positive reaction was observed in the nerve ring, and the ventral nerve cord was stained with anti-GRK5 antibodies (Fig. 9D). As a result of immunoblotting using anti-FLAG antibody M2, a specific protein band of 64 kDa corresponding to the mobility of human GRK5 protein was detected in supernatants from homogenates of both the nematode strain expressing wildtype human GRK5 and the nematode strain expressing K215R mutant human GRK5 (Fig. 9E). No phenotype such as behavior disorder was observed and no effect of transgenes was observed in all the strains which had been subjected to gene transfer.

### [Example 16] Food-sensing Assay - Phosphorylation of α -Synuclein Ser-129 and Enhancement ofα -Synuclein Toxicity of by GRK5 in Dopamine Neurons -

To study whether GRK5 phosphorylates α-synuclein expressed in dopamine neurons, 3-day old nematodes were treated, from which sections were produced to conduct immunofluorescent histochemical staining using anti-α-synuclein antibodies and anti-Ser-129 phosphorylated α-synuclein antibodies. As a result of observation with a confocal microscope, dopamine neurons of a transgenic nematode coexpressing wildtype GRK5 and α-synuclein was found to be phosphorylated α-synuclein positive (Fig. 10B). However, no phosphorylated α-synuclein was found in a transgenic nematode coexpressing K215R mutant GRK5 and α-synuclein.

It was previously reported that a transgenic nematode suffers from a failure specific to food-sensing behavior, which transgenic nematode is configured to express mutant α-synuclein, which causes familial PD, in dopaminergic neurons (Kuwahara T. et al., J Biol Chem 281(1):334-340, 2006). Based on this result, they further studied whether the promotion of α-synuclein phosphorylation by GRK5 causes an abnormality in the food-detecting behavior (Fig. 10D). When exposed to bacteria, normal type N2 strain nematodes showed 60% or more reduced motilities. The cat-2 mutant, which is a mutant in which lacks the dopamine synthesis function in dopamine neurons showed an abnormality in the food-detecting behavior, in which the decrease in the number of bending was about 20% or less when exposed to bacteria. A decrease in motility upon exposure to bacteria was 36% or less in a transgenic nematode coexpressing wildtype GRK5 and α-synuclein, and that in a negative control transgenic nematode coexpressing the K215R mutant GRK5 and α-synuclein was 56% or less.

### [Example 17] Touch Assay - Phosphorylation of α-Synuclein Ser-129 and Enhancement of α-Synuclein Toxicity by GRK5 in Touch Neurons -

As is shown in Examples 3 to 7, serine at position 129 of α-synuclein is phosphorylated by GRK5 in cells. To determine whether α-synuclein expressed in touch neurons is phosphorylated, nematodes of Day 3, Day 6, and Day 9 were treated, from which sections were produced to carry out immunofluorescent histochemical staining using anti- α-synuclein antibodies and anti-Ser-129 phosphorylated α-synuclein antibody. As a result of observation with a confocal microscope, touch neurons of transgenic nematodes coexpressing wildtype GRK5 and α-synuclein were phosphorylated α-synuclein-positive (Fig. 11B). However, no phosphorylated α-synuclein was found in transgenic nematodes coexpressing the K215R mutant GRK5 and α-synuclein even in nematodes of Day 9. The percentage of phosphorylated α-synuclein-positive neurons increased in an age dependent manner (40%, 60%, and 90% or more in touch neurons of 3-day, 6-day, and 9-day old nematodes) (Fig. 11D).

In a normal response, a nematode moves backward when it is lightly contacted at its anterior side, and moves forward when it is lightly contacted at its posterior side. However, nematodes of a mec-7-deleted (e1506) strain lack the response to such contact (Chalfie et al., Cell. 1981 Apr; 24(1):59-69., Savage et al., J Cell Sci. 1994 Aug; 107 (Pt 8):2165-75.). Consequently, a decrease in response to contact reflects the decrease in function of touch neurons. To study whether enhancement of α-synuclein phosphorylation by GRK5 increases neurotoxicity, the neurotoxicity of α-synuclein phosphorylation was evaluated through touch assays (Fig. 11 E). Responses to contact of 3-day, 6-day, and 9-day old transgenic nematodes were evaluated. In any growth stage, both of the two transgenic nematode strains which coexpress α -synuclein and GRK5 and had been produced independently showed a significantly lower response to contact than that of a negative control transgenic nematode coexpressing α-synuclein and K215R mutant GRK5. However, in transgenic nematodes coexpressing GRK5 andα-synuclein in which serine at position 129 was mutated into alanine and in which the site did not undergo phosphorylation, there was no significant difference when the GRK5 was a wildtype GRK5 or a K215R mutant. mec-7 mutant nematodes assayed as a control showed remarkable lack in mechanosensation. In addition, when both cDNAs of wildtype GRK5 and α-synuclein were transduced, a significant progressive decrease in contact response was observed. It was revealed that the deletion of mechanosensation in nematodes is associated with a-synuclein phosphorylation, and that GRK5 enhances α-synuclein phosphorylation and increases the toxicity of α-synuclein.

### Industrial Applicability

Parkinson's disease (PD), a disease common in elderly persons and having an aspect of senile diseases, gradually progresses after onset and rarely directly causes death. However, when a PD patient becomes bedridden, the quality of life (QOL) of the patient and his caregivers is strikingly impaired. Furthermore, in that it is difficult to maintain a healthy general condition in bedridden patients, PD patients often succumb to infective diseases such as pneumonia, and thus have life spans far shorter than those of their healthy counterparts.

The present invention provides methods for testing whether a subject is susceptible to PD by detecting a gene associated with the susceptibility to PD, and detecting mutations in the gene. Itis possible to test whether a subject is susceptible to PD by detecting the gene or a polymorphism in the gene of the present invention. The present invention is expected to assist in the determination of more efficient strategies and courses of treatment in the prevention and treatment of PD.

The progression of PD can be suppressed by the methods of screening for a therapeutic agent for PD and by such therapeutic agent provided by the present invention. Accordingly, when PD occurs in an elderly person, the present invention may enable the patient to live out his lifespan without suffering from a final stage of being bedridden. If the period until becoming bedridden can be delayed, remarkable improvements in QOL can be expected. In addition, the onset of PD itself can be suppressed by diagnosing a person at a high risk of developing PD and administering to the person an agent for suppressing the progression of the disease. This approach also has substantial value. In industrialized countries having graying societies, an increase in absolute numbers of PD patients is expected. Accordingly, improvements in QOL of the patients can lead to reduction not only in the burden on the patients themselves but can also less the burden on their caregivers and the social economy.

## Claims

1. A method of testing whether or not a subject is susceptible to Parkinson's disease, which uses as an index the expression of G-protein-coupled receptor kinase-5 (GRK5) gene in the subject.

2. A method of testing whether or not a subject is susceptible to Parkinson's disease, which comprises the step of detecting a mutation in a GRK5 gene of the subject.

3. The method of claim 2, wherein the mutation is a polymorphic mutation.

4. A method of testing whether or not a subject is susceptible to Parkinson's disease, which comprises the step of determining a nucleotide at a polymorphic site in a GRK5 gene of the subject.

5. The method of claim 4, wherein the polymorphic site is (1) and/or (2):
(1) a polymorphic site on the GRK5 gene located at position 34799 of the nucleotide sequence of SEQ ID NO: 1; and
(2) a polymorphic site on the GRK5 gene located at position 60001 of the nucleotide sequence of SEQ ID NO: 1.

6. The method of claim 5, wherein the subject is deemed to be susceptible to Parkinson's disease when nucleotides in the polymorphic sites (1) and (2) set forth in claim 5 are (1b) and (2b), respectively:
(1b) the nucleotide at a site on the GRK5 gene located at position 34799 of the nucleotide sequence of SEQ ID NO: 1 is G; and
(2b) the nucleotide at a site on the GRK5 gene located at position 60001 of the nucleotide sequence of SEQ ID NO: 1 is C.

7. A method of testing whether or not a subject is susceptible to Parkinson's disease, wherein the subject is deemed to be susceptible to Parkinson's disease when a DNA block showing the haplotype of (1') is detected:
(1') a haplotype in which the nucleotides at polymorphic sites on a GRK5 gene located at positions 27377, 54748, and 60001 of the nucleotide sequence of SEQ ID NO: 1 are G, A, and C, respectively.

8. A method of testing whether or not a subject is susceptible to Parkinson's disease, comprising the steps of:
(a) determining a nucleotide at a polymorphic site on a GRK5 gene of the subject; and
(b) determining the subject as susceptible to Parkinson's disease when the nucleotide determined in step (a) is the same as a nucleotide type at said polymorphic site on a GRK5 gene that shows the haplotype of (1'):
(1') a haplotype in which nucleotides at polymorphic sites on a GRK5 gene located at positions 27377, 54748, and 60001 of the nucleotide sequence of SEQ ID NO: 1 are G, A, and C, respectively.

9. The method of claim 8, wherein the polymorphic site in step (a) is a polymorphic site set forth in (1):
(1) any polymorphic site on the GRK5 gene located at position 27377, 27849, 27876, 28813, 29000, 29855, 30366, 31037, 31706, 31964, 32542, 32723, 32988, 33064, 33618, 33889, 33911, 33994, 34799, 35173, 35188, 35189, 35190, 35240, 35284, 35402, 35415, 35494, 35522, 36405, 36433, 36449, 36546, 36817, 36868, 36882, 37004, 37148, 37295, 37413, 37722, 38393, 39182, 39193, 39301, 39316, 39456, 39487, 39550, 39638, 40097, 40134, 40193, 41149, 41157, 41428, 41498, 41618, 41667, 41843, 42364, 42418, 43017, 43037, 43190, 44600, 44607, 45522, 45998, 48284, 49971, 50122, 50873, 51463, 52944, 53223, 53397, 53703, 53763, 54267, 54748, 54782, 54794, 55139, 55457, 55627, 56254, 56593, 57338, 57606, 57648, 57688, 57843, 57978, 58067, 58456, 58616, 59263, 59908, or 60001 of the nucleotide sequence of SEQ ID NO: 1.

10. The method of claim 8, wherein the polymorphic site in step (a) is the polymorphic site set forth in (1) or (2):
(1) a polymorphic site on the GRK5 gene located at position 34799 of the nucleotide sequence of SEQ ID NO: 1; and
(2) a polymorphic site on the GRK5 gene located at position 60001 of the nucleotide sequence of SEQ ID NO: 1.

11. A method of testing a subject for the presence or absence of a susceptibility for Parkinson's disease, comprising the step of determining the subject to be susceptible to Parkinson's disease when a measured expression level of a GRK5 gene in the subject is elevated as compared to a control.

12. The method of any one of claims 1 to 11, wherein a biological sample derived from the subject is utilized as a test sample.

13. The method of any one of claims 1 to 12, wherein the Parkinson's disease is sporadic Parkinson's disease.

14. A reagent for testing whether or not a subject is susceptible to Parkinson's disease, comprising an oligonucleotide that hybridizes with a DNA comprising the polymorphic site of (1) or (2) of claim 5 and comprises at least 15 nucleotides in length.

15. A reagent for testing whether or not a subject is susceptible to Parkinson's disease, comprising a solid phase to which a nucleotide probe is immobilized, wherein the nucleotide probe hybridizes with a DNA comprising the polymorphic site of (1) or (2) of claim 5.

16. A reagent for testing whether or not a subject is susceptible to Parkinson's disease, comprising a primer oligonucleotide for amplifying a DNA comprising the polymorphic site of (1) or (2) of claim 5.

17. A reagent for testing whether or not a subject is susceptible to Parkinson's disease, comprising as an active ingredient (a) or (b):
(a) an oligonucleotide that hybridizes with a transcript of a GRK5 gene; and
(b) an antibody that recognizes a GRK5 protein.

18. A polynucleotide of (a) or (b):
(a) a polynucleotide which comprises the nucleotide sequence of SEQ ID NO: 5 or a nucleotide sequence in which one or more nucleotides are added, deleted, or substituted in the nucleotide sequence of SEQ ID NO: 5, wherein the polynucleotide has a reduced ability to bind with a Yin Yang-1 (YY1) transcription factor; and
(b) a polynucleotide which comprises the nucleotide sequence of SEQ ID NO: 3 or a nucleotide sequence in which one or more nucleotides are added, deleted, or substituted in the nucleotide sequence of SEQ ID NO: 3, wherein the polynucleotide has an increased ability to bind with a CRE-binding protein-1 (CREB-1) transcription factor.

19. A reagent for screening for an agent for treating or preventing Parkinson's disease, comprising as an active ingredient any one of (a) to (d):
(a) an oligonucleotide that hybridizes with a transcript of a GRK5 gene;
(b) an antibody that recognizes a GRK5 protein;
(c) an antibody that recognizes an α-synuclein protein in which a serine residue at position 129 is phosphorylated; and
(d) the polynucleotide of claim 18.

20. The reagent of any one of claims 14 to 17, and 19, wherein the Parkinson's disease is sporadic Parkinson's disease.

21. An agent for treating or preventing Parkinson's disease, comprising as an active ingredient a substance that suppresses the expression of a GRK5 gene or the function of a protein encoded by the gene.

22. The agent of claim 21, wherein the substance that suppresses the expression of the GRK5 gene is one selected from the group consisting of:
(a) an antisense nucleic acid against a transcript of the GRK5 gene or a portion thereof;
(b) a nucleic acid having a ribozyme activity of specifically cleaving a transcript of the GRK5 gene; and
(c) a nucleic acid having an activity of inhibiting the expression of the GRK5 gene through an RNA interference effect.

23. The agent of claim 21, wherein the substance that suppresses the function of the GRK5 protein is:
(a) an antibody that binds with a GRK5 protein; or
(b) a low-molecular-weight compound that binds with a GRK5 protein.

24. An agent for treating or preventing Parkinson's disease, comprising as an active ingredient a substance that inhibits the formation of a soluble α-synuclein oligomer.

25. A method of screening for an agent for treating or preventing Parkinson's disease, comprising the step of selecting a compound that reduces the expression level of a GRK5 gene or the activity of a protein encoded by the gene.

26. A method of screening for an agent for treating or preventing Parkinson's disease, comprising the steps of:
(a) contacting a test compound with a cell that expresses a GRK5 gene;
(b) measuring the expression level of the GRK5 gene; and
(c) selecting the compound that reduces the expression level as compared with that measured in the absence of the test compound.

27. A method of screening for an agent for treating or preventing Parkinson's disease, comprising the steps of:
(a) contacting a test compound with a cell or cell extract that comprises a DNA having a structure in which a transcriptional regulatory region of a GRK5 gene and a reporter gene are operably linked with each other;
(b) measuring the expression level of the reporter gene; and
(c) selecting the compound that reduces the expression level as compared with that measured in the absence of the test compound.

28. The method of any one of claims 25 to 27, wherein the GRK5 gene is:
(a) a mutant GRK5 gene in which a nucleotide on the GRK5 gene located at position 34799 of the nucleotide sequence of SEQ ID NO: 1 is G; or
(b) a mutant GRK5 gene in which a nucleotide on the GRK5 gene located at position 60001 of the nucleotide sequence of SEQ ID NO: 1 is C,
wherein the expression of the gene is elevated.

29. A method of screening for an agent for treating or preventing Parkinson's disease, comprising the steps of:
(a) contacting a test compound with a GRK5 protein and α-synuclein;
(b) measuring the interaction activity between the GRK5 protein and α-synuclein; and
(c) selecting the compound that reduces the interaction activity as compared with that measured in the absence of the test compound.

30. A method of screening for an agent for treating or preventing Parkinson's disease, comprising the steps of:
(a) contacting a test compound with a GRK5 protein and α-synuclein;
(b) measuring the activity of the GRK5 protein to phosphorylate α-synuclein as a substrate; and
(c) selecting the compound that reduces the phosphorylating activity as compared with that measured in the absence of the test compound.

31. A method of screening for an agent for treating or preventing Parkinson's disease, comprising the steps of:
(a) contacting a test compound with a GRK5 protein and an insoluble α-synuclein oligomer;
(b) measuring the amount of a soluble α-synuclein oligomer; and
(c) selecting the compound that reduces the amount of the soluble α-synuclein oligomer as compared with that measured in the absence of the test compound.

32. A method of screening for an agent for treating or preventing Parkinson's disease, comprising the steps of:
(a) contacting a test compound with a YY1 transcription factor, and a polynucleotide comprising a YY1-recognition sequence or the polynucleotide of claim 18(a);
(b) measuring a binding activity between the polynucleotide and the transcription factor; and
(c) selecting the compound that elevates the binding activity as compared with that measured in the absence of the test compound.

33. A method of screening for an agent for treating or preventing Parkinson's disease, comprising the steps of:
(a) contacting a test compound with a CREB-1 transcription factor, and a polynucleotide comprising a CREB-recognition sequence or the polynucleotide of claim 18(b);
(b) measuring a binding ability between the polynucleotide and the transcription factor; and
(c) selecting the compound that reduces the binding activity as compared with that measured in the absence of the test compound.

34. A transgenic non-human animal, which expresses an exogenous GRK5 protein and α-synuclein.

35. A method of screening for an agent for treating or preventing Parkinson's disease, comprising the steps of:
(a) administering a test compound to the transgenic non-human animal of claim 34;
(b) monitoring a behavior in the animal caused by neurotoxicity; and
(c) selecting the compound that suppresses occurrence of the behavior.
